(19) Europäisches Patentamt
European Patent Office
Office européen des brevets

(11) EP 0 959 975 B1

(12) EUROPEAN PATENT SPECIFICATION

(45) Date of publication and mention of the grant of the patent:
**29.12.2004 Bulletin 2004/53**

(21) Application number: **96930037.5**

(22) Date of filing: **19.09.1996**

(51) Int Cl.[7]: **B01D 15/08**, C07D 251/54

(86) International application number:
**PCT/DK1996/000399**

(87) International publication number:
**WO 1997/010887 (27.03.1997 Gazette 1997/14)**

(54) **NOVEL AFFINITY LIGANDS AND THEIR USE**

NEUARTIGE AFFINITATSLIGANDEN UND IHRE VERWENDUNG

NOUVEAUX LIGANDS A AFFINITE ET LEUR UTILISATION

(84) Designated Contracting States:
**AT BE CH DE DK ES FI FR GB GR IE IT LI LU NL PT SE**
Designated Extension States:
**AL LT LV SI**

(30) Priority: **20.09.1995 GB 9519197**

(43) Date of publication of application:
**01.12.1999 Bulletin 1999/48**

(73) Proprietors:
- **NOVO NORDISK A/S**
  **2880 Bagsvaerd (DK)**
- **ProMetic BioSciences Limited**
  **Ballasalla, Isle of Man IM9 2AP (GB)**

(72) Inventors:
- **LOWE, Christopher, R.**
  **Saffron Walden CB10 2PW (GB)**
- **SPROULE, Kenneth**
  **Cambridge CB3 9NN (GB)**
- **LI, Rongxiu**
  **Cambridge CB4 3LX (GB)**
- **STEWART, David, J.**
  **Huntingdon, NY 11743 (US)**
- **PEARSON, James, C.**
  **Chesterton Cambridge CB4 1DX (GB)**
- **BURTON, Steven, J.**
  **Cambridge CB3 7HP (GB)**

(56) References cited:
**EP-A- 0 302 503**

- **ARCH. IMMUNOL. THER. EXP., Volume 30, No. 1, 1982, M. KONIECZNY et al., "Search for Carriers for Non-Covalent Binding of Interferon Among 1,3,5-Triazine Derivatives of Dextran", pages 1-9.**
- **J. MOL. RECOGN., Volume 5, 1992, N.P. BURTON and C.R. LOWE, "Design of Novel Affinity Adsorbents for the Purification of Trypsin-Like Proteases", pages 55-68.**
- **J. CHROM., Vol. 508, 1990, S. J. BURTON et al., "Design and Applications of Biomimetic Anthraquinone Dyes. III. Anthraquinone-Immobilised C.I. Reactive Blue 2 Analogues and Their Interaction with Horse Liver Alcohol Dehydrogenase and Other Adenine Nucleotide-Binding Proteins", pages 109-125.**
- **A. ATKINSON et al., "The Potential of Organic Dyes as Affinity Ligands in Protein Studies", In: AFFINITY CHROMATOGRAPHY AND RELATED TECHNIQUES, PROC. 4TH INT. SYMP. Edited by T.C.J. GRIBNAU et al., ELSEVIER SCI. PUBL., AMSTERDAM, ANAL. CHEM. SYMP. SERIES, 1982, Vol. 9, pages 399-410.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 959 975 B1

Printed by Jouve, 75001 PARIS (FR)

## Description

The present invention relates to novel affinity ligands, their preparation and attachment to matrices which may consist of solid, semi-solid, particulate or colloidal materials, or soluble polymers. The invention furthermore relates to these novel affinity ligand-matrix conjugates and the preparation and use thereof in the purification of proteinaceous materials such as e.g. immunoglobulins, insulins, Factor VII, or Human Growth Hormone or analogues, derivatives and fragments thereof and precursors.

BACKGROUND OF THE INVENTION

Modern protein purification principles very much rely upon chromatographic separation techniques such as gel permeation chromatography (GPC), ionexchange chromatography (IEC), hydrophobic interaction chromatography (HIC), reversed phase high pressure chromatography (RP-HPLC), and affinity chromatography (AC). These techniques are easily adapted to laboratory scale purification of peptides and proteins meant for research and scientific experiments, resulting in pure and biologically active substances. In most cases little or no interest is paid to process economy, process validation, or cleaning in place procedures, as the material will only rarely be used for clinical experiments and because labour costs far exceed the costs of equipment and matrices.
However, large scale industrial downstream processing must take into consideration factors such as economy, robustness of matrices and cleaning in place with *e.g*. NaOH, urea, or ethanol. Today the demand for inexpensive and robust matrices stable in 1M NaOH, 7M urea, or 80% v/v ethanol is met by a number of commercial suppliers within the field of GPC, IEC, HIC, and RP-HPLC. A combination of these principles has for many years resulted in almost pure protein bulk substances although use of extreme buffers and many purification steps have resulted in poor recoveries, increased costs and questionable stability of the bulk preparations.
It has long been realised that the principle of affinity chromatography could also be applied to large scale operations. Unfortunately, adsorbents created with natural biological ligands such as monoclonal or polyclonal antibodies tend to be expensive to produce because the ligands themselves often require extensive purification, are biologically and chemically labile and tend to be difficult to immobilise with retention of their biological activity. Therefore, there has been a long term need to replace the expensive chemically and biologically labile monoclonal or polyclonal antibodies with less expensive and more robust ligands mimicking the specificity of antibodies.
Affinity chromatography occupies a unique place in separation technology as the protein to be purified adsorbs selectively and reversibly to the complementary binding substance such as an antibody molecule. Purification factors of several thousandfold are often observed with high recoveries, in contrast to the conventional purification methods offering factors from 5-50 times. The high purification factors obtained in affinity chromatography dramatically reduces the number of purification steps in the downstream process. Further, the very low non specific binding observed in affinity chromatography, makes it possible to purify a given protein from complex biological mixtures, to separate incorrectly folded forms from native molecules, and to recover the protein specifically from even large volumes of tissue extracts or fermentation cultures.
The affinity sorbent comprises a solid, usually permeable, support matrix, to which a suitable ligand is covalently attached, contained in a conventional chromatographic column. A crude sample, containing the complementary biopolymer is passed over the support matrix, under conditions which promote specific binding interactions with the immobilised ligand. The column is washed with buffer to remove unretarded molecules followed by an elution step in which the protein is eluted in its pure form. A typical affinity adsorbent is based on a solid support, a spacer arm and a ligand. The solid support can be made of bead-formed agarose with an open pore structure. The spacer arm may encourage protein binding by making the ligand more accessible. The length and nature of the spacer arm can be determined by a person skilled in the art. The ligand should exhibit specific and reversible binding to the protein to be purified even after immobilization. In addition to antibodies, a number of compounds including enzymatic co-factors, amino acids, peptides, proteins, concanavalin A, Lectin, thiols, and dyes have been used as affinity ligands.
Affinity chromatography has been used in many applications. A comprehensive list is given in e.g. "Affinity Chromatography A Practical Approach" from IRL Press, 1985, and "Affinity Chromatography, Principles and Methods" from Pharmacia Fine Chemicals 1979.
Conventional substrate or substrate analogue affinity ligands, especially dyes, have been used for large scale purification of specific enzymes or groups of enzymes. (Scawen M.D. and Atkinson T. 1987, Reactive Dyes in Protein and Enzyme Technology, Ed. Clonis Y.D. *et al;* Macmillan Press, pp. 51-85).
Dye affinity chromatography has over the years gained much interest because of the relative low price of such matrices, their robustness and their ability to withstand NaOH, urea and ethanol. Some of the more widely used ligands in this type of affinity chromatography have been a variety of reactive triazine-based textile dyes immobilised to agarose and other supports. The use of affinity chromatography on immobilized dyes has been reviewed (Lowe C.R. and Pearson J.C. 1984, Methods in Enzymology 104, pp. 97-113). Selective interactions with the $NAD^+$-binding site of horse

liver alcohol dehydrogenase were shown with dye analogues of Cibacron Blue F3G-A (Lowe C.R. *et al* 1986; Journal of Chromatography 376, pp. 121-130). In the search for carriers for non-covalent binding of interferon among 1,3,5-triazine derivatives of dextran compounds of a structure similar to Cibacron Blue were synthesized and bound to dextran of various molecular weight with covalent (ether) binding. The obtained high-molecular weight compounds were subjected to biological trials which indicated that link with quinone system in its molecule is an indispensable but not sufficient agent for affinity to interferon (Mieczyslaw Konieczny et al. 1982, Arch. Immunol. Ther. Exp. **30** pp.1-9). The selective purification approach was further illustrated with the computer aided design of a novel affinity adsorbent mimicking the phenyl-arginine dipeptide substrate for the purification of porcine pancreatic kallikrein (Burton N.P. and Lowe C.R. 1992, Journal of Molecular Recognition 5, pp. 55-58).

US Patent No. 4,562,252 discloses a particular ligand structure consisting of two m-aminophenyl boronic acid groups attached to a triazine ring for glycoprotein separation.

However, despite the rapid progress in affinity technology over the past few years, the need is still to develop a technology by which a specific mimetic ligand can be identified for a protein in order to produce an inexpensive and stable affinity column capable of repeated large scale purification of the said protein, e.g. in the separation and purification of proteinaceous materials, such as immunoglobulins, insulins, Factor VII, or human Growth Hormone or analogues, derivatives and fragments thereof and precursors, whether derived from natural or recombinant sources.

DETAILED DESCRIPTION OF THE INVENTION

The present invention relates to novel affinity ligands, their preparation and attachment to matrices, and the use of these novel affinity ligand-matrices in the purification of proteinaceous materials.

The current invention is based on the notion that the selectivity of hydrophobic ligands may be increased by increasing the complexity and spatial geometry of the hydrophobic component, and the incorporation of various functional groups capable of partaking in electrostatic and hydrogen bonding interactions thereby promoting selective interactions with protein binding sites. This work led to the discovery of a generic group of novel affinity ligands, which have been unexpectedly found to be generally applicable to the isolation and purification of proteins by affinity chromatography.

In contrast to the above mentioned selective approach where enzyme substrates, analogues thereof or substrate mimetics were used as ligands, the ligands defined in this application are directed towards any surface of the protein molecule, making the principle applicable for any protein. The ligands are designed by computer modelling techniques and/or by screening of mimetic ligand libraries. Further, the current invention has the advantage that the structure of the protein binding site architecture is not required for design and development of the ligand, and consequently the materials and techniques described herein have a significantly greater utility.

A feature of the present invention is the provision of a general tool for protein resolution, isolation and purification. A family of subtly different chemical structures has been synthesised, which have the ability to interact with different proteins. A particularly effective ligand structure for a given protein is identified by screening a range of ligands provided by the invention for suitable binding properties.

By way of example, affinity ligands of high selectivity and specificity which are currently available for the separation and purification of immunoglobulins are often proteinaceous materials derived from either bacterial or recombinant sources and include materials such as Protein A, Protein G and Protein L. Immobilisation of these, and similar, proteins often results in a significant loss of biological activity. Continual and repeated use of immobilised proteins as affinity media leads to a further diminution of biological activity. Furthermore, the inherent nature of these biological macromolecules imposes strict limitations with respect to the use of buffer salts, organic solvents and pH levels in affinity chromatography and related techniques.

Novel affinity ligands provided by this invention can be used in place of protein A and Protein G and are significantly more flexible in their use, are more robust, less expensive to produce and offer equivalent levels of purification.

Another example is the use of novel affinity matrices provided by this invention in biotechnology.

The present invention relates to affinity ligand matrix conjugates comprising a ligand with the general formula (a):

(a)

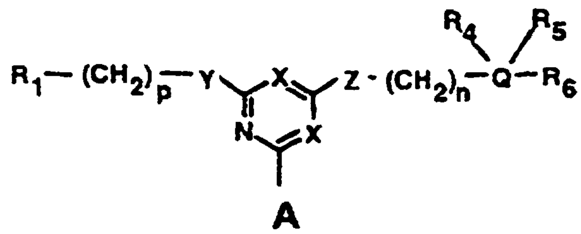

wherein

$R_1$ represents a hydrogen atom, an alkyl group containing from 1 to 6 carbon atoms, a hydroxyalkyl group containing from 1 to 6 carbon atoms, a cyclohexyl group, an amino group, a phenyl group, naphthyl group, 1-phenylpyrazole, indazole, benzthiazole group, benzoxazole group, or a benzimidazole group, each of which benzene, naphthalene, phenylpyrazole, indazole, benzthiazole, benzoxazole or benzimidazole ring is optionally substituted with one or more substituents independently selected from the group consisting of alkyl groups containing from 1 to 6 carbon atoms, alkoxy groups containing from 1 to 6 carbon atoms, acyloxy or acylamino groups containing from 1 to 6 carbon atoms, amino groups, hydroxyl groups, carboxylic acid groups, sulphonic acid groups, carbamoyl groups, sulphamoyl groups, alkylsulphonyl groups containing from 1 to 6 carbon atoms or halogen atoms;

Y represents an oxygen atom, a sulphur atom or a group N-$R_2$;

Z represents an oxygen atom, a sulphur atom or a group N-$R_3$;

$R_2$ and $R_3$ each independently represent a hydrogen atom, an alkyl group containing from 1 to 6 carbon atoms; a hydroxyalkyl group containing from 1 to 6 carbon atoms, a benzyl group or a β-phenylethyl group;

$R_4$, $R_5$ and $R_6$ each independently represent a hydrogen atom, a hydroxyl group, an alkyl group containing from 1 to 6 carbon atoms, an alkoxy group containing from 1 to 6 carbon atoms, an amino group, an acyloxy or acylamino group containing from 1 to 6 carbon atoms, a carboxylic acid group, a sulphonic acid group, a carbamoyl or sulphamoyl group, an alkylsulphonyl group containing from 1 to 6 carbon atoms or a halogen atom;

one of the symbols X represents a nitrogen atom and the other symbol X represents a nitrogen atom or a carbon atom carrying a chlorine atom or a cyano group;

Q represents a benzene; naphthalene, benzthiazole, benzoxazole 1-phenylpyrazole, indazole or benzimidazole ring;

n is an integer between 0 and 6;

p is an integer between 0 and 20;

and

which ligand is attached to a support matrix in position A, optionally through a spacer arm interposed between the matrix and ligand.

with the proviso that formula (a) does not comprise the reaction products of the compounds 4-chloro-2,6-di (phenyl-amino)-1,3,5-triazine-3'-sulfonic acid, 4-chloro-2,6-di (phenyl-amino)-1,3,5-triazine-3',2''-disulfonic acid, and 4-chloro-2-(4''-amino-phenyl-amino)-1,3,5-triazine-3',2''-disulfonic acid with Dextran T 500.

The optional spacer arm is preferably represented by the general formula (b)

$$-T-[-L-V-]_m- \quad (b)$$

wherein T represents an oxygen atom, a sulphur atom or a group N-$R_7$; wherein $R_7$ represents a hydrogen atom or an alkyl group containing from 1 to 6 carbon atoms;

V represents an an oxygen atom, a sulphur atom, a -COO- group, a CONH group or an NHCO group or a -$PO_3H$-group , an NH-arylene-$SO_2$-$CH_2$-$CH_2$ group or an N-$R_8$ group; wherein $R_8$ represents a hydrogen atom or an alkyl group containing from 1 to 6 carbon atoms;

L represents an optionally substituted hydrocarbon linkage containing from 2 to 20 carbon atoms; and

m is 0 or 1.

The support matrix may be any compound or material, particulate or non particulate, soluble or insoluble, porous or non porous which may be used in conjunction with affinity ligands to form a affinity ligand matrix conjugate and which provides a convenient means of separating the affinity ligands from solutes in a contacting solution.

The present invention provides novel affinity ligand-matrix conjugates, which affinity ligand-matrix conjugates may be used in the separation and purification of proteinaceous materials, such as immunoglobulins, insulins, Factor VII, or human Growth Hormone or analogues, derivatives and fragments thereof and precursors, whether derived from

natural or recombinant sources.

In a preferred embodiment, the invention provides novel affinity ligand matrix conjugates which are represented by the General Formula (I):

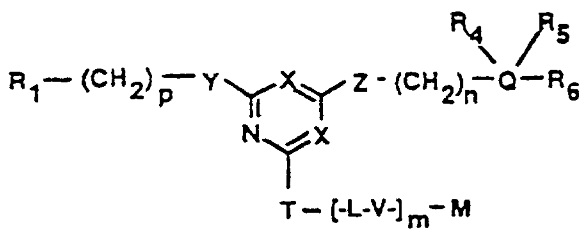

(I)

wherein

$R_1$, Y, Z, $R_2$, $R_3$, $R_4$, $R_5$, $R_6$, X, Q, n and p have the meanings specified above,

T represents an oxygen atom, a sulphur atom or a group $N-R_7$;

V represents an oxygen atom, a sulphur atom, a -COO- group, a CONH group or an NHCO group or a $-PO_3H-$ group , an NH-arylene-$SO_2$-$CH_2$-$CH_2$ group or an $N-R_8$ group;

$R_7$ and $R_8$ each independently represents a hydrogen atom or an alkyl group containing from 1 to 6 carbon atoms;

L represents an optionally substituted hydrocarbon linkage containing from 2 to 20 carbon atoms;

m is 0 or 1; and

M represents the residue of a support matrix.

The term "alkyl group containing from 1 to 6 carbon atoms" as used herein, alone or in combination, refers to a straight or branched, saturated hydrocarbon chain having 1 to 6 carbon atoms such as e.g. methyl, ethyl, n-propyl, isopropyl, n-butyl, sec-butyl, isobutyl, tert-butyl, n-pentyl, 2-methylbutyl, 3-methylbutyl, n-hexyl, 4-methylpentyl, neopentyl, n-hexyl and 2,2-dimethylpropyl.

The term "hydroxyalkyl group containing from 1 to 6 carbon atoms" as used herein, alone or in combination, refers to a straight or branched, saturated hydrocarbon chain having 1 to 6 carbon atoms substituted with one or more hydroxy groups, preferably one hydroxy group, such as e.g. hydroxymethyl, 2-hydroxyethyl, 3-hydroxypropyl, 2-hydroxypropyl, 4-hydroxybutyl, 5-hydroxypentyl, 6-hydroxyhexyl.

The term " alkoxy group containing from 1 to 6 carbon atoms" as used herein, alone or in combination, refers to a straight or branched monovalent substituent comprising an alkyl group containing from 1 to 6 carbon atoms linked through an ether oxygen having its free valence bond from the ether oxygen and having 1 to 6 carbon atoms e.g. methoxy, ethoxy, propoxy, isopropoxy, butoxy, pentoxy.

Term "halogen" means fluorine, chlorine, bromine or iodine.

The term "acyloxy or acylamino containing from 1 to 6 carbon atoms " as used herein refers to a monovalent substituent comprising an alkyl group containing from 1 to 5 carbon atoms linked through a carbonyloxy or oxycarbonyl group such as a methylcarbonyloxy, ethylcarbonyloxy, methyloxycarbonyl or ethyloxycarbonyl group or linked through a carbonylamino or aminocarbonyl group such as a methylcarbonylamino, ethylcarbonylamino, methylaminocarbonyl or ethylaminocarbonyl group.

The term "alkylsulfonyl containing from 1 to 6 carbon atoms" as used herein refers to a monovalent substituent comprising a alkyl group containing from 1 to 6 carbon atoms linked through a sulfonyl group such as e.g. methylsulfonyl, ethylsulfonyl, n-propylsulfonyl, isopropylsulfonyl, n-butylsulfonyl, sec-butylsulfonyl, isobutylsulfonyl, tert-butylsulfonyl, n-pentylsulfonyl, 2-methylbutylsulfonyl, 3-methylbutylsulfonyl, n-hexylsulfonyl, 4-methylpentylsulfonyl, neopentylsulfonyl, n-hexylsulfonyl and 2,2-dimethylpropylsulfonyl.

The term "one or more substituents independently selected from" shall more preferably refer to from 1-3 substituents. The term shall further preferably refer to 1-2 substituents and most preferably refer to one substituent.

In the present specification, whenever the term insulin is used in a plural or a generic sense, it is intended to encompass both naturally occurring insulins and insulin analogues and derivatives thereof and precursors. By the term insulin is thus also meant insulin from any species, e.g. human insulin. By the term "insulin analogue" as used herein is meant human insulin with one or several amino acid substitutions, one or several amino acid deletions, one or several amino acid additions or combinations hereof. The term "insulin derivative" means insulin chemically modified in one or several residues. By "insulin precursor" as used herein is meant any molecule which by enzymatic or chemical conversion results in formation of insulin, insulin fragments, e.g. des-Thr(B30)-insulin, insulin analogues, or insulin derivatives.

The term "optionally substituted hydrocarbon linkage containing from 2 to 20 carbon atoms" as used herein

refers to one or more linear or branched alkyl chains, optionally substituted with for example hydroxy or alkoxy groups containing from 1 to 6 carbonatoms, and optionally linked together by amino, ether, thioether, ester, amide or sulphonamide bonds providing a chain containing from 2 to 20 carbon atoms. The construction is preferably flexible. The construction of such optionally substituted hydrocarbon linkages is for example described in Lowe, C. R. and Dean, P.D. G, 1974, Affinity Chromatography, John Wiley & Sons, London, which hereby are incorporated by reference.

In a preferred embodiment, these conjugates are represented by the General Formula (I):

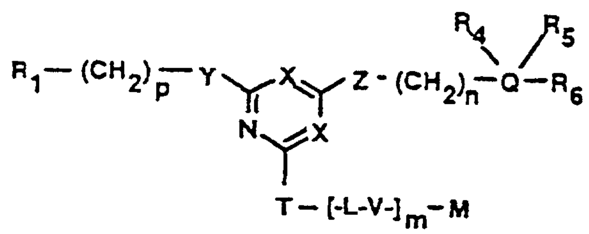

(I)

wherein

$R_1$ represents a hydrogen atom, an alkyl group containing from 1 to 6 carbon atoms, a hydroxyalkyl group containing from 1 to 6 carbon atoms, a cyclohexyl group, an amino group, a phenyl group or a naphthyl group, which may be substituted on the benzene or naphthalene ring by alkyl groups containing from 1 to 6 carbon atoms, alkoxy groups containing from 1 to 6 carbon atoms, acyloxy or acylamino groups containing from 1 to 6 carbon atoms, amino groups, hydroxyl groups, carboxylic acid groups, sulphonic acid groups, carbamoyl groups, sulphamoyl groups, alkylsulphonyl groups or halogen atoms;

T represents an oxygen atom, a sulphur atom or a group N-$R_7$;

Y represents an oxygen atom, a sulphur atom or a group N-$R_2$;

Z represents an oxygen atom, a sulphur atom or a group N-$R_3$;

$R_2$ and $R_3$ each independently represent a hydrogen atom, an alkyl group containing from 1 to 6 carbon atoms; a hydroxyalkyl group containing from 1 to 6 carbon atoms, a benzyl group or a β-phenylethyl group;

$R_4$, $R_5$ and $R_6$ each independently represent a hydrogen atom, a hydroxyl group, an alkyl group containing from 1 to 6 carbon atoms, an alkoxy group containing from 1 to 6 carbon atoms, an amino group, an acyloxy or acylamino group containing from 1 to 6 carbon atoms, a carboxylic acid group, a sulphonic acid group, a carbamoyl or sulphamoyl group, an alkylsulphonyl group or a halogen atom;

one of the symbols X represents a nitrogen atom and the other symbol X represents a nitrogen atom or a carbon atom carrying a chlorine atom or a cyano group;

V represents an oxygen atom, a sulphur atom, a -COO- group, a CONH group or an NHCO group or a -$PO_3H$- group , an NH-arylene-$SO_2$-$CH_2$-$CH_2$ group or an N-$R_8$ group;

$R_7$ and $R_8$ each independently represents a hydrogen atom or an alkyl group containing from 1 to 6 carbon atoms;

L represents an optionally substituted hydrocarbon linkage containing from 2 to 20 carbon atoms;

Q represents a benzene or naphthalene ring;

n is an integer between 0 and 6;

p is an integer between 0 and 20;

m is 0 or 1; and

M represents the residue of a support matrix which may be any compound or material, particulate or non particulate, soluble or insoluble, porous or non-porous which may be used in conjunction with affinity ligands to form a novel affinity ligand-matrix conjugate of General Formula (I) and which provides a convenient means of separating the affinity ligands from solutes in a contacting solution.

It will be appreciated that this invention relates, inter alia, to the use of compounds which are pyridines, diazines or triazines carrying a T-$[L\text{-}V]_{0\text{-}1}$-M substituent, or the precursor thereof, and other substituents linked to the ring via a heteroatom. Such substituents may include any non-interfering group comprising 0 to 10 or 20 C atoms.

In a preferred embodiment of the invention, $R_1$ represents a phenyl or naphthyl group each of which is optionally substituted on the benzene or naphthalene ring with one or more independently selected from the group consisting of hydroxyl groups or carboxylic acid groups.

In another preferred embodiment of the invention, $R_2$ represents a hydrogen atom.

In another preferred embodiment of the invention, $R_3$ represents a hydrogen atom.

In another preferred embodiment of the invention, $R_4$ represents a hydrogen atom, a hydroxyl group, a carboxylic acid group, or an amino group.

In another preferred embodiment of the invention, $R_5$ represents a hydrogen atom, a hydroxyl group, a carboxylic acid group, or an amino group.
In another preferred embodiment of the invention, $R_6$ represents a hydrogen atom, a hydroxyl group, a carboxylic acid group, or an amino group.
In another preferred embodiment of the invention, $R_7$ represents a hydrogen atom.
In another preferred embodiment of the invention, T represents an oxygen atom or an NH group.
In another preferred embodiment of the invention, Y represents N-$R_2$ wherein $R_2$ is as defined above.
In another preferred embodiment of the invention, Z represents N-$R_3$ wherein $R_3$ is as defined above.
In another preferred embodiment of the invention, both X represents a nitrogen atom.
In another preferred embodiment of the invention, Q represents a benzene or naphthalene ring.
In another preferred embodiment of the invention, n represents 0 or 2.
In another preferred embodiment of the invention, p represents 0 or 2.
In another preferred embodiment of the invention, m represents 0 or 1.
In another preferred embodiment of the invention, L represents an ethyl, propyl, hydroxypropyl, butyl, pentyl, hexyl, octyl or decyl group and V and m are as defined above.
In another preferred embodiment of the invention, V represents an oxygen atom, a -COO- group, a -$PO_3$H- group, or an N-$R_8$ group; and more preferred an oxygen atom or an NH group and L and m are as defined above.
In another preferred embodiment of the invention, m represents 1 and L and V are as defined above.
The term "integer between x and y" may include the values x (including zero) and y.
The invention also provides methods for the manufacture of the novel affinity ligand-matrix conjugates according to the invention which comprises reacting, in any order, a halogenoheterocyclic compound of General Formula (II):

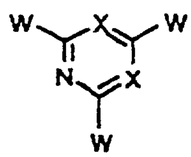

(II)

wherein the symbols X have the meaning hereinbefore specified and W represents a halogen atom with

(i) a compound of General Formula (III):

$$R_1\text{-}(CH_2)_p\text{-}Y\text{-}H \quad \text{(III)}$$

wherein the symbols $R_1$, Y and p have the meanings hereinbefore specified and H is hydrogen,

(ii) a compound of General Formula (IV)

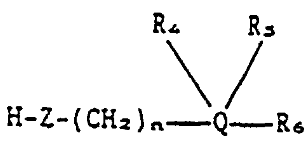

(IV)

wherein the symbols $R_4$, $R_5$, $R_6$, Q, Z and n have the meanings hereinbefore specified, and

(iii) with either an optionally derivatised support matrix of General Formula V

$$H-T-[-L-V-]_m-M \quad (V)$$

wherein the symbols L, M, V, T, and m have the meanings hereinbefore specified
or, with a linking unit of General Formula (VI)

$$H-T-L-V-H \quad (VI)$$

wherein the symbols H, L, V and T have the meanings hereinbefore specified to give a compound of General Formula (VII):

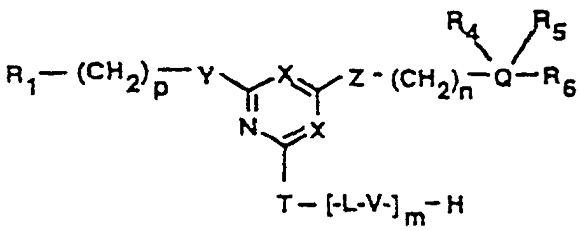

(VII)

wherein $R_1$, $R_4$, $R_5$, $R_6$, Q, L, T, V, X, Y, Z, m, n and p have the meanings hereinbefore specified; the compound of General Formula (VII) is then reacted further with a support matrix whose residue is represented by M using activating and coupling procedures well known to those skilled in the art.

As examples of halogenoheterocyclic compounds of General Formula (II) there may be mentioned 5-chloro-2,4,6-trifluoropyrimidine, 5-cyano-2,4,6-trichloropyrimidine, cyanuric fluoride, cyanuric bromide and, above all, cyanuric chloride.

As examples of compounds of General Formula (III) there may be mentioned amines such as ammonia, methylamine, ethylamine, propylamine, isopropylamine, diisopropylamine, isobutylamine, amylamine, hexylamine, ethanolamine, diethanolamine, aniline, N-methylaniline, N-ethylaniline, N-isopropylaniline, 1,4-diaminobutane, 1,6-diaminohexane, N-tert-butylaniline, p-toluidine, p-butylaniline, 2,4-dimethylaniline, p-anisidine, p-ethoxyaniline, p-aminoacetanilide, p-aminophenol, p-chloroaniline, orthanilic acid, metanilic acid, sulphanilic acid, 4-methylaniline-2-sulphonic acid, 4-methoxyaniline-2-sulphonic acid, aniline-2,5-disulphonic acid, N-methylmetanilic acid, o-, m- and p-aminobenzoic acid, p-aminobenzamide, p-aminobenzenesulphonamide, 1-amino-2-, 3-, 4-, 5-, 6-, 7- and 8-naphthol, 2-amino-3-, 4-, 5-, 6-, 7- and 8-naphthol, 5-, 6- and 7-amino-1-naphthol-3-sulphonic acid, N-benzylaniline, benzylamine, 4-methylbenzylamine, 4-hydroxybenzylamine, 4-methoxybenzylamine, 4-acetoxybenzylamine, 4-acetylaminobenzylamine, N-methylbenzylamine, β-phenylethylamine, N-butyl-benzylamine, N-benzyl-β-phenylethylamine, N-(β-hydroxyethyl)-benzylamine, N-tert-butyl-benzylamine, N-benzyltyramine and tyramine; phenols such as phenol, o-, m- and p-cresol, catechol, resorcinol, hydroquinone, p-chlorophenol, 1-naphthol and 2-naphthol, 1-naphthol-4-sulphonic acid, 2-naphthol-6-sulphonic acid and 2-hydroxy-3-naphthoic acid; thiols such as ethylthiol, thioglycollic acid, thiophenol and thio-p-cresol, and aromatic heterocycles such as 5-amino-1-phenylpyrazole, 6-aminoindazole, 2-aminobenzimidazole, 2-aminobenzthiazole, and 2-amino-5-chlorobenzoxazole.

As examples of compounds of General Formula (IV) there may be mentioned amines such as aniline, N-methylaniline, N-ethylaniline, N-isopropylaniline, N-tert-butylaniline, p-toluidine, p-butylaniline, 2,4-dimethylaniline, p-anisidine, p-ethoxyaniline, p-aminoacetanilide, p-aminophenol, p-chloroaniline, orthanilic acid, metanilic acid, sulphanilic acid, 4-methylaniline-2-sulphonic acid, 4-methoxyaniline-2-sulphonic acid, aniline-2,5-disulphonic acid, N-methylmetanilic acid, o-, m- and p-aminobenzoic acid, 1-amino-2-, 3-, 4-, 5-, 6-, 7- and 8-naphthol, 2-amino-3-, 4-, 5-, 6-, 7- and 8-naphthol, 5-, 6- and 7-amino-1-naphthol-3-sulphonic acid, p-aminobenzamide, p-aminobenzenesulphonamide, N-benzylaniline, benzylamine, 4-methylbenzylamine, 4-hydroxybenzylamine, 4-methoxybenzylamine, 4-acetoxybenzylamine, 4-acetylaminobenzylamine, N-methylbenzylamine, β-phenylethylamine, N-butyl-benzylamine, N-benzyl-β-phenylethylamine, N-(β-hydroxyethyl)-benzylamine, N-tert-butyl-benzylamine, N-benzyltyramine and tyramine; phenols such as phenol, o-, m- and p-cresol, catechol, resorcinol, hydroquinone, p-chlorophenol, 1- and 2-naphthol, 1-naphthol-4-sulphonic acid, 2-naphthol-6-sulphonic acid and 2-hydroxy-3-naphthoic acid; thiols such as thiophenol and thio-p-cresol, and aromatic heterocycles such as 5-amino-1-phenylpyrazole, 6-aminoindazole, 2-aminobenzimidazole, 2-aminobenzthiazole, and 2-amino-5-chlorobenzoxazole.

As examples of support matrices whose residue is represented by M, there may be mentioned insoluble support matrices such as a naturally occurring polymer, for example a polypeptide or protein such as cross linked albumin or a polysaccharide such as agarose, alginate, carrageenan, chitin, cellulose, dextran or starch; synthetic polymers such as polyacrylamide, polystyrene, polyacrolein, polyvinyl alcohol, polymethylacrylate, perfluorocarbon; inorganic compounds such as silica, glass, kieselguhr, alumina, iron oxide or other metal oxides or co-polymers consisting of any combination of two or more of a naturally occurring polymer, synthetic polymer or inorganic compounds. Also included within the definition of support matrices whose residue is represented by M are soluble support matrices comprising polymers such as dextran, polyethylene glycol, polyvinyl alcohol or hydrolysed starch which provide affinity-ligand matrix conjugates for use in liquid partitioning; or support matrices comprising compounds such as perfluorodecalin which provide affinity-ligand matrix conjugates for use in the formation of affinity emulsions. For the avoidance of doubt, a support matrix is defined herein as any compound or material whether particulate or non-particulate, soluble or insoluble, porous or non-porous which may be used to form a novel affinity ligand-matrix conjugate according to the invention and which provides a convenient means of separating the affinity ligand from solutes in a contacting solution.

Also included within the definition of support matrices whose residue is represented by M are support matrices such as agarose, cellulose, dextran, starch, alginate, carrageenan, synthetic polymers, silica, glass and metal oxides which have been, or are, modified by treatment with an activating agent prior to, or during, attachment of the ligand.

In a preferred embodiment of the invention M represents optionally activated agarose, silica, cellulose, glass, toyopearl, hydroxyethylmethacrylate, polyacrylamide, styrenedivinylbenzene, Hyper D, perfluorocarbons.

Preferably M represents optionally tresyl activated, sulphonylchloride activated, tosyl activated, vinylsulphone activated or epoxy activated agarose.

Preferred affinity ligand matrix conjugates according to the invention are

i)

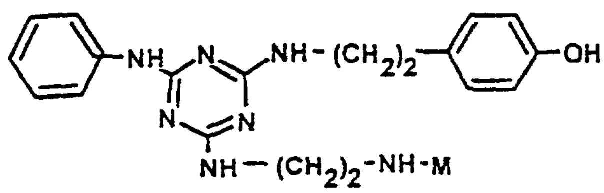

ii)

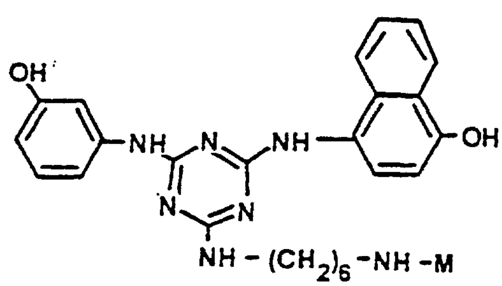

iii)

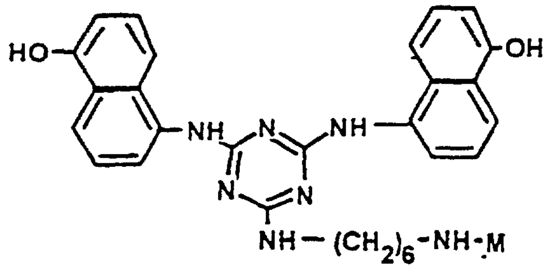

iv)

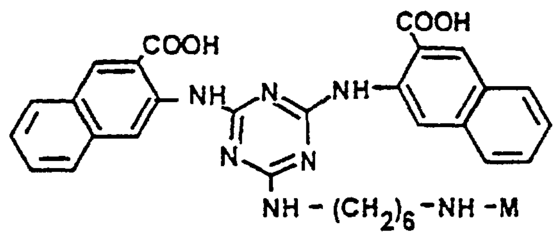

v)

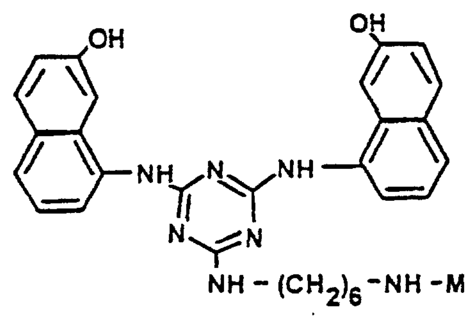

vi)

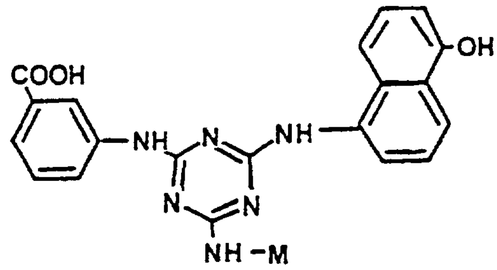

vii)

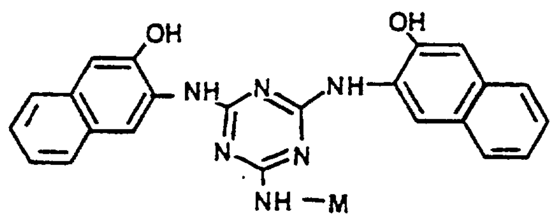

viii)

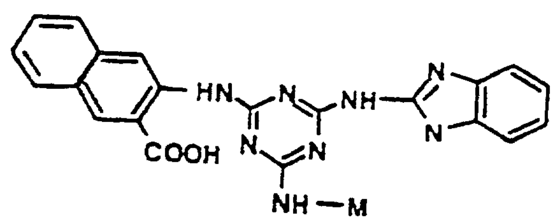

wherein M is as defined above.

There exists a considerable number of activating agents which have found use for the general purpose of attaching ligands to support matrices. These compounds and their method of use are well known to those skilled in the art and, since the nub of the present invention lies in the nature of the ligand attached to the matrix and not in the mode of attachment, any of these activating agents will serve in the preparation of the new matrix-ligand conjugates of the invention. As non-limiting examples of such activating agents there may be mentioned such diverse compounds as cyanogen bromide, cyanuric chloride, epichlorohydrin, divinyl sulphone, p-toluenesulphonyl chloride, 1,1'-carbonyldiimidazole, sodium meta-periodate, 2-fluoro-1-methylpyridiniumtoluene-4-sulphonate, glycidoxypropyltrimethoxysilane and 2,2,2-trifluoroethanesulphonyl chloride. As indicated above, the procedures by which such activating steps are carried out are well known to those skilled in the art.

Similarly, a wide variety of condensing agents may be used to attach the compounds of General Formulae (VI) to support matrices such as agarose, cellulose, dextran, starch, alginate, carrageenan, silica or glass. Again these compounds, and their method of use are well known to those skilled in the art and, again, since the nub of the present

invention lies in the nature of the ligand and not in the mode of attachment, any of these condensing agents will serve in the preparation of the new matrix-ligand conjugates of the invention. As non-limiting examples of such condensing agents, there may be mentioned N-ethoxycarbonyl-2-ethoxy-1,2-dihydroquinoline, dicyclohexyl carbodiimide and 1-ethyl-3-(3-dimethylaminopropyl)-carbodiimide.

As examples of linking units of General Formula (VI) which may be used to produce compounds of General Formula (VII) there may be mentioned diamines such as ethylene diamine, N,N'-dimethylethylene diamine, N-ethylethylene diamine, N-(β-hydroxyethyl)-ethylene diamine, propylene diamine, N-methylpropylene diamine, N-(β-hydroxyethyl)-propylene diamine, 1,4-diaminobutane, 1,5-diaminopentane, 1,6-diaminohexane, 1,7-diaminoheptane, 1,8-diaminooctane, 1,9-diaminononane, 1,10-diaminodecane, 1,12-diaminododecane, piperazine, 3-hydroxy-1,5-diaminopentane, m- and p-phenylene diamine, m- and p-aminobenzylamine; amino alcohols such as ethanolamine, N-methylethanolamine, N-propylethanolamine, diethanolamine, 3-hydroxypropylamine, 2,3-dihydroxypropylamine, isopropanolamine, 5-aminopentan-1-ol and 6-aminohexan-1-ol; aminophenols such as o-, m- and p-aminophenol, aminocarboxylic acids such as glycine, N-methylglycine, 3- and 4-aminobutyric acid, 3-aminoisobutyric acid, 5-aminovaleric acid, 6-aminocaproic acid, 7-aminoheptanoic acid, m- and p-aminobenzoic acid; aminophosphonic acids such as m-aminobenzenephosphonic acid and p-aminobenzylphosphonic acid; and aminoarylene vinylsulphone precursors such as aniline-3-β-sulphatoethylsulphone and aniline-4-β-sulphatoethylsulphone.

The reaction of halogenoheterocyclic compounds of General Formula (II) with compounds of General Formulae (III), (IV) and (V) or (VI) may be carried out in an organic solvent which is not miscible with water; or in an organic solvent which is miscible with water, or in a mixture of water and a water miscible organic solvent. Examples of suitable organic solvents which are not miscible with water are toluene, xylene or chlorobenzene; Examples of suitable organic solvents which are miscible with water are acetone, methyl ethyl ketone or dioxan. The first reaction of the halogenoheterocyclic compound may be carried out at temperatures between 0°C and 25°C, ideally between 0°C and 5°C; the second reaction may be carried out at temperatures between 20°C and 50°C, ideally between 30°C and 45°C and the third reaction at temperatures between 20°C and 100°C. During such reactions, the inorganic acid such as hydrochloric acid or hydrofluoric acid which is produced is neutralised by the use of an acid binding agent such as sodium hydroxide, sodium carbonate, sodium bicarbonate, calcium hydroxide or calcium carbonate.

Additionally, compounds of General Formula (VII) may be reacted with a reactive polymerisable monomer to form a polymerisable compound of General Formula (VIII):

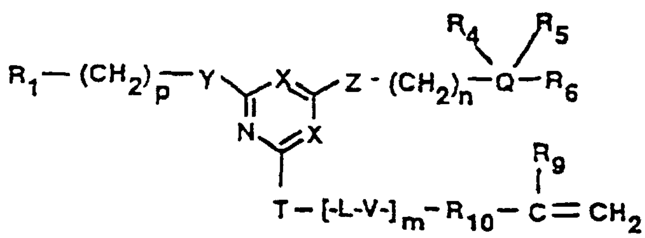

(VIII)

wherein $R_1$, $R_4$, $R_5$, $R_6$, Q, L, T, V, X, Y, Z, m, n and p have the meanings hereinbefore specified; $R_9$ represents a hydrogen atom or an alkyl group containing from 1 to 6 carbon atoms; $R_{10}$ represents a carbonyl group, a methylene group, a $-NH-CH_2-$ group or a $-S-CH_2-$ group. Examples of reactive polymerisable monomers include acryloyl chloride, methacryloyl chloride, allyl bromide, allylamine or 3,4-epoxybutene. Polymerisable compounds of General Formula (VIII) may be polymerised, optionally in the presence of other polymerisable monomers, to form affinity ligand matrix conjugates of General Formula (I). Such polymerisation procedures are well known to those skilled in the art.

The invention further covers the use of all such affinity ligand-support matrices in the separation, isolation, purification, quantification, identification and characterisation of proteinaceous materials, such as immunoglobulins, insulins, Factor VII, or Human Growth Hormone or analogues, derivatives and fragments thereof and precursors.

Immunoglobulins are a family of proteins, often abbreviated as $I_g$, which share a common structure. Immunoglobulins are also known collectively as antibodies and either word may be used to describe this group of proteins. Immunoglobulins exist in a number of different forms, for example, the most significant antibody types being $I_gA$, $I_gD$, $I_gE$, $I_gG$, $I_gM$ and $I_gY$ and various subclasses thereof. Immunoglobulins may occur in body fluids, such as plasma, ascities, saliva, milk or egg yolk or may be produced using genetic engineering methodologies. Immunoglobulins may be altered by a variety of techniques to confer desirable properties upon them. Such procedures are well known to those skilled in the art and the resulting modified antibodies are also subject to the Claims of this invention. As non-limiting Examples of antibody modification techniques, antibody fragments, labelled antibodies, antibody conjugates

or antibody-fusion proteins may be obtained through chemical modification, by treatment with one or more enzymes or by a combination of both techniques. There exists a considerable number of chemical modification reagents and enzymes which have found use in antibody modification and these compounds and their use are well known to those skilled in the art. A further way of obtaining modified or novel antibodies is to produce them using genetic engineering methodologies. Such methodologies and their use are well known to those skilled in the art and may be used to produce, for example, antibody fragments or antibody-fusion products. Modified or novel antibodies derived by genetic engineering methodologies are also subject to the Claims of this invention.

A valuable group of affinity ligand-support matrices is represented by the General Formula (IX):

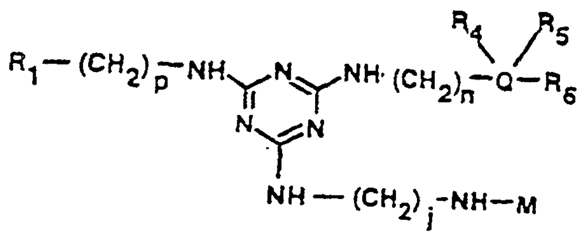

(IX)

wherein $R_1$, $R_4$, $R_5$, $R_6$, M, Q, n and p have the meanings hereinbefore specified and j is an integer between 2 and 20.

An especially valuable group of affinity ligand-support matrices is represented by the general Formula (X):

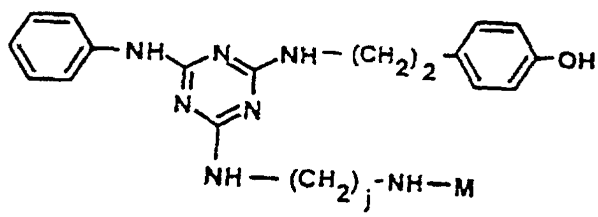

(X)

wherein j and M have the meanings hereinbefore specified.

Typically, reaction of compounds of General Formula (XI)

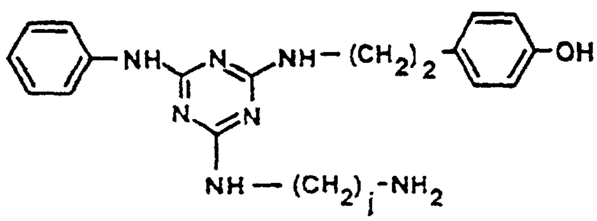

(XI)

with 3-propoxy-(1,2-epoxy)-agarose at temperatures between 10°C and 30°C in the presence of an acid binding agent produces novel affinity ligand-matrix conjugates which are of outstanding value in the purification of proteinaceous materials. These new affinity ligand-matrix conjugates possess high affinity for the immunoglobulin group of proteins. This unique property makes them of exceptional value in the separation, isolation, purification quantification, identification and characterisation of proteins of this class.

In another embodiment the invention relates to novel affinity ligands of General Formula (XII):

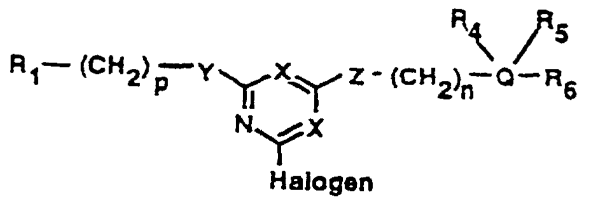

(XII)

wherein $R_1$, $R_4$, $R_5$, $R_6$, Q, X, Y, Z, n and p have the meanings specified above and Halogen represents a fluorine, chlorine, bromine or iodine atom.

Furthermore, the invention relates to a method of attaching novel affinity ligands of General Formula (XII) as defined above to a matrix of General Formula (V) as defined above by reacting the novel affinity ligands with the matrix at temperatures between -20°C and 121°C, optionally in the presence of an acid binding agent.

In another embodiment the invention relates to novel affinity ligands of General Formula (XIII):

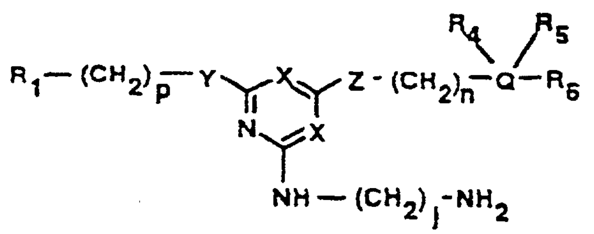

(XIII)

wherein $R_1$, $R_4$, $R_5$, $R_6$, Q, X, Y, Z, m, n and p have the meanings specified above and j is an integer between 2 and 20.

Furthermore the invention relates to a method of preparing above novel affinity ligands of General Formula (XIII) by reacting a compound of above General Formula (XII) with an alkylene diamine of General Formula $H_2N\text{-}(CH_2)_j\text{-}NH_2$ at temperatures between 0°C and 100°C in the presence of an acid binding agent.

In another embodiment the invention relates to novel affinity ligands of General Formula (XIV):

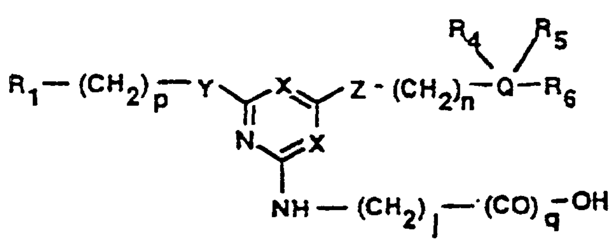

(XIV)

wherein $R_1$, $R_4$, $R_5$, Q, X, Y, Z, m, n and p have the meanings specified above, q is 0 or 1 and j is an integer between 2 and 20.

Furhermore the invention relates to a method of preparing novel affinity ligands of above General Formula (XIV) by reacting a compound of above General Formula (XII) with an amino hydroxy compound of General Formula $H_2N\text{-}(CH_2)_j\text{-}(CO)_q\text{-}OH$ at temperatures between 0°C and 100°C, optionally in the presence of an acid binding agent.

In another embodiment the invention relates to novel affinity ligands of above General Formula (VIII) wherein $R_1$, $R_4$, $R_5$, $R_6$, L, Q, T, V, X, Y, Z, m, n and p have the meanings specified above; $R_9$ represents a hydrogen atom or an alkyl group containing from 1 to 6 carbon atoms; $R_{10}$ represents a carbonyl group, a methylene group, an $-NH-CH_2-$ group or an $-S-CH_2-$ group; preferably L is an ethyl, butyl or hexyl group, preferably T represents a -NH- group, preferably V represents a -NH- group and m is prefably 1.

In another embodiment the invention relates to novel affinity ligands of General Formula (XV):

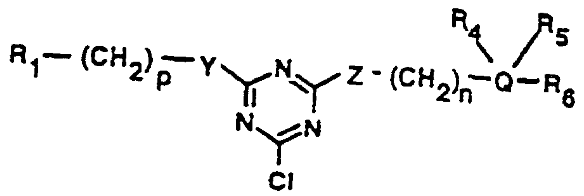

(XV)

wherein $R_1$, $R_4$, $R_5$, $R_{6,}$, Q, n and p have the meanings specified above.

In another embodiment the invention relates to novel affinity ligands of General Formula (XVI):

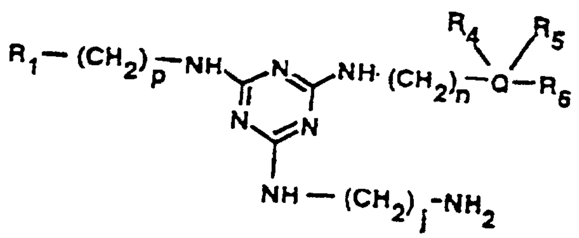

(XVI)

wherein $R_1$, $R_4$, $R_5$, $R_6$, Q, n and p have the meanings specified above and j is an integer between 2 and 20..

In another preferred embodiment the invention relates to affinity ligands of General Formula (XII), (XIII), (XIV), (VIII), (XV) and (XVI) wherein $R_1$ represents a phenyl or naphthyl group each of which is optionally substituted on the benzene or naphthalene ring with one or more independently selected from hydroxyl groups or carboxylic acid groups.

In another preferred embodiment the invention relates to affinity ligands of General Formula (XII), (XIII), (XIV), (VIII), (XV) and (XVI) wherein $R_4$ represents a hydrogen atom, a hydroxyl group, a carboxylic acid group or an amino group.

In another preferred embodiment the invention relates to affinity ligands of General Formula (XII), (XIII), (XIV), (VIII), (XV) and (XVI) wherein $R_5$ represents a hydrogen atom, a hydroxyl group, a carboxylic acid group or an amino group

In another preferred embodiment the invention relates to affinity ligands of General Formula (XII), (XIII), (XIV), (VIII), (XV) and (XVI) wherein $R_6$ represents a hydrogen atom, a hydroxyl group, a carboxylic acid group or an amino group.

In another preferred embodiment the invention relates to affinity ligands of General Formula (XII), (XIII), (XIV), (VIII), (XV) and (XVI) wherein Q represents a benzene or naphthalene ring.

In another preferred embodiment the invention relates to affinity ligands of General Formula (XII), (XIII), (XIV), and (VIII) wherein X represents a nitrogen atom.

In another preferred embodiment the invention relates to affinity ligands of General Formula (XII), (XIII), (XIV), and (VIII) wherein Y represents a -NH- group.

In another preferred embodiment the invention relates to affinity ligands of General Formula (XII), (XIII), (XIV), and (VIII) wherein Z represents a -NH- group.

In another preferred embodiment the invention relates to affinity ligands of General Formula (XII), (XIII), (XIV), (VIII), (XV) and (XVI) wherein n is 0 or 2.

In another preferred embodiment the invention relates to affinity ligands of General Formula (XII), (XIII), (XIV), (VIII), (XV) and (XVI) wherein p is 0 or 2.

In another preferred embodiment the invention relates to affinity ligands of General Formula (XIII), (XIV) and (XVI) wherein j is 2, 4 or 6.

In another embodiment the invention relates to affinity ligands of above General Formula (XI) wherein j is an integer betweem 2 and 20.

Preferred affinity ligands according to the invention are:

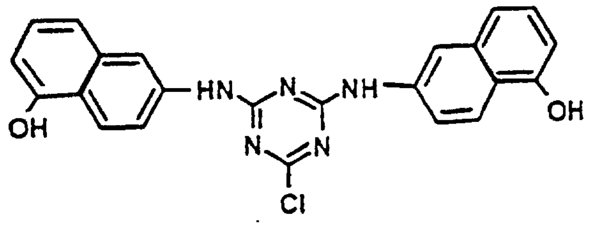

(1)

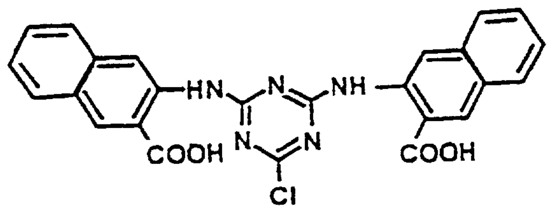

(2)

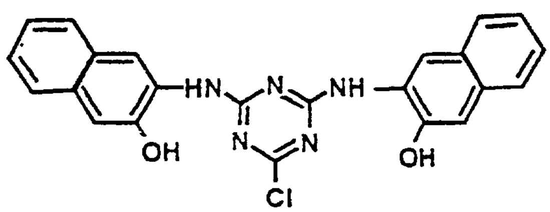

(3)

(4)

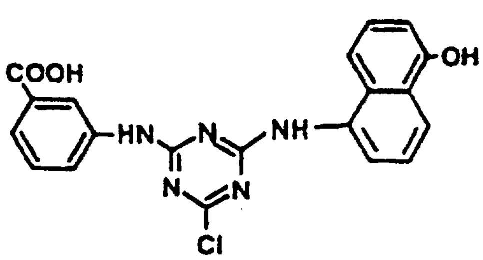

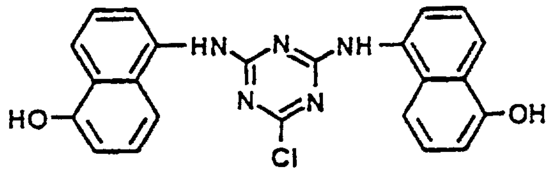

(5)

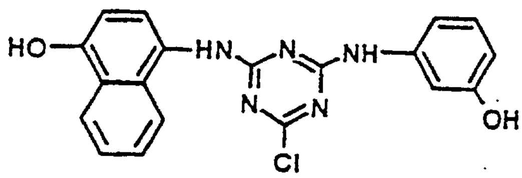

(6)

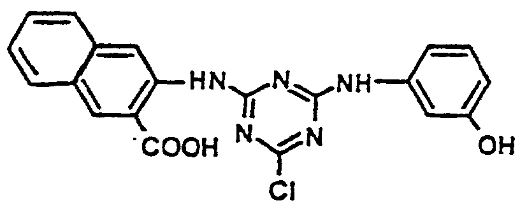

(7)

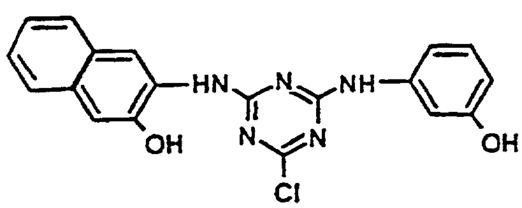

(8)

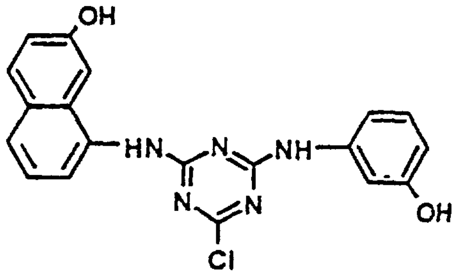

(9)

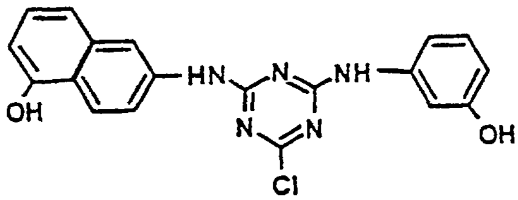

(10)

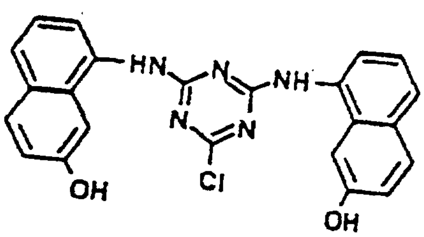

(11)

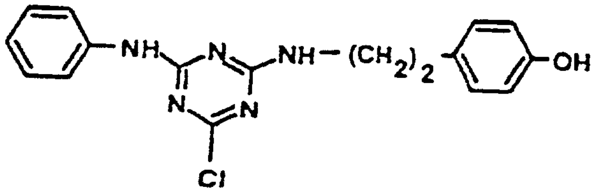

(12)

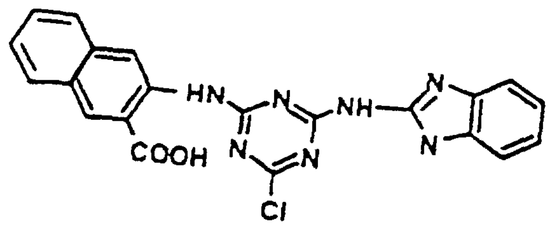

(13)

Furthermore the invention relates to a method of attaching the novel affinity ligands of General Formulae (VII) as defined above, (XIII) as defined above, (XVI) as defined above and (XI) as defined above to carbohydrate or organic polymer matrices by reacting the carbohydrate or organic polymer matrix with an activating agent followed by reaction of the activated matrix with the novel affinity ligand, optionally in the presence of an acid binding agent. The invention also relates to a method of attaching the novel affinity ligands of General Formulae (XIV) as defined above to carbohydrate or organic polymer matrices by condensation with the matrix. The invention furthermore relates to a method of attaching the novel affinity ligands of General Formulae (VII) as defined above, (XIII) as defined above, (XVI) as defined above and (XI) as defined above to metal oxide, glass or silica matrices, optionally coated with an organic polymer by reacting the optionally coated metal oxide, glass or silica matrix with an activating agent followed by reaction of the activated matrix with the novel affinity ligand, optionally in the presence of an acid binding agent. Another embodiment of the invention relates to a method of attaching the novel affinity ligands of General Formulae (XIV) as defined above to metal oxide, glass or silica matrices, optionally coated with an organic polymer by condensation with the matrix. In another embodiment the invention realates to a method of attaching novel affinity ligands of General Formula (XV) as defined above and (XII) as defined in above to a matrix of General Formula (V) as defined above by reacting the novel affinity ligands with the matrix at temperatures between -20°C and 121°C, optionally in the presence of an acid binding agent. The invention also relates to all the affinity ligand-matrix conjugates, prepared as described in the above methods.

In another embodiment the invention relates to the use of the affinity ligands according to the invention and the affinity ligand-matrix conjugates comprising such ligands according to the invention for the separation, isolation, purification, characterisation, identification or quantification of proteins or proteinaceous materials, such as immunoglobulins or subclasses, fragments, precursors or derivatives thereof, whether derived from natural or recombinant sources e.g. immunoglobulin G (IgG), immunoglobulin M (IgM), immunoglobulin A (IgA) or subclasses, fragments, precursors or derivatives thereof, whether derived from natural or recombinant sources. In another embodiment the invention relates to the separation, isolation, purification, characterisation, identification or quantification of immunoglobulins by any process whereby the said immunoglobulins are applied to affinity ligand-matrix conjugates according to the invention at a pH in the range 5.0 to 12.0 and subsequently removed, eluted or desorbed by reducing the pH to 4.9 or lower.

The invention also relates to a process for the separation or purification of proteinaceous materials comprising carrying out affinity chromatography using as the biospecific ligand a ligand of general formula (a) as defined above.

Another embodiment of the invention relates to the use of the affinity ligands according to the invention and the affinity ligand-matrix conjugates comprising such ligands according to the invention for the separation, isolation, purification, characterisation, identification or quantification of insulins and analogues, derivatives and fragments thereof and precursors, whether derived from natural or recombinant sources and Factor VII, or human Growth Hormone or analogues, derivatives and fragments thereof and precursors.

By way of example, a library comprising more than 60 different ligands were designed. The library was screened with partly purified insulin precursor and selected ligands were immobilised by solid phase synthesis to agarose. The matrices were optimized with respect to coupling technology, length and nature of spacer arm, ligand density etc. resulting in 3 prototypes of dynamic binding capacity of 2-5 mg/ml. A recombinantly derived insulin precursor was purified to more than 95% purity from clarified yeast fermentation broth by a single mimetic affinity purification step under very mild conditions.

An especially preferred use of the affinity ligands according to the invention and the affinity ligand-matrix conjugates comprising such ligands according to the invention is thus the purification of insulins and analogues, derivatives and fragments thereof and precursors, whether derived from natural or recombinant sources. Especially preferred affinity ligand-matrix conjugates for this use comprises a ligand selected among the following:

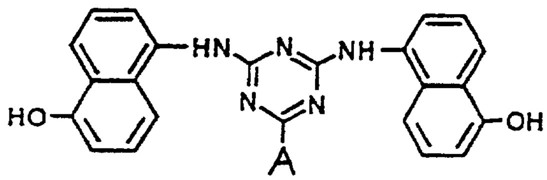

(5a)

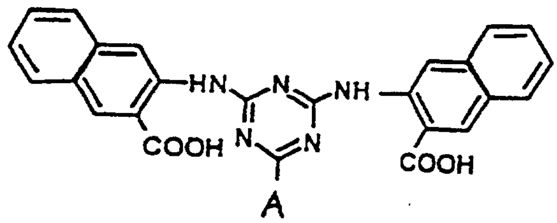

(2a)

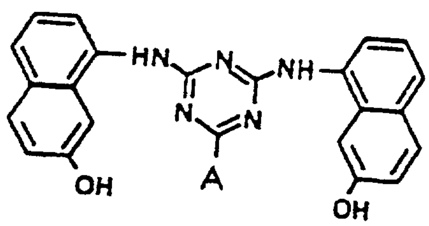

(11a)

which ligand is attached to a support matrix in position A as specified above, optionally through a spacerarm interposed between the matrix and ligand represented by the general formula (b) as defined above. Preferably, the ligand is 11a, and the support matrix is preferably optionally activated agarose, cellulose, silica or glass.

In another embodiment the invention relates to the separation, isolation, purification, characterisation, identification or quantification of insulins or insulin analogues or derivatives thereof and precursors by any process whereby the said insulins, insulin derivatives, analogues, and precursors are applied to affinity ligand-matrix conjugates according to the invention at a pH in the range 4,0 to 9,0 and subsequently removed, eluted or desorbed by reducing the pH to 3,99 or lower or to 9,01 or higher. The elution procedure can e.g. alternatively be carried out by lowering the ionic strength or by addition of cosolvents such as organic solvents.

The invention will now be described in further detail with reference to the following examples. The examples are provided for illustrative purposes, and are not to be construed as limiting the scope of the invention in any way.

**Example 1**

This Example illustrates the synthesis of a typical affinity ligand defined by the reaction of halogenoheterocyclic compounds of General Formula (II) with compounds of General Formula (III) and (IV).

19.8 parts of aniline were dissolved in 50 parts of acetone and the solution run into a suspension formed by pouring a solution of 36.8 parts of cyanuric chloride in parts of 200 parts of acetone into a mixture of 50 parts of ice and 50 parts of water. The mixture was stirred for 2 hours during which time the pH was maintained at between 6 and 7 by the addition of a solution of 16.8 parts of sodium hydrogen carbonate in 300 parts of water. At the end of this time,

the precipitate of 2-anilino-4,6-dichloro-1,3,5-triazine was filtered off, washed with water, dried in vauco and crystallised from dichloromethane.

A solution of 2.74 parts of tyramine in a mixture of 50 parts of acetone and 10 parts of water was added to a solution of 4.82 parts of 2-anilino-4,6-dichloro-1,3,5-triazine in 100 parts of acetone. The mixture was heated at 50°C and held at this temperature for 2 hours whilst the pH was maintained between 6 and 7 by the addition of a solution of 1.68 parts of sodium hydrogen carbonate in 30 parts of water. At the end of this time the precipitate of 2-anilino-4-[β-(4'-hydroxyphenyl)-ethylamino]-6-chloro-1,3,5-triazine was filtered off, washed with water and dried in vacuo.

**Example 2**

This Example illustrates the immobilisation of the product of Example 1 onto a solid phase support.

Ten parts of agarose bearing amino groups was transferred into an acetone solvent by solvent exchange using 100 parts of 30% aqueous acetone followed by 100 parts of 70% aqueous acetone and then 100 parts of acetone. One part of 2-anilino-4-[-β-(4'-hydroxyphenyl)-ethylamino]-6-chloro-1,3,5-triazine was dissolved in 20 parts of acetone and warmed to 50°C. This warm acetone solution was added to the acetone suspension of an agarose derivative bearing amino groups. A solution of 0.42 parts of sodium hydrogen carbonate in 5 parts of water was added to the resultant suspension and the mixture then stirred for 16 hours at 50°C. At the end of this time, the agarose support matrix was washed free of non-bonded 2-anilino-4-[-β-(4'-hydroxyphenyl)-ethylamino]-6-chloro-1,3,5-triazine with acetone. The resulting derivatised affinity support was then transferred back to water by washing first with 70% aqueous acetone, then with 30% aqueous acetone and finally with water.

**Example 3**

This Example demonstrates the use of the novel affinity matrix described in Example 2 as a specific chromatography matrix for immunoglobulins.

One and one quarter parts of human plasma was diluted with 3.75 parts of aqueous phosphate buffer having a pH of 7 and a concentration of 10 millimoles per litre and applied to a column consisting of 1 part of affinity matrix whose preparation is described in Example 2. The affinity column was then washed with phosphate buffer until UV monitoring of the wash liquors showed that all non-bound protein had been removed. The affinity matrix was then washed with a citrate buffer having a pH of 3.8 and a concentration of 0.2 millimoles per litre until UV monitoring of the wash liquor showed that removal of bound protein had ceased. The protein contained in this wash liquor was shown to be immunoglobulin G.

Table 1 gives further Examples of novel affinity ligands of the invention which may be prepared by the method of Example 1 but replacing the 36.8 parts of cyanuric chloride used in Example 1 by the corresponding amount of the halogenoheterocyclic compound listed in Column II of the Table; replacing the 19.8 parts of aniline used in Example 1 by the corresponding amount of the amine listed in Column III of the Table and replacing the 2.74 parts of tyramine used in Example 1 by the corresponding amount of the amine listed in Column IV of the Table. The number of the Example is given in Column I of the Table.

Table 1

| I | II | III | IV |
|---|---|---|---|
| 4 | 5-cyano-2,4,6-trichloropyrimidine | aniline | tyramine |
| 5 | 5-chloro-2,4,6-trifluoropyrimidine | aniline | tyramine |
| 6 | cyanuric chloride | tyramine | tyramine |
| 7 | cyanuric chloride | aniline | 4-hydroxy-benzylamine |
| 8 | cyanuric chloride | aniline | benzylamine |
| 9 | cyanuric chloride | N-methyl-aniline | 4-hydroxy-benzylamine |
| 10 | cyanuric chloride | p-anisidine | p-aminophenol |
| 11 | cyanuric chloride | aniline | 4-acetylamino-benzylamine |
| 12 | cyanuric chloride | p-toluidine | tyramine |
| 13 | cyanuric chloride | p-chloro-aniline | tyramine |
| 14 | cyanuric chloride | p-aminophenol | tyramine |

Table 1 (continued)

| I | II | III | IV |
|---|---|---|---|
| 15 | cyanuric chloride | cyclohexyl-amine | tyramine |
| 16 | cyanuric chloride | β-phenyl-ethylamine | tyramine |
| 17 | cyanuric chloride | 4-hydroxy-benzylamine | tyramine |
| 18 | cyanuric chloride | 4-hydroxy-benzylamine | 4-hydroxy-benzylamine |
| 19 | cyanuric chloride | benzylamine | tyramine |
| 20 | cyanuric chloride | tyramine | 3-methylsulphonyl-aniline |
| 21 | cyanuric chloride | N-tert-butyl-benzylamine | tyramine |
| 22 | cyanuric chloride | N-isopropyl-benzylamine | tyramine |
| 23 | cyanuric chloride | p-amino-acetanilide | tyramine |
| 24 | cyanuric chloride | di-isopropyl-amine | tyramine |
| 25 | cyanuric chloride | aniline | N-benzyltyramine |
| 26 | cyanuric chloride | tyramine | N-benzyltyramine |
| 27 | cyanuric chloride | p-amino-benzamide | tyramine |
| 28 | cyanuric chloride | p-aminobenzene sulphonamide | tyramine |
| 29 | cyanuric chloride | p-amino-benzoic acid | tyramine |
| 30 | cyanuric chloride | p-amino-benzoic acid | 1-amino-4-naphthol |
| 31 | cyanuric chloride | p-aminophenol | 1-amino-5-naphthol |
| 32 | cyanuric chloride | p-amino-benzoic acid | 1-amino-5-naphthol |
| 33 | cyanuric chloride | p-aminophenol | p-aminophenol |
| 34 | cyanuric chloride | m-amino-benzoic acid | aniline |
| 35 | cyanuric chloride | m-amino-benzoic acid | tyramine |
| 36 | cyanuric chloride | 1-amino-5-naphthol | 1-amino-5-naphthol |
| 37 | cyanuric chloride | m-aminophenol | 1-amino-5-naphthol |
| 38 | cyanuric chloride | 1-amino-4-naphthol | 1-amino-5-naphthol |

These ligands described in Examples 4-38 may be attached to an agarose matrix by the procedure detailed in Example 2 and used in the purification of immunoglobulin G by the procedure given in Example 3.

**Example 39**

6 Parts of ethylene diamine were added to a solution of 2.5 parts of 2-anilino-4-[β-(4'-hydroxyphenyl)-ethylamino]-6-chloro-1,3,5-triazine in 20 parts of toluene and the mixture heated at 100°C for 16 hours. The toluene was then evaporated off from the mixture under reduced pressure and the residue washed 5 times with 100 parts of water and then dried at 70°C.

Table 2 gives further Examples of novel affimity ligands of the invention which may be prepared by the method of Example 39 but replacing the 2.5 parts of 2-anilino-4-[β-(4'hydroxyphenyl)-ethylamino]-6-chloro-1,3,5-triazine used in Example 39 by the corresponding amount of the chlorotriazine listed in Column II of Table 2 and replacing the ethylene diamine used in Example 39 by the corresponding amount of the diamine or amino hydroxy compound listed in Column III of Table 2.

Table 2

| I | II | III |
|---|---|---|
| 40 | Chlorotriazine described in Example 1 | propylene diamine |

Table 2 (continued)

| I | II | III |
|---|---|---|
| 41 | Chlorotriazine described in Example 1 | 1,6-diaminohexane |
| 42 | Chlorotriazine described in Example 1 | 1,8-diaminooctane |
| 43 | Chlorotriazine described in Example 1 | 1,9-diaminononane |
| 44 | Chlorotriazine described in Example 1 | 1,10-diaminodecane |
| 45 | Chlorotriazine described in Example 1 | 1,12-diaminododecane |
| 46 | Chlorotriazine described in Example 14 | 1,6-diaminohexane |
| 47 | Chlorotriazine described in Example 30 | 1,6-diaminohexane |
| 48 | Chlorotriazine described in Example 31 | 1,6-diaminohexane |
| 49 | Chlorotriazine described in Example 33 | 1,6-diaminohexane |
| 50 | Chlorotriazine described in Example 35 | 1,6-diaminohexane |
| 51 | Chlorotriazine described in Example 38 | 1,6-diaminohexane |
| 52 | Chlorotriazine described in Example 1 | N,N'-dimethylethylene diamine |
| 53 | Chlorotriazine described in Example 1 | N-β-hydroxyethylethylene diamine |
| 54 | Chlorotriazine described in Example 1 | piperazine |
| 55 | Chlorotriazine described in Example 1 | ethanolamine |
| 56 | Chlorotriazine described in Example 1 | diethanolamine |
| 57 | Chlorotriazine described in Example 1 | isopropanolamine |

**Example 58**

This Example illustrates the immobilisation of the compound produced in Example 39 onto a solid phase support.

Sixty parts of agarose bearing epoxide groups was washed with 300 parts of water followed by 300 parts of a solution consisting of 5.95 parts of potassium hydrogen carbonate dissolved in 180 parts of methanol and 120 parts of water. One part of the compound prepared according to Example 39 was dissolved in 60 parts of methanol and added to a solution of 40 parts of aqueous potassium hydrogen carbonate solution. One hundred parts of this aqueous methanol solution was then added to the prepared suspension of 60 parts of agarose bearing epoxy groups and the resulting suspension gently agitated at ambient room temperature for 16 hours. The agarose based matrix obtained was washed first with a mixture of 360 parts of methanol and 240 parts of water, and then with 600 parts of water.

**Example 59**

If Example 3 is repeated but replacing the column consisting of 1 part of affinity matrix whose preparation is described in Example 2 by a column consisting of 1 part of affinity matrix whose preparation is described in Example 58. The protein contained in the pH 3.8 wash liquor was again shown to be immunoglobulin G.

**Example 60**

This Example illustrates the immobilisation of the product obtained in Example 39 onto a solid phase support.

Eighty parts of agarose was washed with 1050 parts of water followed by 1050 parts of 30% aqueous acetone, then 1050 parts of 70% aqueous acetone and finally 1425 parts of acetone. The eighty parts of agarose were then suspended in 100 parts of acetone and the suspension warmed to 37°C. Fifteen parts of p-toluenesulphonyl chloride dissolved in 15 parts of pyridine and 25 parts of acetone was added to the agarose suspension and the mixture kept at 37°C for 8 hours. The activated agarose was then washed with 225 parts of acetone followed by 225 parts of 70% aqueous acetone, then 225 parts of 30% aqueous acetone and, finally, 225 parts of water.

Fortyfive parts of this activated matrix was washed with 225 parts of a solution consisting of 2.3 parts of potassium hydrogen carbonate dissolved in a mixture of 178 parts of methanol and 47 parts of water. A solution of

0.75 parts of the compound prepared according to Example 39 in a mixture of 45 parts of methanol and 30 parts of water containing 1.5 parts of potassium hydrogen carbonate was added to the activated agarose suspension and the mixture then left to stand for 16 hours at room temperature. The resulting affinity matrix was washed with 270 parts of methanol and 180 parts of water containing 9 parts of potassium hydrogen carbonate, followed by 450 parts of water.

Prior to use, 115 parts of the resulting affinity matrix was suspended in a solution of 0.36 parts of sodium hydroxide in 45 parts of water.

**Example 61**

If Example 3 is repeated but replacing the column consisting of 1 part of the affinity matrix whose preparation is described in Example 2 by a column consisting of 1 part of the affinity matrix whose preparation is described in Example 60, the protein contained in the pH 3.8 wash liquor was again shown to be immunoglobulin G.

**Example 62**

Ten parts of the affinity matrix prepared according to Example 60 was allowed to stand in a phosphate buffer having a pH of 8.0 for 16 hours. Example 3 was repeated but replacing the matrix used therein with an equivalent amount of this new affinity matrix and using cell free supernatant from murine immunoglobulin cell culture medium in place of the human plasma used in Example 3. The protein contained in the pH 3.8 wash liquor was again shown to be murine immunoglobulin M.

Table 3 gives further Examples of the attachment of the novel affinity ligands of the invention which may be prepared by the method of Example 58 but replacing the novel affinity ligand used in Example 58 by the corresponding amount of the novel affinity ligand specified in Column II of Table 3 and by replacing the 39 parts of epichlorohydrin activated agarose used in Example 58 by the corresponding amount of the carbohydrate listed in Column III of Table 3 activated by the reagent listed in Column IV of Table 3.

Table 3

| I | II | III | IV |
|---|---|---|---|
| 61 | Ligand of Example 39 | agarose | 1,4-butanediol diglycidyl ether |
| 62 | Ligand of Example 39 | agarose | cyanogen bromide |
| 63 | Ligand of Example 39 | agarose | epichlorohydrin |
| 64 | Ligand of Example 39 | agarose | sodium metaperiodate |
| 65 | Ligand of Example 39 | agarose | 2,2,2-trifluoroethanesulphonylchloride |
| 66 | Ligand of Example 39 | agarose | 1,1'-carbonyldiimidazole |
| 67 | Ligand of Example 39 | agarose | 2-fluoro-1-methyl-pyridinium toluene-4-sulphonate |
| 68 | Ligand of Example 39 | agarose | divinyl sulphone |
| 69 | Ligand of Example 39 | agarose | cyanuric chloride |

**Example 70**

If the preparation of the ligand detailed in Example 1 is repeated but reacting the 2.74 parts of tyramine with 4.84 parts of 2-phenoxy-4,6-dichloro-1,3,5-triazine instead of 4.82 parts of 2-anilino-4,6-dichloro-1,3,5-triazine, there is obtained 2-phenoxy-4-[β-(4'-hydroxyphenyl)-ethylamino]-6-chloro-1,3,5-triazine, which may be used in place of 2-anilino-4-[β-(4'-hydroxyphenyl)-ethylamino]-6-chloro-1,3,5-triazine in the preparation of an affinity matrix by the method of Example 2. Again, immunoglobulin G may be conveniently isolated from human plasma by the technique described in Example 3.

**Example 71**

Use of 5.16 parts of 2-phenylthio-4,6-dichloro-1,3,5-triazine in place of the 4.84 parts of 2-phenoxy-4,6-dichloro-1,3,5-triazine used in Example 70 gives 2-phenylthio-4-[β-(4'-hydroxyphenyl)-ethylamino]-6-chloro-1,3,5-triazine, which may be similarly used to purify immunoglobulin G.

**Example 72**

This Example illustrates an alternative approach to producing affinity ligand-matrix conjugates as exemplified in Example 2 and Example 58.

Ten parts of agarose bearing aminoethylamino groups were stirred at 0-5°C with 5 parts of acetone and 5 parts of an aqueous phosphate buffer having a pH of 7 and a concentration of 0.5 moles per litre. To this suspension was added 2.5 parts of a solution comprising 1 part of cyanuric chloride in 10 parts of acetone and the mixture then agitated for an hour at a temperature of 0-5°C. The resulting dichlorotriazine activated agarose was washed sequentially with 50 parts of 50% aqueous acetone, 50 parts of water, 50 parts of 50% aqueous acetone and 100 parts of water. Twenty parts of the washed dichlorotriazine activated agarose was added to 20 parts of an aqueous solution comprising 1 part of tyramine in 200 parts of water and the resulting suspension agitated for 16 hours at ambient room temperature. At the end of this period, the resulting derivatised matrix was washed with 200 parts of water and added to 20 parts of an aqueous solution comprising 0.55 parts of aniline dissolved in 200 parts of water. The resulting suspension was agitated for 16 hours at 90°C after which time the derivatised matrix was washed with 200 parts of water.

**Example 73**

If Example 3 is repeated but replacing the column consisting of 1 part of the affinity matrix whose preparation is described in Example 2 by a column consisting of 1 part of the affinity matrix whose preparation is described in Example 72, the protein contained in the pH 3.8 wash liquor was again shown to be immunoglobulin G.

**Example 74**

This example illustrates the synthesis of a library of affinity ligand-matrix conjugates of this invention which may be subsequently screened to determine their protein binding properties.

One part of agarose bearing amino groups was mixed with 1 part 1 M potassium phosphate buffer, pH 7.0 and settled under gravity. The buffered amine agarose was transferred to a reaction vessel and mixed at 0-5°C with 0.5 parts 0.5M potassium phosphate buffer, pH 7.0 and 0.5 parts acetone. One quarter of a part of a solution comprising 1 part cyanuric chloride in 10 parts acetone was added and the mixture stirred at 0-5°C for 1 hour, after which the mixture was filtered and washed sequentially with 10 parts 50% acetone, 6 parts water, 6 parts 50% acetone and 10 parts water to provide 2,4-dichloro-s-triazin-6-yl activated agarose.

One part of the 2,4-dichloro-s-triazin-6-yl activated agarose was added to 5 mole equivalents of the amine compound listed in Column II of Table 4 dissolved in 2 to 3 parts of a solution comprising 1 part acetone and 1 part water and adjusted to neutral pH by addidtion of sodium hydroxide. The suspension was mixed for 24 hours at 30°C. The resulting monochloro-s-triazin-6-yl activated agaroses were washed sequentially with 10 parts of dimethylformamide, 10 parts of a solution comprising 3 parts propan-2-ol and 7 parts 0.2M sodium hydroxide, 10 parts of water and settled under gravity.

One part of the monochloro-s-triazin-6-yl-activated agarose was added to 5 mole equivalents of the amine compound listed in Column III of Table 4 dissolved in 2 to 3 parts of a solution comprising 1 part dimethylformamide and 1 part water and adjusted to neutral pH by addition of sodium hydroxide. The suspension was mixed for 72 hours at 85 to 95°C. The resulting affinity ligand-matrix conjugates were washed sequentially with 10 parts of dimethylformamide, 10 parts of a solution comprising 3 parts propan-2-ol and 7 parts 0.2M sodium hydroxide, 10 parts of water and settled under gravity. A library of affinity ligand-matrix conjugates of this Invention was synthesised, Examples of which are identified in Column I of Table 4.

Table 4

| I | II | III |
|---|---|---|
| 75 | 1,3-diaminopropane | tyramine |
| 76 | 1,3-diaminopropane | N-benzylphenethylamine |
| 77 | 1,4-diaminobutane | 3-aminophenol |
| 78 | 1,4-diaminobutane | tyramine |
| 79 | 1,4-diaminobutane | 1-amino-5-naphthol |
| 80 | 1,4-diaminobutane | N-isopropylbenzylamine |
| 81 | 1,4-diaminobutane | N-benzylphenethylamine |

Table 4 (continued)

| I | II | III |
|---|---|---|
| 82 | aniline | diaminopropane |
| 83 | aniline | aniline |
| 84 | aniline | 3-aminophenol |
| 85 | aniline | 3-aminobenzoic acid |
| 86 | aniline | tyramine |
| 87 | aniline | 1-amino-4-naphthol |
| 88 | aniline | 1-amino-2-naphthol |
| 89 | aniline | 3-amino-2-naphthol |
| 90 | aniline | 1-amino-6-naphthol |
| 91 | aniline | N-isopropylbenzylamine-4-phenylbutylamine |
| 92 | aniline | N-benzylphenethylamine |
| 93 | 3-aminophenol | 3-aminophenol |
| 94 | 3-aminophenol | 1-amino-5-naphthol |
| 95 | 1,6-diaminohexane | tyramine |
| 96 | 1,6-diaminohexane | 4-phenylbutylamine |
| 97 | 3-aminobenzoic acid | 1-amino-5-naphthol |
| 98 | tyramine | tyramine |
| 99 | tyramine | 1-amino-5-naphthol |
| 100 | tyramine | 1-amino-7-naphthol |
| 101 | tyramine | 1-amino-6-naphthol |
| 102 | 1-amino-5-naphthol | aniline |
| 103 | 1-amino-5-naphthol | 3-aminophenol |
| 104 | 1-amino-5-naphthol | 4-aminobenzoic acid |
| 105 | 1-amino-5-naphthol | 3,5-diaminobenzoic acid |
| 106 | 1-amino-5-naphthol | benzylamine |
| 107 | 1-amino-5-naphthol | tyramine |
| 108 | 1-amino-5-naphthol | 1-amino-5-naphthol |
| 109 | 1 -amino-7-naphthol | 3-aminophenol |
| 110 | 1-amino-7-naphthol | 1-amino-7-naphthol |
| 111 | 1-amino-4-naphthol | 3-aminophenol |
| 112 | 1-amino-4-naphthol | 1-amino-4-naphthol |
| 113 | 1-amino-2-naphthol | 3-aminophenol |
| 114 | 1-amino-2-naphthol | 1-amino-2-naphthol |
| 115 | 3-amino-2-naphthol | 3-aminophenol |
| 116 | 3-amino-2-naphthol | 3-amino-2-naphthol |
| 117 | 1-amino-6-naphthol | 3-aminophenol |
| 118 | 1 -amino-6-naphthol | 1 -amino-6-naphthol |
| 119 | 2-amino-1-naphthol | 3-aminophenol |

Table 4 (continued)

| I | II | III |
|---|---|---|
| 120 | 2-amino-1-naphthol | 2-amino-1-naphthol |
| 121 | 6-amino-1-naphthol | 3-aminophenol |
| 122 | 6-amino-1-naphthol | 6-amino-1-naphthol |
| 123 | 1-amino-6-naphthalenesulphonic acid | 3,5-diaminobenzoic acid |
| 124 | 1-amino-6-naphthalenesulphonic acid | benzylamine |
| 125 | 1-amino-6-naphthalenesulphonic acid | tyramine |
| 126 | 3-amino-2-napthoic acid | 3-aminophenol |
| 127 | 3-amino-2-napthoic acid | 3-amino-2-napthoic acid |

**Example 128**

This example demonstrates the screening process by which the protein binding properties of affinity ligand-matrix conjugates described in example 74 may be identified.

Chromatography columns of 1 ml total volume were packed with affinity ligand-matrix conjugates of Examples 75-127. The columns were equilibrated by flushing with 10 ml of 50 mM sodium phosphate buffer, pH 8.0. One ml of a solution comprising 1.5 mg of human IgG per ml of 50 mM sodium phosphate buffer, pH 8.0 was applied to each chromatography column which were subsequently washed with 10 ml of 50 mM sodium phosphate buffer, pH 8.0 to remove non-bound IgG. Bound IgG was eluted by flushing each column with 5 ml of 50 mM sodium citrate buffer, pH 3.0. The IgG content of the wash and elution fractions was determined by measurement of absorbance at 280 nm against a buffer blank. Analysis of the results revealed affinity ligand-matrix conjugates of Examples 75 to 127 all exhibited human IgG binding. Of these, almost quantitative elution of bound IgG was achieved for affinity ligand-matrix conjugates of Examples 92, 99, 101, 102, 103, 111, 113 and 119 which, as a consequence, are considered to be of exceptional value in the separation, isolation or purification of human IgG.

**Example 129**

**Selective binding and elution of Recombinant Coagulation Factor VIIa (rFVIIa) applied to the affinity matrix according to Example 181 from cell culture media.**

*Procedure:*

0.85 ml settled volume of the affinity matrix prepared as in example 181 was packed into a 5 by 50 mm column (Pharmacia HR 5/5 ) and equilibrated with 20 mL of buffer A: 20 mM Tris, 50 mM NaCl, 5 mM $CaCl_2$, pH 8.0.
5 ml of conditioned BHK Cell culture supernatant enriched with 1.4 mg rFVIIa was applied to the packed column.
After washing off non-binding proteins with 10 mL of buffer A, rFVIIa was eluted by applying 5 mL of buffer B: 20 mM Tri-Sodium-Citrate, 50 mM Tris pH 8.0.

The flow rate during the chromatographic procedure was 0.3 mL/ minute.

The column effluent was passed through an in line UV-monitor and collected in 1 mL fraction, and each fraction analyzed for the content of rFVIIa and total protein by analytical reversed phase High Performance Liquid Chromatography(RP-HPLC)

*Results:*

The UV- monitor out put showed that most of the UV (280nm) absorbing material came out during applying the supernatant and the following wash with buffer A. During the following elution with buffer B a distinct peak was monitored, that matched the expected size for the applied amount of rFVIIa.

The RP-HPLC analysis of the collected fractions showed that 90 % of the applied amount of rFVIIa came out in the fractions during elution with buffer B. The purity of rFVIIa in these fractions were above 95%.

The results show that selective binding of rFVIIa from enriched culture media to the used ligand is achieved.

**Example 130**

**Purification of insulin B-chain$^{1-29}$-A-A-K-insulin A-chain$^{1-21}$ on an affinity ligand-matrix conjugate according to Example 171:**

255 mg of insulin B-chain$^{1-29}$-Ala-Ala-Lys-insulin A-chain$^{1-21}$, (batch A202558) was suspended in 51 ml of $H_2O$. 10 drops of 1 M acetic acid were added to solubilize the precursor. 0.2 M potassium citrate pH 5.5 was added to a total volume of 510 ml resulting in a solution of 0.12 mg/ml (by RP-HPLC analysis). pH was measured to 5.53, the ionic strength to 30.0 mS/cm and the redox potential to 273 mV.
400 ml of the above solution was applied a Pharmacia K16 (1.6 x 6 cm) column packed with 12 ml of the above matrix conjugate, equilibrated in 0.2 M potassium citrate pH 5.5, at 1.8 ml/min at ambient temperature. The column was washed in 50 ml of 0.2 M potassium citrate pH 5.5 and then eluted with 0.1 M acetic acid at 1.8 ml/min. 12 fractions of 5.0 ml were collected.
The column was cleaned with 50 ml of 0.5 M NaOH and regenerated with 50 ml of 0.1 M citric acid, 60% v/v ethanol. Samples for RP-HPLC analysis were diluted with 2 M acetic acid prior to analysis.

| Sample | Identification | MI3 conc. | Volume | Amount | % |
|---|---|---|---|---|---|
| Application | E091b | 0.12 mg/ml | 400 ml | 48.8 mg | 100 |
| Run through | E091c | 0.00 mg/ml | | 0.0 mg | 0 |
| Wash | E093a | 0.00 mg/ml | | 0.0 mg | 0 |
| Fraction 3 | E093b | 0.01 mg/ml | 5.0 ml | 0.1 mg | |
| Fraction 4 | E093c | 5.00 mg/ml | 5.0 ml | 25.0 mg | |
| Fraction 5 | E093d | 2.62 mg/ml | 5.0 ml | 13.1 mg | |
| Fraction 6 | E093e | 0.52 mg/ml | 5.0 ml | 2.6 mg | |
| Fractions | | | | 40.8 mg | 83 |

Thus a total recovery of 83% was obtained.
The purity of the product was determined to 94% by RP-HPCL analysis. The remaining impurities were insulin related.

**Example 131**

**Purification of des-Thr$^{B30}$-insulin on an affinity ligand-matrix conjugate according to Example 171:**

150 mg of des-Thr$^{830}$-insulin (INS-J-009) precipitate was suspended in 50 ml $H_2O$. 5 drops of 2 M acetic acid were added to dissolve the suspension. 0.2 M of potassium citrate pH 5.5 was added to a volume of 500 ml resulting in a concentration of 0.076 mg/ml (by RP-HPLC analysis). pH was measured to 5.52, the ionic strength to 30.0 mS/cm and the redox potential to 264 mV. 400 ml of the above solution was applied a Pharmacia K16 (1.6 x 6 cm) column packed with 12 ml of the above matrix conjugate, equilibrated in 0.2 M potassium citrate pH 5.5, at 1.8 ml/min at ambient temperature. The column was washed in 50 ml of 0.2 M potassium citrate pH 5.5 and then eluted with 0.1 M acetic acid at 1.8 ml/min. 10 fractions of 5.0 ml were collected.
The column was cleaned with 50 ml of 0.5 M NaOH and regenerated with 50 ml of 0.1 M citric acid, 60% v/v ethanol. Samples for RP-HPLC analysis were diluted with 2 M acetic acid prior to analysis.

| Sample | Identification | MI3 conc. | Volume | Amount | % |
|---|---|---|---|---|---|
| Application | E105a | 0.076mg/ml | 400 ml | 30.0mg | 100 |
| Run through | E105b | 0.00 mg/ml | | 0.0 mg | 0 |
| Wash | E105d | 0.00 mg/ml | | 0.0 mg | 0 |
| Fraction 3 | E105e | 0.00 mg/ml | 5.0 ml | 0.0 mg | 0 |
| Fraction 4 | E105f | 0.41 mg/ml | 5.0 ml | 2.1 mg | |
| Fraction 5 | E105n | 1.93 mg/ml | 5.0 ml | 9.7 mg | |

(continued)

| Sample | Identification | MI3 conc. | Volume | Amount | % |
|---|---|---|---|---|---|
| Fraction 6 | E105o | 1.14 mg/ml | 5.0 ml | 5.7 mg | |
| Fraction 7 | E105p | 0.48 mg/ml | 5.0 ml | 2.4 mg | |
| Fraction 8 | E105j | 0.31 mg/ml | 5.0 ml | 1.6 mg | |
| Fractions | | | | 21.5 mg | 71% |

Thus a total recovery of 71 % was obtained.
The purity of the product was determined to 91 % by RP-HPCL analysis. the remaining impurities were insulin related.

**Example 132**

**Purification of insulin B-chain$^{1-29}$-A-A-K-insulin A-chain$^{1-21}$ on an affinity ligand-matrix conjugate according to Example 145:**

2 l of centrifuged broth (batch 628) was adjusted to pH 5.5 with 5 M NaOH and filtered through a Leitz Tiefenfilter (Seitz EK) filter followed by filtration through a Leitz Tiefenfilter (Seitz EKS) filter. The concentration was measured to 0.006 mg/ml by RP-HPLC. The ionic strength was measured to 12.2 mS/cm and the redox potential to 316 mV.
1000 ml of the above solution was applied a Pharmacia K16 (1.6 x 6 cm) column packed with 12 ml of the above matrix conjugate, equilibrated in 0.2 M potassium citrate pH 5.5, at 1.8 ml/min at ambient temperature. The column was washed in 50 ml of 0.2 M potassium citrate pH 5.5 and then eluted with 0.1 M acetic acid at 1.8 ml/min. 11 fractions of 5.0 ml were collected.
The column was cleaned with 50 ml of 0.5 M NaOH and regenerated with 50 ml of 0.1 M citric acid, 60% v/v ethanol. Samples for RP-HPLC analysis were diluted with 2 M acetic acid prior to analysis.

| Sample | Identification | MI3 conc. | Volume | Amount | % |
|---|---|---|---|---|---|
| Application | E111e | 0.006mg/ml | 1000 ml | 6.0mg | 100 |
| Run through | E115a | 0.00 mg/ml | | 0.0 mg | 0 |
| Wash | E115b | 0.00 mg/ml | | 0.0 mg | 0 |
| Fraction 3 | E115c | 0.00 mg/ml | 5.0 ml | 0.0 mg | 0 |
| Fraction 4 | E115d | 0.58 mg/ml | 5.0 ml | 2.9mg | |
| Fraction 5 | E115e | 0.11 mg/ml | 5.0 ml | 0.6 mg | |
| Fraction 6 | E115f | 0.04 mg/ml | 5.0 ml | 0.2 mg | |
| Fraction 7 | E115g | 0.02 mg/ml | 5.0 ml | 0.1 mg | |
| Fractions | | | | 3.8 mg | 63% |

Thus a total recovery of 63% was obtained.
The purity of the product was determined to 88% by RP-HPLC analysis. The remaining impurities were insulin related.

**Example 133**

**Purification of insulin B-chain$^{1-29}$-A-A-K-insulin A-chain$^{1-21}$ on an affinity ligand-matrix conjugate according to Example 171:**

2 l of centrifuged broth (batch 628) was adjusted to pH 5.5 with 5 M NaOH and filtered through a Leitz Tiefenfilter (Seitz EK) filter followed by filtration through a Leitz Tiefenfilter (Seitz EKS) filter. The concentration was measured to 0.006 mg/ml by RP-HPLC. The ionic strength was measured to 12.2 mS/cm and the redox potential to 316 mV.
1000 ml of the above solution was applied a Pharmacia K16 (1.6 x 6 cm) column packed with 12 ml of the above matrix

conjugate, equilibrated in 0.2 M potassium citrate pH 5.5, at 1.8 ml/min at ambient temperature. The column was washed in 50 ml of 0.2 M potassium citrate pH 5.5 and then eluted with 0.1 M acetic acid at 1.8 ml/min. 11 fractions of 5.0 ml were collected.
The column was cleaned with 50 ml of 0.5 M NaOH and regenerated with 50 ml of 0.1 M citric acid, 60% v/v ethanol. Samples for RP-HPLC analysis were diluted with 2 M acetic acid prior to analysis.

| Sample | Identification | MI3 conc. | Volume | Amount | % |
|---|---|---|---|---|---|
| Application | E111e | 0.006mg/ml | 830 ml | 5.0mg | 100 |
| Run through | E113a | 0.00 mg/ml | | 0.0 mg | 0 |
| Wash | E113b | 0.00 mg/ml | | 0.0 mg | 0 |
| Fraction 3 | E113c | 0.00 mg/ml | 5.0 ml | 0.0 mg | 0 |
| Fraction 4 | E113d | 0.58 mg/ml | 5.0 ml | 2.9mg | |
| Fraction 5 | E113e | 0.10 mg/ml | 5.0 ml | 0.5 mg | |
| Fraction 6 | E113f | 0.04 mg/ml | 5.0 ml | 0.2 mg | |
| Fraction 7 | E113g | 0.02 mg/ml | 5.0 ml | 0.1 mg | |
| Fractions | | | | 3.7 mg | 74% |

Thus a total recovery of 74% was obtained.
The purity of the product was determined to 86% by RP-HPLC analysis. The remaining impurities were insulin related.

**Example 134**

<u>**Purification of EEAEPK-insulin B-chain(1-29)-AAK-insulin A-chain(1-21) on an affinity ligand-matrix conjugate according to Example 171:**</u>

Centrifuged yeast broth (batch Y44) was filtered through a Leitz Tiefenfilter (Seitz EK) filter resulting in a concentration of 0.35 mg/ml (by RP-HPLC analysis). pH was measured to 5.27, the ionic strength to 7.38 mS/cm and the redox potential to 221 mV.
120 ml of the above solution was applied a Pharmacia K16 (1.6 x 6 cm) column packed with 12 ml of the above matrix conjugate, equilibrated in 0.2 M potassium citrate pH 5.5, at 1.8 ml/min at ambient temperature. The column was washed in 50 ml of 0.2 M potassium citrate pH 5.5 and then eluted with 0.1 M acetic acid at 1.8 ml/min. 11 fractions of 5.0 ml were collected.
The column was cleaned with 50 ml of 0.5 M NaOH and regenerated with 50 ml of 0.1 M citric acid, 60% v/v ethanol. Samples for RP-HPLC analysis were diluted with 2 M acetic acid prior to analysis.

| Sample | Identification | MI3 conc. | Volume | Amount | % |
|---|---|---|---|---|---|
| Application | E127b | 0.35 mg/ml | 120 ml | 42.0mg | 100 |
| Run through | E127c | 0.00 mg/ml | | 0.0 mg | 0 |
| Wash | E127d | 0.00 mg/ml | | 0.0 mg | 0 |
| Fraction 3 | E127e | 2.26 mg/ml | 5.0 ml | 11.3 mg | |
| Fraction 4 | E127f | 2.07 mg/ml | 5.0 ml | 10.4mg | |
| Fraction 5 | E127g | 1.12 mg/ml | 5.0 ml | 5.6 mg | |
| Fraction 6 | E127h | 1.27 mg/ml | 5.0 ml | 6.4 mg | |
| Fractions | | | | 33.6mg | 79% |

Thus a total recovery of 79% was obtained. The purity of the product was determined to 93% by RP-HPLC analysis. The remaining impurities were insulin related.

**Example 135**

**Purification of [Asp$^{B28}$]-insulin-B-chain$^{1-29}$-A-A-K-insulin-A-chain$^{1-21}$ on an affinity ligand-matrix conjugate according to Example 171:**

Centrifuged broth (batch GSG9414) was adjusted to pH 5.5 with 5 M NaOH and filtered through a Leitz Tiefenfilter (Seitz EK) filter followed by filtration through a Leitz Tiefenfilter (Seitz EKS) filter. The concentration was measured to 0.02 mg/ml by RP-HPLC. The ionic strength was measured to 17.0 mS/cm and the redox potential to 308 mV.
800 ml of the above solution was applied a Pharmacia K16 (1.6 x 6 cm) column packed with 12 ml of the above matrix conjugate, equilibrated in 0.2 M potassium citrate pH 5.5, at 1.8 ml/min at ambient temperature. The column was washed in 50 ml of 0.2 M potassium citrate pH 5.5 and then eluted with 0.1 M acetic acid at 1.8 ml/min. 12 fractions of 5.0 ml were collected.
The column was cleaned with 50 ml of 0.5 M NaOH and regenerated with 50 ml of 0.1 M citric acid, 60% v/v ethanol. Samples for RP-HPLC analysis were diluted with 2 M acetic acid prior to analysis.

| Sample | Identification | MI3 conc. | Volume | Amount | % |
|---|---|---|---|---|---|
| Application | E107b | 0.02mg/ml | 800 ml | 16.0mg | 100 |
| Run through | E107c | 0.00 mg/ml | | 0.0 mg | 0 |
| Wash | E107d | 0.00 mg/ml | | 0.0 mg | 0 |
| Fraction 2 | E109a | 0.00 mg/ml | 5.0 ml | 0.0 mg | 0 |
| Fraction 3 | E109b | 0.12 mg/ml | 5.0 ml | 0.6 mg | |
| Fraction 4 | E109c | 0.76 mg/ml | 5.0 ml | 3.8 mg | |
| Fraction 5 | E109d | 1.04 mg/ml | 5.0 ml | 5.2 mg | |
| Fraction 6 | E109e | 0.31 mg/ml | 5.0 ml | 1.6 mg | |
| Fraction 7 | E109f | 0.10 mg/ml | 5.0 ml | 0.5 mg | |
| Fraction 8 | E109g | 0.06 mg/ml | 5.0 ml | 0.3 mg | |
| Fractions | | | | 12 mg | 75% |

Thus a total recovery of 75% was obtained.
The purity of the product was determined to 84% by RP-HPLC analysis. The remaining impurities were insulin related.

**Example 136**

This example further demonstrates the screening process by which the protein binding properties of affinity ligand-matrix conjugates of this invention may be identified.
A library of affinity ligand matrix conjugates of this invention were synthesised according to example 74 except that the 5 mole equivalents of the amine compound listed in Column II of Table 4 were replaced by the corresponding amount of the amine compound listed in Column II of Table 5 and the 5 mole equivalents of the amine compound listed in Column III of Table 4 were replaced by the corresponding amount of the amine compound listed in Column III of Table 5. A library of affinity ligand-matrix conjugates of this invention was synthesised, Examples of which are identified in Column I of Table 5.
Chromatography columns of 1 ml total volume were packed with affinity ligand-matrix conjugates of Examples 137-180. The columns were equilibrated by flushing with 10 ml of 0.2 M sodium acetate, 0.1 M sodium chloride buffer, pH 5.0. Twelve ml of clarified fermenter broth containing 50 mg/ml human insulin precursor was applied to each chromatography column which were subsequently washed with 12 ml of 0.2 M sodium acetate, 0.1 M sodium chloride buffer, pH 5.0 to remove non-bound material. Bound human insulin precursor was eluted by flushing each column with 3 ml of 2M acetic acid. The human insulin precursor content of the flow-through, wash and elution fractions was determined by high performance liquid chromatography (HPLC) using a C 18 reverse-phase silica column (4 x 250 mm) and a solvent system comprising buffer A (0.2 M sodium sulphate, 40 mM phosphoric acid and 10% (v/v) acetonitrile, pH 2) and buffer B (50% (v/v acetonitrile) delivered at a flow rate of 1 ml per minute in the proportions 55% buffer A to 45% buffer B. The elution time of human insulin precursor was determined by comparison to a reference standard.

Analysis of the results revealed affinity ligand-matrix conjugates of Examples 139, 140, 145, 148, 153, 159, 162, 163, 164, 166, 167, 170, 171, 173 all bound human insulin precursor which was eluted under the conditions described in this Example. As a consequence, affinity ligand-matrix conjugates of Examples 139, 140, 145, 148, 153, 159, 162, 163, 164, 166, 167, 170, 171, 173 are considered to be of execeptional value in the separation, isolation or purification of human insulin precursor.

Table 5

| I | II | III |
|---|---|---|
| 137 | 1-amino-6-naphthalenesulphonic acid | benzylamine |
| 138 | 1-amino-6-naphthalenesulphonic acid | 3,5-diaminobenzoic acid |
| 139 | 1-amino-5-naphthol | benzylamine |
| 140 | 1-amino-5-naphthol | 3,5-diaminobenzoic acid |
| 141 | benzylamine | 3-aminobenzoic acid |
| 142 | benzylamine | 4-aminobenzoic acid |
| 143 | tyramine | 3-aminobenzoic acid |
| 144 | tyramine | 4-aminobenzoic acid |
| 145 | 1-amino-5-naphthol | 1-amino-5-naphthol |
| 146 | 1-amino-5-naphthol | 3-aminobenzoic acid |
| 147 | 1-amino-5-naphthol | 4-aminobenzoic acid |
| 148 | 1-amino-5-naphthol | tyramine |
| 149 | 1-amino-6-naphthalenesulphonic acid | tyramine |
| 150 | 1-amino-5-naphthol | 3-amino-1,2-propanediol |
| 151 | 1-amino-5-naphthol | 3-aminopropan-1-ol |
| 152 | 1-amino-5-naphthol | 5-aminopentan-1-ol |
| 153 | 1-amino-5-naphthol | 3-aminophenol |
| 154 | 1-amino-5-naphthol | 6-aminocaproic acid |
| 155 | 1-aminonaphthalene | benzylamine |
| 156 | 1-aminonaphthalene | 3,5-diaminobenzoic acid |
| 157 | 1-aminonaphthalene | 3-aminobenzoic acid |
| 158 | 1-aminonaphthalene | 4-aminobenzoic acid |
| 159 | 3-amino-2-naphthoic acid | 3-aminophenol |
| 160 | 4-amino-1-naphthol | 3-aminophenol |
| 161 | 1-amino-2-naphthol | 3-aminophenol |
| 162 | 3-amino-2-naphthol | 3-aminophenol |
| 163 | 1-amino-6-naphthol | 3-aminophenol |
| 164 | 1-amino-7-naphthol | 3-aminophenol |
| 165 | 2-amino-1-naphthol | 3-aminophenol |
| 166 | 6-amino-1-naphthol | 3-aminophenol |
| 167 | 3-amino-2-naphthoic acid | 3-amino-2-naphthoic acid |
| 168 | 4-amino-1-naphthol | 4-amino-1-naphthol |
| 169 | 1-amino-2-naphthol | 1-amino-2-naphthol |
| 170 | 3-amino-2-naphthol | 3-amino-2-naphthol |

Table 5 (continued)

| I | II | III |
|---|---|---|
| 171 | 1-amino-7-naphthol | 1-amino-7 -naphthol |
| 172 | 2-amino-1-naphthol | 2-amino-1-naphthol |
| 173 | 6-amino-1-naphthol | 6-amino-1-naphthol |
| 174 | 3-amino-2-naphtoic acid | 1-amino-5-naphthol |
| 175 | 3-amino-2-naphthoic acid | 4-amino-1-naphthol |
| 176 | 3-amino-2-naphthoic acid | 2-amino-1-naphthol |
| 177 | 1-amino-7-naphthol | 1-amino-5-naphthol |
| 178 | 1-amino-7-naphthol | 3-amino-2-naphthoic acid |
| 179 | 1-amino-7-naphthol | 4-amino-1-naphthol |
| 180 | 1-amino-7-naphthol | 2-amino-1-naphthol |

**Example 181**

This example demonstrates the process by which affinity ligand-matrix conjugates of this invention may be synthesised which are of value for the purification of Factor VII.

An affinity ligand-matrix conjugate of this invention was synthesised according to Example 74 except that the 5 mole equivalents of the amine compound listed in Column II of Table 4 were replaced by the corresponding amount of 2-aminobenzimidazole and the 5 mole equivalents of the amine compound listed in Column III of Table 4 were replaced by 3-amino-2-naphthoic acid. This affinity ligand-matrix conjugate may be used for the purification of Factor VIIa according to Example 129 of this invention.

**Example 182**

**Purification of EEAEPK-insulin B-chain$^{1-29}$-A-A-K-insulin A-chain$^{1-21}$ on an affinity ligand-matrix conjugate according to Example 171:**

50 ml of ionexchange purified insulin precursor (EEAEPK-insulin B-chain$^{1-29}$-A-A-K-insulin A-chain$^{1-21}$ at 2.2 mg/ml) was applied a Pharmacia K16 (1.6 x 6 cm) column packed with 12 ml of the conjugate matrix, equilibrated in 0.2 M potassium citrate pH 5.5, at 1.8 ml/min at ambient temperature. The column was washed in 50 ml of 0.1 M potassium citrate, 0.2 M potassium sulfate pH 5.5 and then eluted with 0.1 M acetic acid at 1.8 ml/min. 5.0 ml fractions were collected.
The column was cleaned with 50 ml of 0.5 M NaOH and regenerated with 50 ml of 0.1 M citric acid, 60% v/v ethanol. Samples for RP-HPLC analysis were diluted with 2 M acetic acid prior to analysis.

| Sample | Identification | Conc. mg/ml | Volume ml | Amount mg | % |
|---|---|---|---|---|---|
| Application | R-029e | 2.27 | 50 | 114 | 100 |
| Run through | R-033a | 0.03 | 50 | 1.4 | 1.2 |
| Wash | R-033b | 0.09 | 50 | 4.3 | 3.8 |
| Pool 1 | R-033c | 5.61 | 15 | 84.2 | 74 |
| Pool 2 | R-033d | 0.55 | 10 | 5.5 | 4.8 |

A dynamic binding capacity of 9.5 mg/ml matrix was demonstrated with a yield of 88% of the precursor.

**Claims**

**1.** Affinity ligand-matrix conjugates comprising a ligand with the general formula (a):

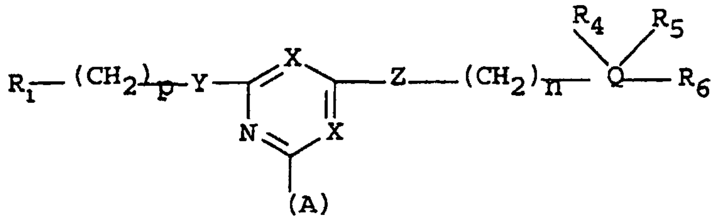 (a)

wherein

$R_1$ represents a hydrogen atom, an alkyl group containing from 1 to 6 carbon atoms, a hydroxyalkyl group containing from 1 to 6 carbon atoms, a cyclohexyl group, an amino group, a phenyl group, naphthyl group, 1-phenylpyrazole, indazole, benzthiazole group, benzoxazole group or a benzimidazole group, each of which benzene, naphthalene, 1-phenylpyrazole, indazole, benzthiazole, benzoxazole or benzimidazole ring is optionally substituted with one or more substituents independently selected from the group consisting of alkyl groups containing from 1 to 6 carbon atoms, alkoxy groups containing from 1 to 6 carbon atoms, acyloxy or acylamino groups containing from 1 to 6 carbon atoms, amino groups, hydroxyl groups, carboxylic acid groups, sulphonic acid groups, carbamoyl groups, sulphamoyl groups, alkylsulphonyl groups containing from 1 to 6 carbon atoms and halogen atoms;

Y represents an oxygen atom, a sulphur atom or a group N-$R_2$;

Z represents an oxygen atom, a sulphur atom or a group N-$R_3$;

$R_2$ and $R_3$ each independently represent a hydrogen atom, an alkyl group containing from 1 to 6 carbon atoms; a hydroxyalkyl group containing from 1 to 6 carbon atoms, a benzyl group or a β-phenylethyl group;

$R_4$, $R_5$ and $R_6$ each independently represent a hydrogen atom, a hydroxyl group, an alkyl group containing from 1 to 6 carbon atoms, an alkoxy group containing from 1 to 6 carbon atoms, an amino group, an acyloxy or acylamino group containing from 1 to 6 carbon atoms, a carboxylic acid group, a sulphonic acid group, a carbamoyl or sulphamoyl group, an alkylsulphonyl group containing from 1 to 6 carbon atoms or a halogen atom;

one of the symbols X represents a nitrogen atom and the other symbol X represents a nitrogen atom or a carbon atom carrying a chlorine atom or a cyano group;

Q represents a benzene, naphthalene, 1-phenylpyrazole, indazole, benzthiazole, benzoxazole or benzimidazole ring;

n is an integer between 0 and 6;

p is an integer between 0 and 20;

and

which ligand is attached to a support matrix in position (A), optionally through a spacer arm interposed between the matrix and ligand,

with the proviso that formula (a) does not comprise the reaction products of the compounds 4-chloro-2,6-di (phenyl-amino)-1,3,5-triazine-3'-sulfonic acid, 4-chloro-2,6-di (phenyl-amino)-1,3,5-triazine-3',2"-disulfonic acid, and 4-chloro-2-(4"-amino-phenyl-amino)-1,3,5-triazine-3',2"-disulfonic acid with Dextran T 500.

2. Affinity ligand-matrix conjugates according to claim 1 wherein the optional spacer arm interposed between the ligand and the matrix is represented by the general formula (b)

$$-T-[-L-V-]_m- \quad (b)$$

wherein T represents an oxygen atom, a sulphur atom or a group N-$R_7$; wherein $R_7$ represents a hydrogen atom or an alkyl group containing from 1 to 6 carbon atoms;

V represents an oxygen atom, a sulphur atom, a -COO- group, a CONH group or an NHCO group or a -$PO_3H$- group , an NH-arylene-$SO_2$-$CH_2$-$CH_2$ group or an N-$R_8$ group; wherein $R_8$ represents a hydrogen atom or an alkyl group containing from 1 to 6 carbon atoms;

L represents an optionally substituted hydrocarbon linkage containing from 2 to 20 carbon atoms; and

m is 0 or 1.

3. Affinity ligand-matrix conjugates according to claim 1 which are represented by the General Formula (I):

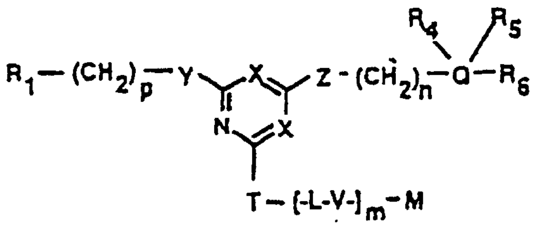

(I)

wherein

$R_1$, Y, Z, $R_2$, $R_3$, $R_4$, $R_5$, $R_6$, X, Q, n and p are as defined above

T represents an oxygen atom, a sulphur atom or a group N-$R_7$;

V represents an oxygen atom, a sulphur atom, a -COO- group, a CONH group or an NHCO group or a -$PO_3$H-group , an NH-arylene-$SO_2$-$CH_2$-$CH_2$ group or an N-$R_8$ group;

$R_7$ and $R_8$ each independently represents a hydrogen atom or an alkyl group containing from 1 to 6 carbon atoms;

L represents an optionally substituted hydrocarbon linkage containing from 2 to 20 carbon atoms;

m is 0 or 1; and

M represents the residue of a support matrix.

4. Affinity ligand matrix conjugates according to anyone of the preceding claims wherein M represents the residue of a support matrix which may be any compound or material, particulate or non particulate, soluble or insoluble, porous or non-porous which may be used in conjunction with affinity ligands to form a novel affinity ligand-matrix conjugate according to anyone of the preceding claims and which provides a convenient means of separating the affinity ligands from solutes in a contacting solution.

5. Affinity ligand-matrix conjugates according to anyone of the preceding claims wherein $R_1$ represents a phenyl or naphthyl group each of which is optionally substituted on the benzene or naphthalene ring with one or more independently selected from hydroxyl groups and carboxylic acid groups.

6. Affinity ligand-inatrix conjugates according to anyone of the preceding claims wherein $R_2$ represents a hydrogen atom.

7. Affinity ligand-matrix conjugates according to anyone of the preceding claims wherein $R_3$ represents a hydrogen atom.

8. Affinity ligand-matrix conjugates according to anyone of the preceding claims wherein $R_4$ represents a hydrogen atom, a hydroxyl group, a carboxylic acid group, or an amino group.

9. Affinity ligand-matrix conjugates according to anyone of the preceding claims wherein $R_5$ represents a hydrogen atom, a hydroxyl group, a carboxylic acid group, or an amino group.

10. Affinity ligand-matrix conjugates according to anyone of the preceding claims wherein $R_6$ represents a hydrogen atom, a hydroxyl group, a carboxylic acid group, or an amino group.

11. Affinity ligand-matrix conjugates according to anyone of the preceding claims wherein $R_7$ represents a hydrogen atom.

12. Affinity ligand-matrix conjugates according to anyone of the preceding claims wherein T represents an oxygen atom or an NH group.

13. Affinity ligand-matrix conjugates according to anyone of the preceding claims wherein Y represents N-$R_2$-wherein $R_2$ is as defined above.

14. Affinity ligand-matrix conjugates according to anyone of the preceding claims wherein Z represents N-$R_3$ wherein $R_3$ is as defined above.

**15.** Affinity ligand-matrix conjugates according to anyone of the preceding claims wherein both X represents a nitrogen atom.

**16.** Affinity ligand-matrix conjugates according to anyone of the preceding claims wherein Q represents a benzene or naphthalene ring.

**17.** Affinity ligand-matrix conjugates according to anyone of the preceding claims wherein n represents 0 or 2.

**18.** Affinity ligand-matrix conjugates according to anyone of the preceding claims wherein p represents 0 or 2.

**19.** Affinity ligand-matrix conjugates according to anyone of the preceding claims wherein m represents 0 or 1.

**20.** Affinity ligand-matrix conjugates according to anyone of the preceding claims wherein L represents an ethyl, propyl, hydroxy propyl, butyl, pentyl, hexyl, octyl or decyl group and V and m are as defined above.

**21.** An affinity ligand-matrix conjugate according to anyone of the preceding claims wherein V represents an oxygen atom or an NH group and L and m are as defined above.

**22.** Affinity ligand-matrix conjugates according to anyone of the preceding claims wherein m represents 1 and L and V are as defined above.

**23.** Affinity ligand-matrix conjugates according to anyone of the preceding claims wherein the residue of a support matrix M represents optionally activated agarose, silica, cellulose, glass, toyopearl, hydroxyethylmethacrylate, polyacrylamide, styrenedivinylbenzene, Hyper D, perfluorocarbons.

**24.** Affinity ligand-matrix conjugates according to claim 23 wherein M represents optionally tresyl activated, sulphonylchloride activated, tosyl activated, vinylsulphone activated or epoxy activated agarose.

**25.** Affinity ligand-matrix conjugates according to anyone of the preceding claims selected from:

i)

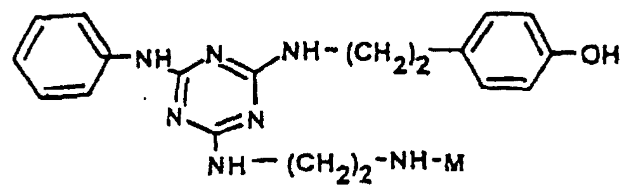

ii)

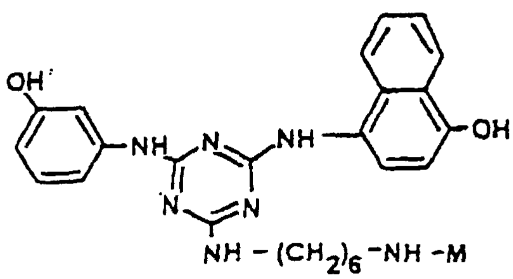

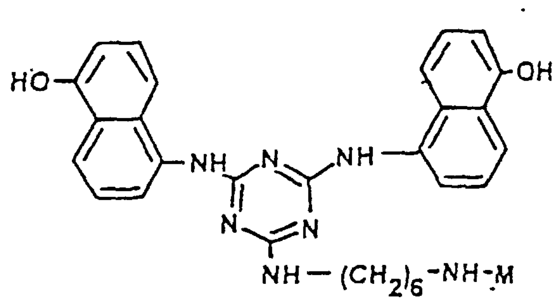

iii)

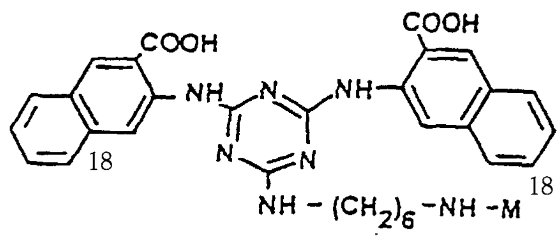

iv)

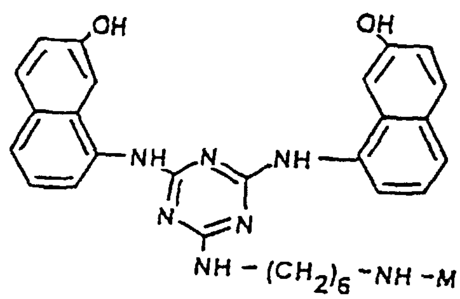

v)

vi) 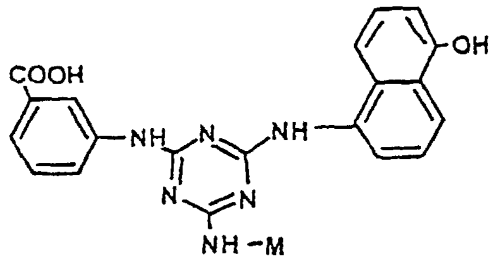

vii) 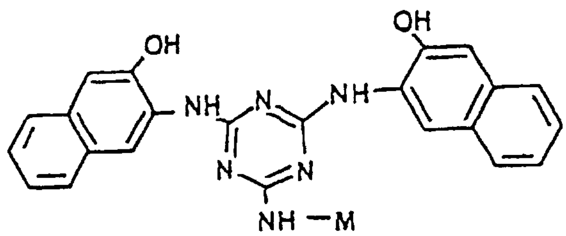

viii) 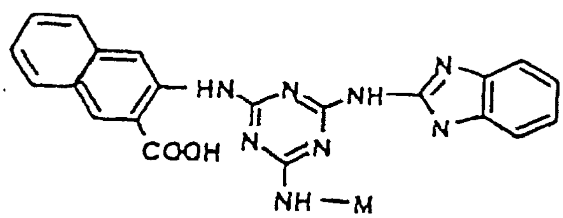

wherein M is as defined above.

**26.** A method for the manufacture of the novel affinity ligand-matrix conjugates as defined in anyone of the preceding claims which comprises reacting, in any order, a halogeno-heterocylic compound of General Formula (II):

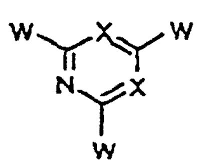 (II)

wherein the symbols X have the meaning specified in anyone of the preceding claims and W represents a halogen

atom with

(i) a compound of General Formula (III):

$$R_1\text{-}(CH_2)_p\text{-}Y\text{-}H \quad (III)$$

wherein the symbols $R_1$, p and Y have the meanings specified in anyone of the preceding claims,

(ii) a compound of General Formula (IV)

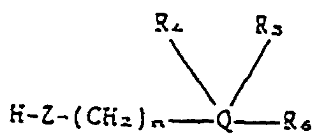

(IV)

wherein the symbols $R_4$, $R_5$, $R_6$, Q, Z and n have the meanings specified in anyone of the preceding claims and

(iii) an optionally derivatised support matrix of General Formula V

$$H\text{-}T\text{-}[\text{-}L\text{-}V\text{-}]_m\text{-}M \quad (V)$$

wherein the symbols L, M, V, T, and m have the meanings specified in anyone of the preceding claims.

**27.** A method for the manufacture of the novel affinity ligand-matrix conjugates as defined in anyone of the preceding claims which comprises reacting, in any order, a halogenoheterocyclic compound of General Formula (II), as defined in claim 26, with

(i) a compound of General Formula (III), as defined in Claim 26
(ii) a compound of General Formula (IV), as defined in Claim 26 and
(iii) with a linking unit of General Formula (VI)

$$H\text{-}T\text{-}L\text{-}V\text{-}H \quad (VI)$$

wherein the symbols L, V and T have the meanings specified in anyone of the preceding claims to give a compound of General Formula (VII):

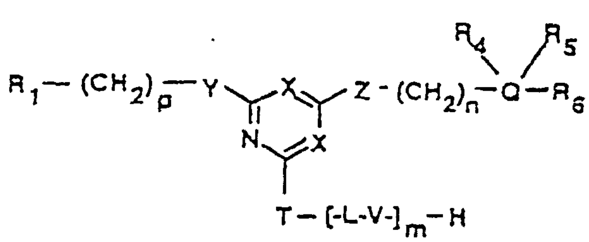

(VII)

wherein $R_1$, $R_4$, $R_5$, $R_6$, T, Q, L, V, X, Y, Z, m, n and p have the meanings specified in anyone of the preceding claims followed by reaction of the compound of General Formula (VII) with a matrix support.

**28.** Novel affinity ligands of General Formula (XII):

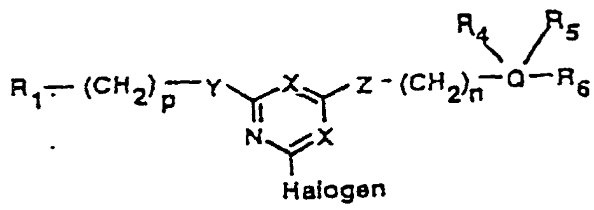

(XII)

wherein $R_1$, $R_4$, $R_5$, $R_6$, Q, X, Y, Z, n and p have the meanings specified in anyone of the preceding claims and Halogen represents a fluorine, chlorine, bromine or iodine atom, with the proviso that formula (XII) does not comprise the compounds 4-chloro-2,6-di (phenylamino)-1,3,5-triazine-3'-sulfonic acid, 4-chloro-2,6-di (phenyl-amino)-1,3,5-triazine-3',2''-disulfonic acid, and 4-chloro-2-(4''-amino-phenyl-amino)-1,3,5-triazine-3',2''-disulfonic acid.

**29.** A method of attaching novel affinity ligands of General Formula (XII) as claimed in Claim 28 to a matrix of General Formula (V) as defined in Claim 26 by reacting the novel affinity ligands with the matrix at temperatures between -20°C and 121°C, optionally in the presence of an acid binding agent.

**30.** Novel affinity ligands of General Formula (XIII):

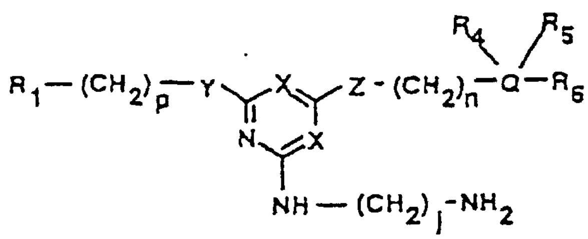

(XIII)

wherein $R_1$, $R_4$, $R_5$, $R_6$, Q, X, Y, Z, m, n and p have the meanings specified in anyone of the preceding claims and j is an integer between 2 and 20.

**31.** A method of preparing novel affinity ligands of General Formula (XIII) as claimed in Claim 30 by reacting a compound of General Formula (XII) as claimed in Claim 28 with an alkylene diamine of General Formula $H_2N-(CH_2)_j-NH_2$ at temperatures between 0°C and 100°C in the presence of an acid binding agent.

**32.** Novel affinity ligands of General Formula (XIV):

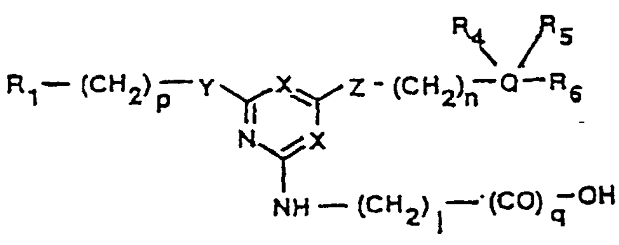

(XIV)

wherein $R_1$, $R_4$, $R_5$, Q, X, Y, Z, m, n and p have the meanings specified in anyone of the preceding claims, q is 0 or 1 and j is an integer between 2 and 20.

**33.** A method of preparing novel affinity ligands of General Formula (XIV) as claimed in Claim 32 by reacting a compound of General Formula (XII) as claimed in Claim 28 with an amino hydroxy compound of General Formula $H_2N-(CH_2)_j-(CO)_q-OH$ at temperatures between 0°C and 100°C, optionally in the presence of an acid binding agent.

**34.** Novel affinity ligands of General Formula (VIII):

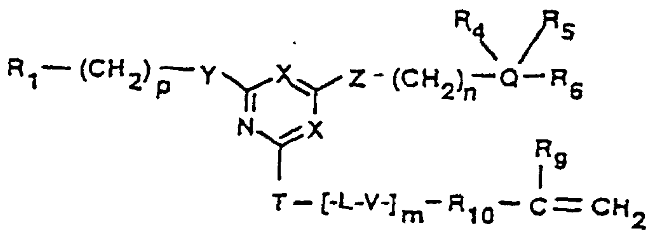

(VIII)

wherein $R_1$, $R_4$, $R_5$, $R_6$, L, Q, T, V, X, Y, Z, m, n and p have the meanings specified in anyone of the preceding claims; $R_9$ represents a hydrogen atom or an alkyl group containing from 1 to 6 carbon atoms; $R_{10}$ represents a carbonyl group, a methylene group, an $-NH-CH_2-$ group or an $-S-CH_2-$ group.

**35.** Novel affinity ligands of General Formula (XV):

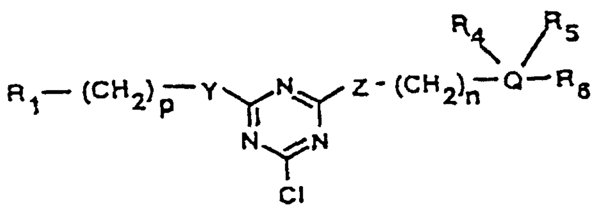

(XV)

wherein $R_1$, $R_4$, $R_5$, $R_{6,}$, Q, n and p have the meanings specified in anyone of the preceding claims with the proviso that formula (XV) does not comprise the compounds 4-chloro-2,6-di (phenyl-amino)-1,3,5-triazine-3'-sulfonic acid, 4-chloro-2,6-di (phenyl-amino)-1,3,5-triazine-3',2''-disulfonic acid, and 4-chloro-2-(4''-amino-phenyl-amino)-1,3,5-triazine-3',2''-disulfonic acid.

**36.** Novel affinity ligands of General Formula (XVI):

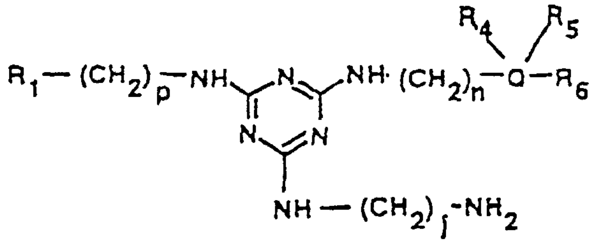

(XVI)

wherein $R_1$, $R_4$, $R_5$, $R_6$, Q, n and p have the meanings specified in anyone of the preceding claims and j is an integer between 2 and 20.

**37.** Affinity ligands according to anyone of claims 28, 30, 32, 34, 35 or 36 wherein $R_1$ represents a phenyl or naphthyl group each of which is optionally substituted on the benzene or naphthalene ring with one or more independently selected from hydroxyl groups and carboxylic acid groups.

**38.** Affinity ligands according to anyone of claims 28, 30, 32, 34, 35 or 36 wherein $R_4$ represents a hydrogen atom, a hydroxyl group, a carboxylic acid group or an amino group.

**39.** Affinity ligands according to anyone of claims 28, 30, 32, 34, 35 or 36 wherein $R_5$ represents a hydrogen atom, a hydroxyl group, a carboxylic acid group or an amino group.

**40.** Affinity ligands according to anyone of claims 28, 30, 32, 34, 35 or 36 wherein $R_6$ represents a hydrogen atom, a hydroxyl group, a carboxylic acid group or an amino group.

**41.** Affinity ligands according to anyone of claims 28, 30, 32, 34, 35 or 36 wherein Q represents a benzene or naphthalene ring.

**42.** Affinity ligands according to anyone of claims 28, 30, 32, or 34 wherein X represents a nitrogen atom.

**43.** Affinity ligands according to anyone of claims 28, 30, 32, 34 or 35 wherein Y represents a -NH- group.

**44.** Affinity ligands according to anyone of claims 28, 30, 32, 34 or 35 wherein Z represents a -NH- group.

**45.** Affinity ligands according to anyone of claims 28, 30, 32, 34, 35 or 36 wherein n is 0 or 2.

**46.** Affinity ligands according to anyone of claims 28, 30, 32, 34, 35 or 36 wherein p is 0 or 2.

**47.** Affinity ligands according to anyone of claims 30, 32, or 36 wherein j is 2, 4 or 6.

**48.** Affinity ligands according to claim 34 wherein L is an ethyl, butyl, or hexyl group.

**49.** Affinity ligands according to claim 34 wherein T represents a -NH- group.

**50.** Affinity ligands according to claim 34 wherein V represents a -NH- group.

**51.** Affinity ligands according to claim 34 wherein m is 1.

**52.** Novel affinity ligands of General Formula (XI):

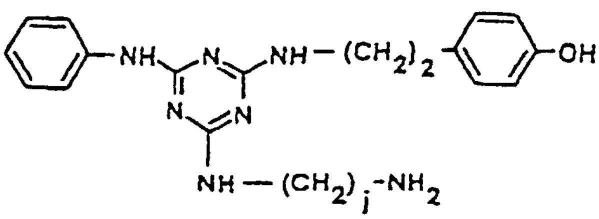

(XI)

wherein j is an integer betweem 2 and 20.

**53.** Affinity ligands according to anyone of claims 28 to 51 selected among the following:

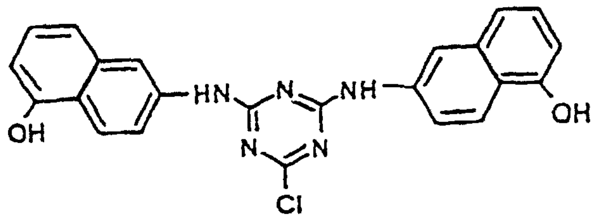

(1)

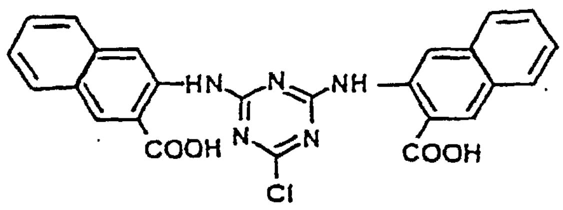

(2)

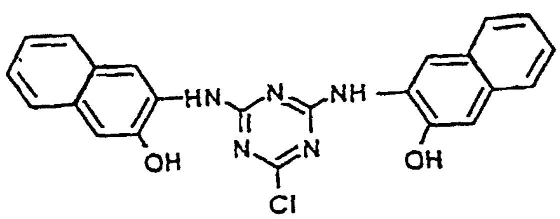

(3)

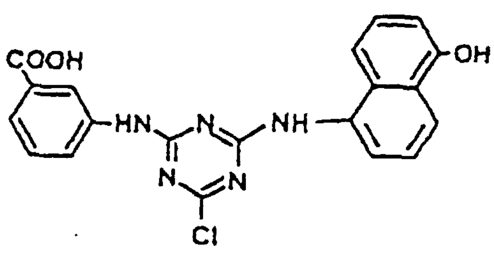

(4)

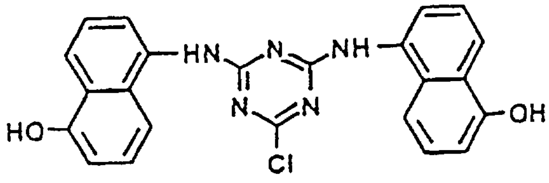

(5)

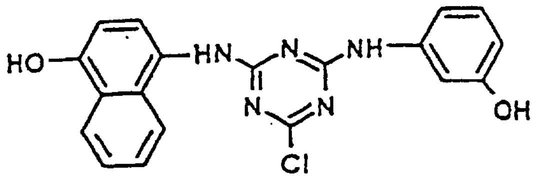

(6)

(7)

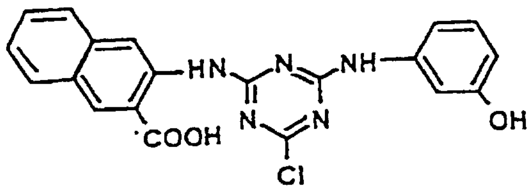

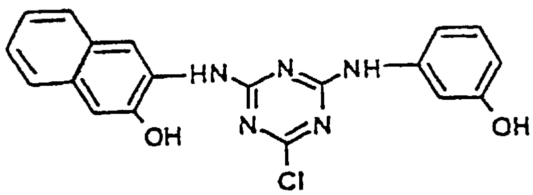

(8)

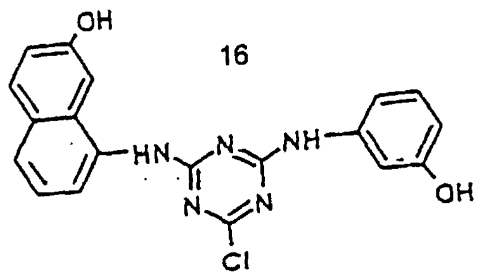

16

(9)

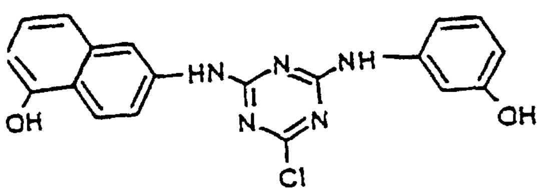

(10)

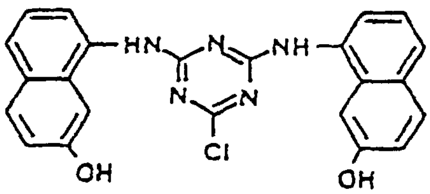

(11)

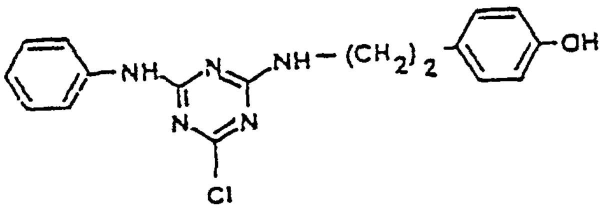

(12)

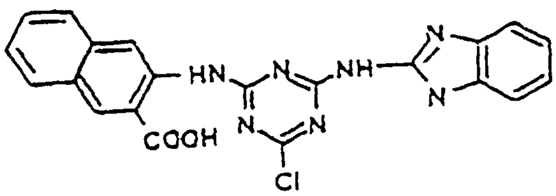

(13)

**54.** The use of affinity ligands according to anyone of the preceding claims for the preparation of affinity ligand matrix conjugates

**55.** A method of attaching the novel affinity ligands of General Formulae (VII) as defined in claim 27 and (XIII) as defined in Claim 30, (XVI) as defined in Claim 36 and (XI) as defined in Claim 52 to carbohydrate or organic polymer matrices by reacting the carbohydrate or organic polymer matrix with an activating agent followed by reaction of the activated matrix with the novel affinity ligand, optionally in the presence of an acid binding agent.

**56.** A method of attaching the novel affinity ligands of General Formulae (XIV) as defined in Claim 32 to carbohydrate or organic polymer matrices by condensation with the matrix.

**57.** A method of attaching the novel affinity ligands of General Formulae (VII) as defined in Claim 27 and (XIII) as defined in Claim 30, (XVI) as defined in Claim 36 and (XI) as defined in Claim 52 to metal oxide, glass or silica matrices, optionally coated with an organic polymer by reacting the optionally coated metal oxide, glass or silica matrix with an activating agent followed by reaction of the activated matrix with the novel affinity ligand, optionally in the presence of an acid binding agent.

**58.** A method of attaching the novel affinity ligands of General Formulae (XIV) as defined in Claim 32 to metal oxide, glass or silica matrices, optionally coated with an organic polymer by condensation with the matrix.

**59.** A method of attaching novel affinity ligands of General Formula (XV) as defined in Claim 35 and (XII) as defined in Claim 28 to a matrix of General Formula (V) as defined in Claim 26 by reacting the novel affinity ligands with the matrix at temperatures between -20°C and 121°C, optionally in the presence of an acid binding agent.

**60.** Affinity ligand-matrix conjugates, prepared as claimed in Claims 26, 27, 29, 54, 55, 56, 57 and 58.

**61.** The use of affinity ligand-matrix conjugates, according to anyone of the preceding claims to affinity ligand-matrix conjugates for the separation, isolation, purification, characterisation, identification or quantification of proteins.

**62.** The use according to claim 61 wherein the proteinaceous material is $I_gG$, $I_gM$, $I_gA$, insulins, Factor VII, or Human Growth Hormone or analogues, derivatives and fragments thereof and precursors.

**63.** A process for the separation or purification of proteinaceous materials comprising carrying out affinity chromatography using as the biospecific ligand a ligand of general formula (a) as defined above.

**64.** The use according to claim 61 wherein the proteinaceous material is immunoglobulins or subclasses, fragments, precursors or derivatives thereof, whether derived from natural or recombinant sources.

**65.** The use according to claim 61 wherein the proteinaceous material is immunoglobulin G (IgG), immunoglobulin M (IgM), immunoglobulin A (IgA) or subclasses, fragments, precursors or derivatives thereof, whether derived from natural or recombinant sources.

**66.** The use according to claim 61 wherein the proteinaceous material is insulins or insulin analogues, derivatives and fragments thereof and precursors, whether derived from natural or recombinant sources.

**67.** The use according to claim 61 wherein the proteinaceous material is FVII or analogues, derivatives and fragments thereof and precursors, whether derived from natural or recombinant sources.

**68.** The use according to claim 61 wherein the affinity ligand-matrix conjugates comprises a ligand selected among the following:

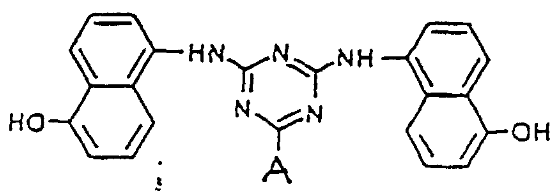

(5a)

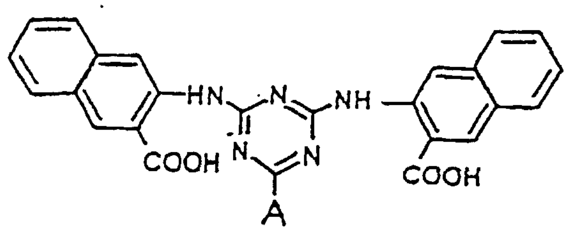

(2a)

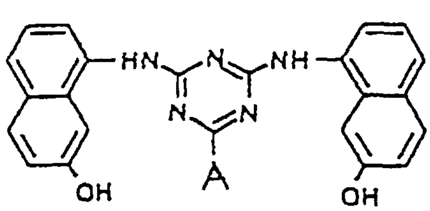

(11a)

which ligand is attached to a support matrix in position (A), optionally through a spacerarm represented by the general formula (b) as specified above.

**69.** The use according to claim 68 wherein the ligand is 11a.

**70.** The use according to claim 68 or 69 wherein the support matrix is optionally activated agarose, cellulose, silica or glass.

**71.** The separation, isolation, purification, characterisation, identification or quantification of immunoglobulins by any process whereby the said immunoglobulins are applied to affinity ligand-matrix conjugates, as defined in anyone of above affinity ligand-matrix conjugate claims at a pH in the range 5.0 to 12.0 and subsequently removed, eluted or desorbed by reducing the pH to 4.9 or lower.

**72.** The separation, isolation, purification, characterisation, identification or quantification of insulins or insulin analogues or derivatives thereof and precursors by any process whereby the said insulins, insulin derivatives, analogues, and precursors are applied to affinity ligand-matrix conjugates, as defined in anyone of above affinity ligand-matrix conjugate claims at a pH in the range 4,0 to 9,0 and subsequently removed, eluted or desorbed by reducing the pH to 3,99 or lower or to 9,01 or higher.

**73.** The separation of recombinant FVIIa by selective binding of FVIIa to the afinity matrix of the formula

**viii)**

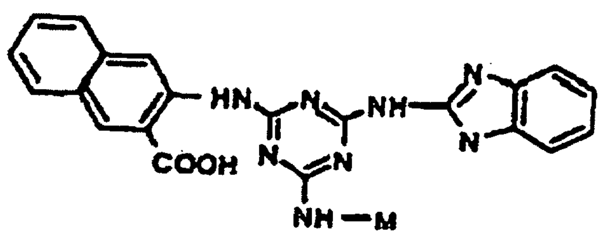

wherein M is agarose bearing amino groups from cell culture media, whereby the said FVIIa is applied to said affinity ligand-matrix conjugate in the presence of 5mM $Ca^{2+}$ and subsequently eluted.

## Patentansprüche

**1.** Affinitätsligand-Matrix-Konjugate, umfassend einen Liganden der allgemeinen Formel (a)

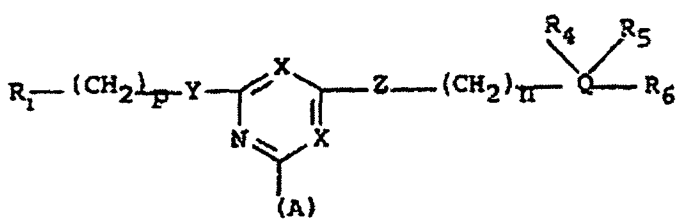

(a)

wobei

$R_1$ ein Wasserstoffatom, eine 1 bis 6 Kohlenstoffatome enthaltende Alkylgruppe, eine 1 bis 6 Kohlenstoffatome enthaltende Hydroxyalkylgruppe, eine Cyclohexylgruppe, eine Aminogruppe, eine Phenylgruppe, Naphthylgruppe, 1-Phenylpyrazol-, Indazol-, Benzthiazolgruppe, Benzoxazolgruppe oder eine Benzimidazolgruppe, wobei jeder der Benzol-, Naphthalin-, 1-Phenylpyrazol-, Indazol-, Benzthiazol- oder Benzimidazolringe gegebenenfalls mit einem oder mehreren Substituenten, unabhängig voneinander ausgewählt aus der Gruppe, bestehend aus 1 bis 6 Kohlenstoffatome enthaltenden Alkylgruppen, 1 bis 6 Kohlenstoffatome enthaltenden Alkoxygruppen, 1 bis 6 Kohlenstoffatome enthaltenden Acyloxy- oder Acylaminogruppen, Aminogruppen, Hydroxygruppen, Carbonsäuregruppen, Sulfonsäuregruppen, Carbamoylgruppen, Sulfamoylgruppen, 1 bis 6 Kohlenstoffatome enthaltenden Alkylsulfonylgruppen und Halogenatomen, substituiert ist, darstellt;

Y ein Sauerstoffatom, ein Schwefelatom oder eine N-$R_2$-Gruppe darstellt;
Z ein Sauerstoffatom, ein Schwefelatom oder eine N-$R_3$-Gruppe darstellt;
$R_2$ und $R_3$ unabhängig voneinander ein Wasserstoffatom, eine 1 bis 6 Kohlenstoffatome enthaltende Alkylgruppe, eine 1 bis 6 Kohlenstoffatome enthaltende Hydroxyalkylgruppe, eine Benzylgruppe oder eine β-Phenylethylgruppe darstellen;
$R_4$, $R_5$ und $R_6$ unabhängig voneinander ein Wasserstoffatom, eine Hydroxygruppe, eine 1 bis 6 Kohlenstoffatome enthaltende Alkylgruppe, eine 1 bis 6 Kohlenstoffatome enthaltende Alkoxygruppe, eine Aminogruppe, eine 1 bis 6 Kohlenstoffatome enthaltende Acyloxy- oder Acylaminogruppe, eine Carbonsäuregruppe, eine Sulfonsäuregruppe, eine Carbamoyl- oder Sulfamoylgruppe, eine 1 bis 6 Kohlenstoffatome enthaltende Alkylsulfonylgruppe oder ein Halogenatom darstellen;
eines der Symbole X ein Stickstoffatom und das andere Symbol X ein Stickstoffatom oder ein Kohlenstoffatom, das ein Chloratom oder eine Cyanogruppe trägt, darstellt;
Q einen Benzol-, Naphthalin-, 1-Phenylpyrazol-, Indazol-, Benzthiazol-, Benzoxazol- oder Benzimidazolring darstellt;
n eine ganze Zahl zwischen 0 und 6 ist;
p eine ganze Zahl zwischen 0 und 20 ist;
und
wobei der Ligand gegebenenfalls durch einen zwischen die Matrix und den Liganden eingeschobenen Abstandhalterarm an eine Trägermatrix in Position (A) angelagert ist,
mit der Maßgabe, dass Formel (a) nicht die Reaktionsprodukte der Verbindungen 4-Chlor-1,6-di(phenylamino)-1,3,5-triazin-3'-sulfonsäure, 4-Chlor-2,6-di(phenylamino)-1,3,5-triazin-3',2"-disulfonsäure und 4-Chlor-2-(4"-aminophenylamino)-1,3,5-triazin-3',2"-disulfonsäure mit Dextran T 500 umfasst.

**2.** Affinitätsligand-Matrix-Konjugate nach Anspruch 1, wobei der optionale zwischen den Liganden und die Matrix eingeschobene Abstandhalterarm durch die allgemeine Formel (b)

$$-T-[-L-V-]_m- \qquad (b)$$

dargestellt ist, wobei
T ein Sauerstoffatom, ein Schwefelatom oder eine N-$R_7$-Gruppe darstellt, wobei $R_7$ ein Wasserstoffatom oder eine 1 bis 6 Kohlenstoffatome enthaltende Alkylgruppe darstellt;
V ein Sauerstoffatom, ein Schwefelatom, eine -COO-Gruppe, eine CONH-Gruppe oder eine NHCO-Gruppe oder eine -$PO_3H$-Gruppe, eine NH-Arylen-$SO_2$-$CH_2$-$CH_2$-Gruppe oder eine N-$R_8$-Gruppe darstellt, wobei $R_8$ ein Wasserstoffatom oder eine 1 bis 6 Kohlenstoffatome enthaltende Alkylgruppe darstellt;
L eine gegebenenfalls substituierte, 2 bis 20 Kohlenstoffatome enthaltende Kohlenwasserstoffbindung darstellt; und
m 0 oder 1 ist.

**3.** Affinitätsligand-Matrix-Konjugate nach Anspruch 1, die durch die allgemeine Formel (I)

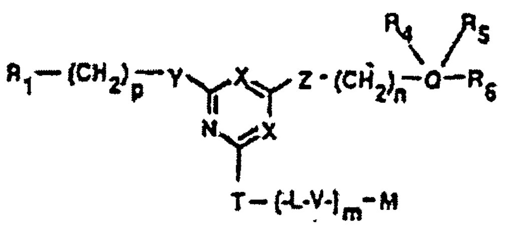

(I)

dargestellt sind, wobei
$R_1$, Y, Z, $R_2$, $R_3$, $R_4$, $R_5$, $R_6$, X, Q, n und p wie vorstehend definiert sind;
T ein Sauerstoffatom, ein Schwefelatom oder eine N-$R_7$-Gruppe darstellt;
V ein Sauerstoffatom, ein Schwefelatom, eine -COO-Gruppe, eine CONH-Gruppe oder eine NHCO-Gruppe oder eine -$PO_3H$-Gruppe, eine NH-Arylen-$SO_2$-$CH_2$-$CH_2$-Gruppe oder eine N-$R_8$-Gruppe darstellt;
$R_7$ und $R_8$ unabhängig voneinander ein Wasserstoffatom oder eine 1 bis 6 Kohlenstoffatome enthaltende Alkylgruppe darstellen;
L eine gegebenenfalls substituierte, 2 bis 20 Kohlenstoffatome enthaltende Kohlenwasserstoffbindung darstellt;

m 0 oder 1 ist; und
M den Rest einer Trägermatrix darstellt.

4. Affinitätsligand-Matrix-Konjugate nach einem der vorangehenden Ansprüche, wobei M den Rest einer Trägermatrix darstellt, die eine beliebige Verbindung oder ein beliebiges Material, teilchenförmig oder nicht-teilchenförmig, löslich oder unlöslich, porös oder nicht-porös, sein kann, die/das zusammen mit Affinitätsliganden verwendet wird, um ein neues Affinitätsligand-Matrix-Konjugat nach einem der vorangehenden Ansprüche zu bilden, und die/das ein günstiges Mittel zum Abtrennen der Affinitätsliganden von gelösten Stoffen in einer Kontaktlösung bereitstellt.

5. Affinitätsligand-Matrix-Konjugate nach einem der vorangehenden Ansprüche, wobei $R_1$ eine Phenyl- oder Naphthylgruppe darstellt, wobei jede davon gegebenenfalls am Benzol- oder Naphthalinring mit einem oder mehreren, unabhängig voneinander ausgewählt aus Hydroxygruppen und Carbonsäuregruppen, substituiert ist.

6. Affinitätsligand-Matrix-Konjugate nach einem der vorangehenden Ansprüche, wobei $R_2$ ein Wasserstoffatom darstellt.

7. Affinitätsligand-Matrix-Konjugate nach einem der vorangehenden Ansprüche wobei $R_3$ ein Wasserstoffatom darstellt.

8. Affinitätsligand-Matrix-Konjugate nach einem der vorangehenden Ansprüche, wobei $R_4$ ein Wasserstoffatom, eine Hydroxygruppe, eine Carbonsäuregruppe oder eine Aminogruppe darstellt.

9. Affinitätsligand-Matrix-Konjugate nach einem der vorangehenden Ansprüche, wobei $R_5$ ein Wasserstoffatom, eine Hydroxygruppe, eine Carbonsäuregruppe oder eine Aminogruppe darstellt.

10. Affinitätsligand-Matrix-Konjugate nach einem der vorangehenden Ansprüche, wobei $R_6$ ein Wasserstoffatom, eine Hydroxygruppe, eine Carbonsäuregruppe oder eine Aminogruppe darstellt.

11. Affinitätsligand-Matrix-Konjugate nach einem der vorangehenden Ansprüche, wobei $R_7$ ein Wasserstoffatom darstellt.

12. Affinitätsligand-Matrix-Konjugate nach einem der vorangehenden Ansprüche, wobei T ein Sauerstoffatom oder eine NH-Gruppe darstellt.

13. Affinitätsligand-Matrix-Konjugate nach einem der vorangehenden Ansprüche, wobei Y N-$R_2$ darstellt, wobei $R_2$ wie vorstehend definiert ist.

14. Affinitätsligand-Matrix-Konjugate nach einem der vorangehenden Ansprüche, wobei Z N-$R_3$ darstellt, wobei $R_3$ wie vorstehend definiert ist.

15. Affinitätsligand-Matrix-Konjugate nach einem der vorangehenden Ansprüche, wobei beide X ein Stickstoffatom darstellen.

16. Affinitätsligand-Matrix-Konjugate nach einem der vorangehenden Ansprüche, wobei Q einen Benzol- oder Naphthalinring darstellt.

17. Affinitätsligand-Matrix-Konjugate nach einem der vorangehenden Ansprüche, wobei n 0 oder 2 darstellt.

18. Affinitätsligand-Matrix-Konjugate nach einem der vorangehenden Ansprüche, wobei p 0 oder 2 darstellt.

19. Affinitätsligand-Matrix-Konjugate nach einem der vorangehenden Ansprüche, wobei m 0 oder 1 darstellt.

20. Affinitätsligand-Matrix-Konjugate nach einem der vorangehenden Ansprüche, wobei L eine Ethyl-, Propyl-, Hydroxypropyl-, Butyl-, Pentyl-, Hexyl-, Octyl- oder Decylgruppe darstellt und V und m wie vorstehend definiert sind.

21. Affinitätsligand-Matrix-Konjugat nach einem der vorangehenden Ansprüche, wobei V ein Sauerstoffatom oder eine NH-Gruppe darstellt und L und m wie vorstehend definiert sind.

**22.** Affinitätsligand-Matrix-Konjugate nach einem der vorangehenden Ansprüche, wobei m 1 darstellt und L und V wie vorstehend definiert sind.

**23.** Affinitätsligand-Matrix-Konjugate nach einem der vorangehenden Ansprüche, wobei der Rest einer Trägermatrix M gegebenenfalls aktivierte/s Agarose, Siliciumdioxid, Celllulose, Glas, Toyopearl, Hydroxyethylmethacrylat, Polyacrylamid, Styroldivinylbenzol, Hyper D, Perfluorkohlenstoffe darstellt.

**24.** Affinitätsligand-Matrix-Konjugate nach Anspruch 23, wobei M gegebenenfalls Tresyl-aktivierte, Sulfonylchlorid-aktivierte, Tosylaktivierte, Vinylsulfon-aktivierte oder Epoxy-aktivierte Agarose darstellt.

**25.** Affinitätsligand-Matrix-Konjugate nach einem der vorangehenden Ansprüche, ausgewählt aus

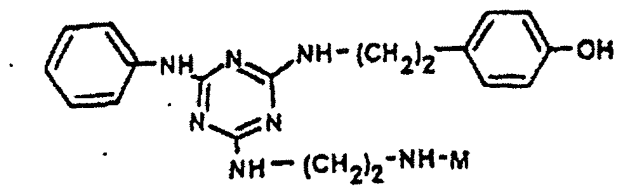

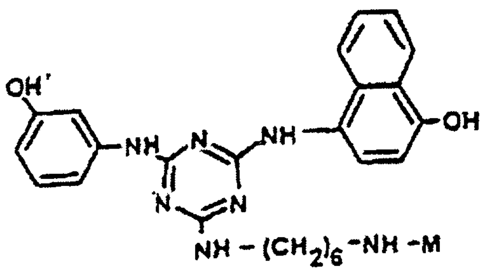

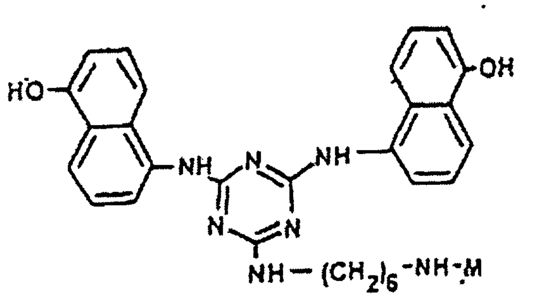

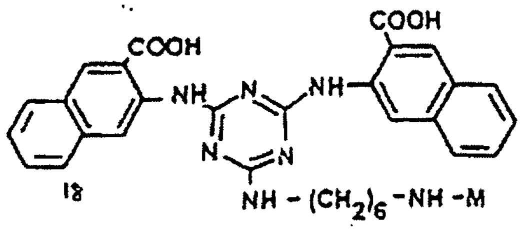

18

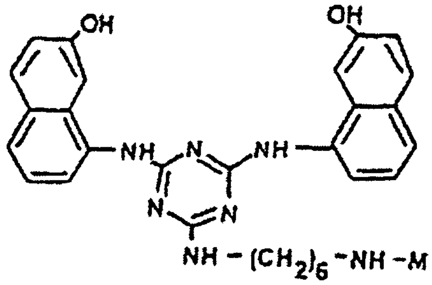

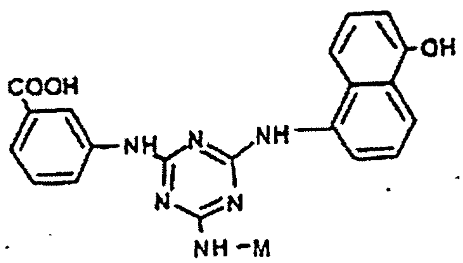

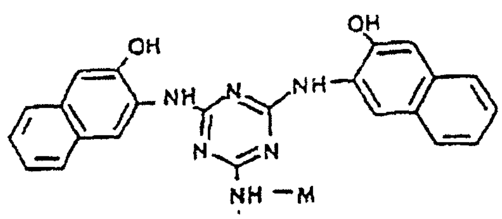

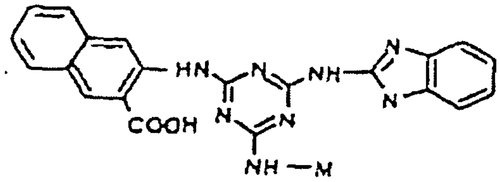

wobei M wie vorstehend definiert ist.

**26.** Verfahren zur Herstellung der neuen wie in einem der vorangehenden Ansprüche definierten Affinitätsligand-Matrix-Konjugate, umfassend die Umsetzung in beliebiger Reihenfolge einer heterocylcischen Halogenverbindung der Allgemeinen Formel (II)

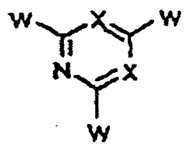

(II)

wobei die Symbole X die in einem der vorangehenden Ansprüche spezifizierte Bedeutung aufweisen und W ein Halogenatom darstellt, mit

(i) einer Verbindung der Allgemeinen Formel (III)

$$R_1\text{-}(CH_2)_p\text{-}Y\text{-}H \qquad \text{(III)}$$

wobei die Symbole $R_1$ p und Y die in einem der vorangehenden Ansprüche spezifizierte Bedeutung aufweisen,

(ii) einer Verbindung der Allgemeinen Formel (IV)

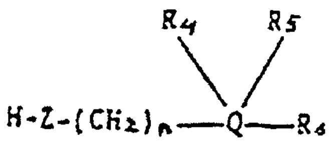

(IV)

wobei die Symbole $R_4$, $R_5$, $R_6$, Q, Z und n die in einem der vorangehenden Ansprüche spezifizierte Bedeutung aufweisen, und

(iii) einer gegebenenfalls derivatisierten Trägermatrix der Allgemeinen Formel (V)

$$H\text{-}T\text{-}[\text{-}L\text{-}V\text{-}]_m\text{-}M \qquad \text{(V)}$$

wobei die Symbole L, M, V T und m die in einem der vorangehenden Ansprüche spezifizierte Bedeutung aufweisen.

**27.** Verfahren zur Herstellung der neuen wie in einem der vorangehenden Ansprüche definierten Affinitätsligand-Matrix-Konjugate, umfassend das Umsetzen in beliebiger Reihenfolge einer wie in Anspruch 26 definierten heterocyclischen Halogenverbindung der Allgemeinen Formel (II) mit

(i) einer wie in Anspruch 26 definierten Verbindung der Allgemeinen Formel (III),
(ii) einer wie in Anspruch 26 definierten Verbindung der Allgemeinen Formel (IV) und
(iii) mit einer Verbindungseinheit der Allgemeinen Formel (VI)

$$H\text{-}T\text{-}L\text{-}V\text{-}H \qquad \text{(VI)}$$

wobei die Symbole L, V und T die in einem der vorangehenden Ansprüche spezifizierte Bedeutung aufweisen,
um eine Verbindung der Allgemeinen Formel (VII) zu erhalten,

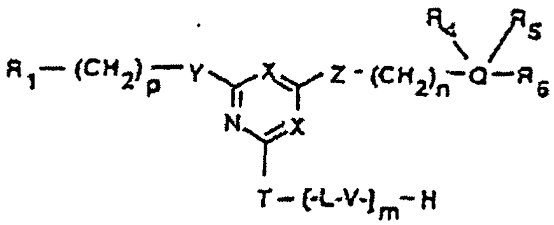

(VII)

wobei $R_1$, $R_4$, $R_5$, $R_6$, T, Q, L, V, X, Y, Z, m, n und p die in einem der vorangehenden Ansprüche spezifizierte Bedeutung aufweisen,
gefolgt von der Umsetzung der Verbindung der Allgemeinen Formel (VII) mit einem Matrixträger.

**28.** Neue Affinitätsliganden der Allgemeinen Formel (XII)

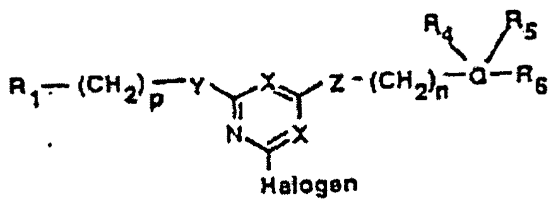

(XII)

wobei $R_1$, $R_4$, $R_5$, $R_6$, Q, X, Y, Z, n und p die in einem der vorangehenden Ansprüche spezifizierte Bedeutung aufweisen und Halogen ein Fluor-, Chlor-, Brom- oder Iodatom darstellt, mit der Maßgabe dass Formel (XII) nicht die Verbindungen 4-Chlor-2,6-di(phenylamino)-1,3,5-triazin-3'sulfonsäure, 4-Chlor-2,6-di(phenylamino)-1,3,5-triazin-3',2"-disulfon-säure und 4-Chlor-2-(4"-aminophenylamino)-1,3,5-triazin-3',2"-disulfon-säure umfasst.

**29.** Verfahren zum Anlagern von neuen Affinitätsliganden der wie in Anspruch 28 beanspruchten Allgemeinen Formel (XII) an eine Matrix der wie in Anspruch 26 definierten Allgemeinen Formel (V) durch Umsetzen der neuen Affinitätsliganden mit der Matrix bei Temperaturen zwischen - 20°C und 121°C gegebenenfalls in Gegenwart eines Säurebindemittels.

**30.** Neue Affinitätsliganden der Allgemeinen Formel (XIII)

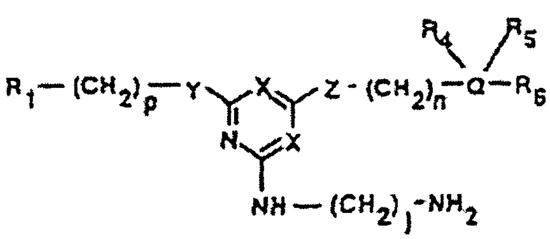

(XIII)

wobei $R_1$, $R_4$, $R_5$, $R_6$, Q, X, Y, Z, m, n und p die in einem der vorangehenden Ansprüche spezifizierte Bedeutung aufweisen und j eine ganze Zahl zwischen 2 und 20 ist.

**31.** Verfahren zur Herstellung von neuen Affinitätsliganden der wie in Anspruch 30 beanspruchten Allgemeinen Formel (XIII) durch Umsetzen einer Verbindung der wie in Anspruch 28 beanspruchten Allgemeinen Formel (XII) mit einem Alkylendiamin der Allgemeinen Formel $H_2N\text{-}(CH_2)_j\text{-}NH_2$ bei Temperaturen zwischen 0°C und 100°C in Gegenwart

eines Säurebindemittels.

**32.** Neue Affinitätsliganden der Allgemeinen Formel (XIV)

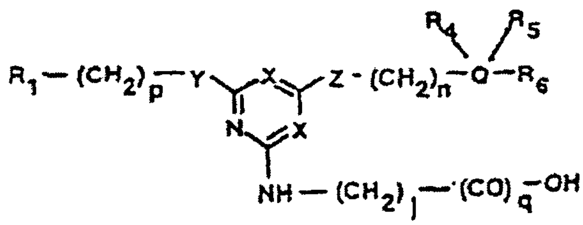

(XIV)

wobei $R_1$, $R_4$, $R_5$, Q, X, Y, Z, m, n und p die in einem der vorangehenden Ansprüche spezifizierte Bedeutung aufweisen, q 0 oder 1 ist und j eine ganze Zahl zwischen 2 und 20 ist.

**33.** Verfahren zur Herstellung von neuen Affinitätsliganden der wie in Anspruch 32 beanspruchten Allgemeinen Formel (XIV) durch Umsetzen einer Verbindung der wie in Anspruch 28 beanspruchten Allgemeinen Formel (XII) mit einer Hydroxyverbindung der Allgemeinen Formel $H_2N\text{-}(CH_2)_j\text{-}(CO)_qOH$ bei Temperaturen zwischen 0°C und 100°C in Gegenwart eines Säurebindemittels.

**34.** Neue Affinitätsliganden der Allgemeinen Formel (VIII)

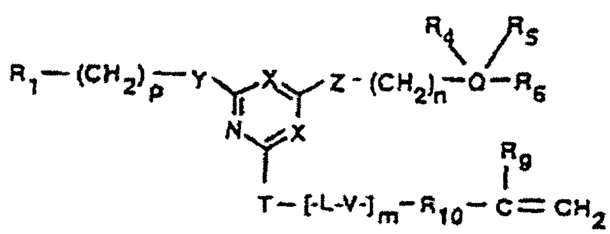

(VIII)

wobei $R_1$, $R_4$, $R_5$, $R_6$, L, Q, T, V, X, Y, Z, m, n und p die in einem der vorangehenden Ansprüche spezifizierte Bedeutung aufweisen, $R_9$ ein Wasserstoffatom oder eine 1 bis 6 Kohlenstoffatome enthaltende Alkylgruppe darstellt, $R_{10}$ eine Carbonylgruppe, eine Methylengruppe, eine $-NH-CH_2$-Gruppe oder eine $-S-CH_2$-Gruppe darstellt.

**35.** Neue Affinitätsliganden der Allgemeinen Formel (XV)

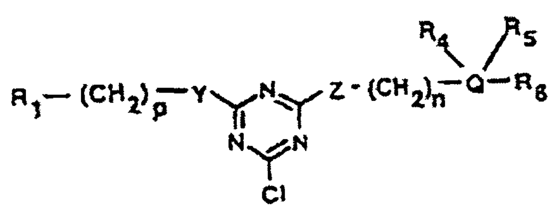

(XV)

wobei $R_1$, $R_4$, $R_5$, $R_6$, Q, n und p die in einem der vorangehenden Ansprüche spezifizierte Bedeutung aufweisen, mit der Maßgabe dass Formel (XV) nicht die Verbindungen 4-Chlor-2,6-di(phenylamino)-1,3,5-triazin-3'-sulfonsäure, 4-Chlor-2,6-di(phenylamino)-1,3,5-triazin-3',2"disulfon-säure und 4-Chlor-2-(4"-aminophenylamino)-1,3,5-triazin-3',2"disulfon-säure umfasst.

**36.** Neue Affinitätsliganden der Allgemeinen Formel (XVI)

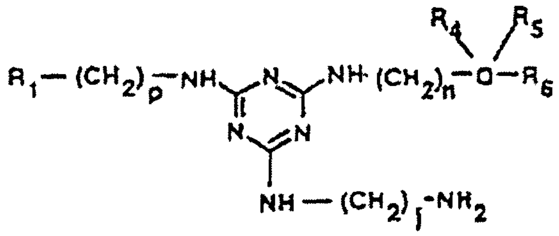

(XVI)

wobei $R_1$, $R_4$, $R_5$, $R_6$, Q, n und p die in einem der vorangehenden Ansprüche spezifizierte Bedeutung aufweisen und j eine ganze Zahl zwischen 2 und 20 ist.

**37.** Affinitätsliganden nach einem der Ansprüche 28, 30, 32, 34, 35 oder 36, wobei $R_1$ eine Phenyl- oder Naphthylgruppe darstellt, wobei jede davon gegebenenfalls am Benzol- oder Naphthalinring mit einem oder mehreren, unabhängig voneinander ausgewählt aus Hydroxygruppen und Carbonsäuregruppen, substituiert ist.

**38.** Affinitätsliganden nach einem der Ansprüche 28, 30, 32, 34, 35 oder 36, wobei $R_4$ ein Wasserstoffatom, eine Hydroxygruppe, eine Carbonsäuregruppe oder eine Aminogruppe darstellt.

**39.** Affinitätsliganden nach einem der Ansprüche 28, 30, 32, 34, 35 oder 36, wobei $R_5$ ein Wasserstoffatom, eine Hydroxygruppe, eine Carbonsäuregruppe oder eine Aminogruppe darstellt.

**40.** Affinitätsliganden nach einem der Ansprüche 28, 30, 32, 34, 35 oder 36, wobei $R_6$ ein Wasserstoffatom, eine Hydroxygruppe, eine Carbonsäuregruppe oder eine Aminogruppe darstellt.

**41.** Affinitätsliganden nach einem der Ansprüche 28, 30, 32, 34, 35 oder 36, wobei Q einen Benzol- oder Naphthalinring darstellt.

**42.** Affinitätsliganden nach einem der Ansprüche 28, 30, 32 oder 34, wobei X ein Stickstoffatom darstellt.

**43.** Affinitätsliganden nach einem der Ansprüche 28, 30, 32, 34 oder 35, wobei Y eine -NH-Gruppe darstellt.

**44.** Affinitätsliganden nach einem der Ansprüche 28, 30, 32, 34 oder 35, wobei Z eine -NH-Gruppe darstellt.

**45.** Affinitätsliganden nach einem der Ansprüche 28, 30, 32, 34, 35 oder 36, wobei n 0 oder 2 ist.

**46.** Affinitätsliganden nach einem der Ansprüche 28, 30, 32, 34, 35 oder 36, wobei p 0 oder 2 ist.

**47.** Affinitätsliganden nach einem der Ansprüche 30, 32 oder 36, wobei j 2, 4 oder 6 ist.

**48.** Affinitätsliganden nach Anspruch 34, wobei L eine Ethyl-, Butyl- oder Hexylgruppe ist.

**49.** Affinitätsliganden nach Anspruch 34, wobei T eine -NH-Gruppe darstellt.

**50.** Affinitätsliganden nach Anspruch 34, wobei V eine -NH-Gruppe darstellt.

**51.** Affinitätsliganden nach Anspruch 34, wobei m 1 ist.

**52.** Neue Affinitätsliganden der Allgemeinen Formel (XI)

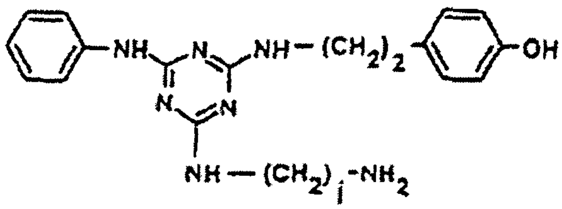

(XI)

wobei j eine ganze Zahl zwischen 2 und 20 ist.

**53.** Affinitätsliganden nach einem der Ansprüche 28 bis 51, ausgewählt aus

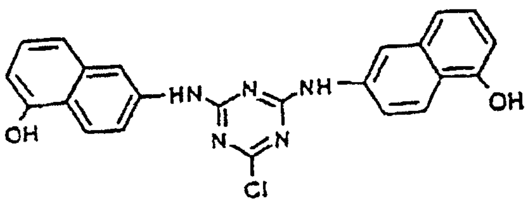

(1)

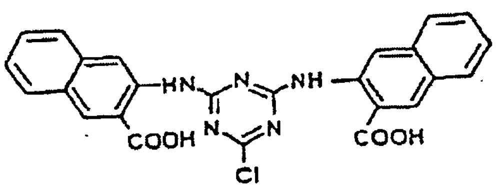

(2)

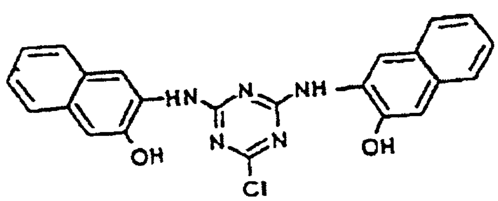

(3)

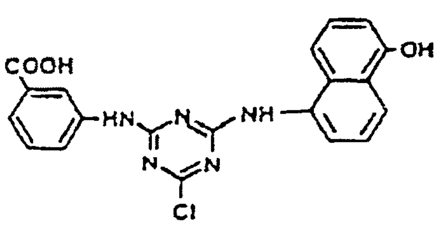

(4)

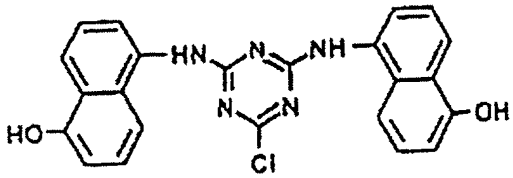

(5)

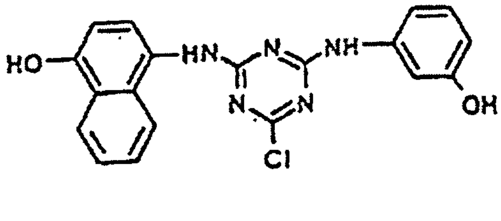

(6)

(7)

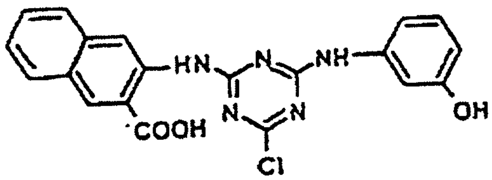

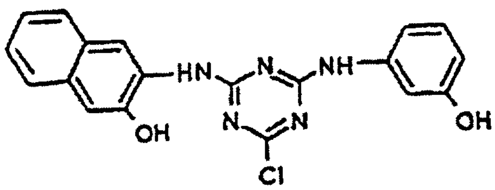

(8)

16

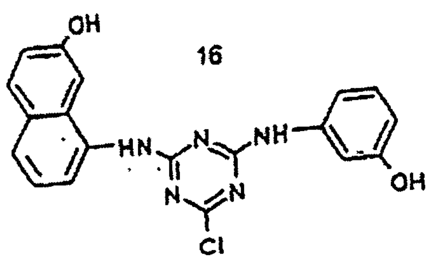

(9)

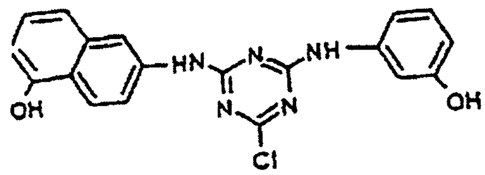

(10)

(11)

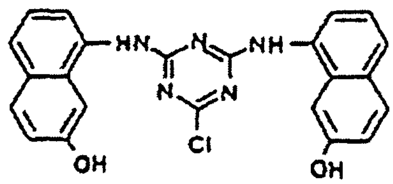

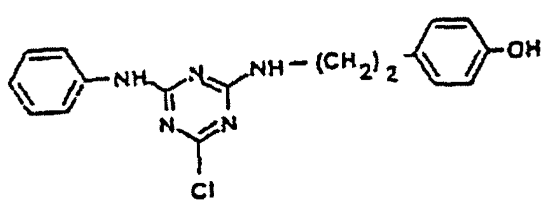

(12)

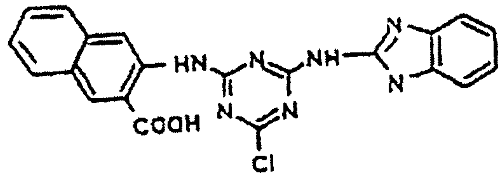

(13)

**54.** Verwendung von Affinitätsliganden nach einem der vorangehenden Ansprüche zur Herstellung von Affinitätsligand-Matrix-Konjugaten.

**55.** Verfahren zum Anlagern der neuen Affinitätsliganden der wie in Anspruch 27 definierten Allgemeinen Formeln (VII) und wie in Anspruch 30 definierten (XIII), wie in Anspruch 36 definierten (XVI) und wie in Anspruch 52 definierten (XI) an Kohlenhydrat- oder organischen Polymermatrizen durch Umsetzen der Kohlenhydrat- oder organischen Polymermatrix mit einem Aktivierungsmittel, gefolgt von der Umsetzung der aktivierten Matrix mit dem neuen Affinitätsliganden gegebenenfalls in Gegenwart eines Säurebindemittels.

**56.** Verfahren zum Anlagern der neuen Affinitätsliganden der wie in Anspruch 32 definierten Allgemeinen Formeln (XIV) an Kohlenhydrat- oder organischen Polymermatrizen durch Kondensation mit der Matrix.

**57.** Verfahren zum Anlagern der neuen Affinitätsliganden der wie in Anspruch 27 definierten Allgemeinen Formeln (VII) und wie in Anspruch 30 definierten (XIII), wie in Anspruch 36 definierten (XVI) und wie in Anspruch 52 definierten (XI) an gegebenenfalls mit einem organischen Polymer beschichtete Metalloxid-, Glas- oder Siliciumdioxidmatrizen durch Umsetzen der gegebenenfalls beschichteten Metalloxid-, Glas- oder Siliciumdioxidmatrix mit einem Aktivierungsmittel, gefolgt von der Umsetzung der aktivierten Matrix mit dem neuen Affinitätsliganden gegebenenfalls in Gegenwart eines Säurebindemittels.

**58.** Verfahren zum Anlagern der neuen Affinitätsliganden der wie in Anspruch 32 definierten Allgemeinen Formel (XIV) an gegebenenfalls mit einem organischen Polymer beschichtete Metalloxid-, Glas oder Siliciumdioxidmatrizen durch Kondensation mit der Matrix.

**59.** Verfahren zum Anlagern von neuen Affinitätsliganden der wie in Anspruch 35 definierten Allgemeinen Formel (XV) und wie in Anspruch 28 definierten (XII) an eine Matrix der wie in Anspruch 26 definierten Allgemeinen Formel (V) durch Umsetzen der neuen Affinitätsliganden mit der Matrix bei Temperaturen zwischen -20°C und 121°C gegebenenfalls in Gegenwart eines Säurebindemittels.

**60.** Affinitätsligand-Matrix-Konjugate, hergestellt wie in den Ansprüchen 26, 27, 29, 54, 55, 56, 57 und 58 beansprucht.

**61.** Verwendung der Affinitätsligand-Matrix-Konjugate nach einem der vorangehenden Ansprüche als Affinitätsligand-Matrix-Konjugate zur Abtrennung, Isolierung, Reinigung, Charakterisierung, Identifizierung oder Quantifizierung von Proteinen.

**62.** Verwendung nach Anspruch 61, wobei es sich bei dem proteinhaltigen Material um IgG, IgM, IgA, Insuline, Faktor VII oder Menschliches Wachstumshormon oder Analoga, Derivate und Fragmente davon und Vorstufen handelt.

**63.** Verfahren zur Abtrennung oder Reinigung von proteinhaltigen Materialien, umfassend das Durchführen von Affinitätschromatographie unter Verwendung eines Liganden der wie vorstehend definierten allgemeinen Formel (a) als den biospezifischen Liganden.

**64.** Verwendung nach Anspruch 61, wobei es sich bei dem proteinhaltigen Material um Immunglobuline oder Unterklassen, Fragmente, Vorstufen oder Derivate davon handelt, die entweder von natürlichen oder rekombinanten Quellen abgeleitet sind.

**65.** Verwendung nach Anspruch 61, wobei es sich bei dem proteinhaltigen Material um Immunglobulin G (IgG), Immunglobulin M (IgM), Immunglobulin A (IgA) oder Unterklassen, Fragmente, Vorstufen oder Derivate davon handelt, die entweder von natürlichen oder rekombinanten Quellen abgeleitet sind.

**66.** Verwendung nach Anspruch 61, wobei es sich bei dem proteinhaltigen Material um Insuline oder Insulinanaloga, Derivate und Fragmente davon und Vorstufen handelt, die entweder von natürlichen oder rekombinanten Quellen abgeleitet sind.

**67.** Verwendung nach Anspruch 61, wobei es sich bei dem proteinhaltigen Material um FVII oder Analoga, Derivate und Fragmente davon und Vorstufen handelt, die von natürlichen oder rekombinanten Quellen abgeleitet sind.

**68.** Verwendung nach Anspruch 61, wobei die Affinitätsligand-Matrix-Konjugate einen Liganden umfassen, der aus Folgendem ausgewählt ist:

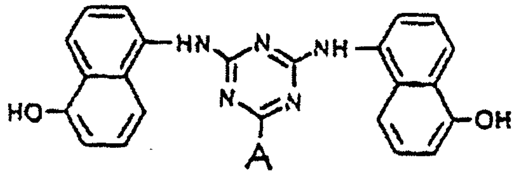

(5a)

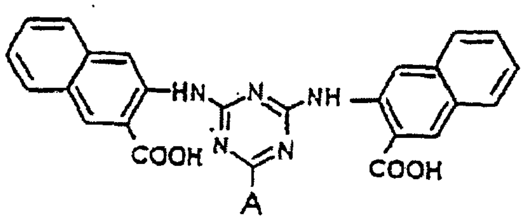

(2a)

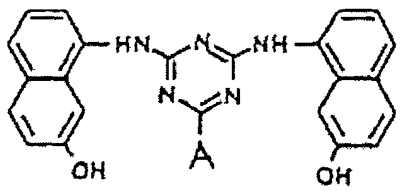

(11a)

wobei der Ligand gegebenenfalls durch einen Abstandhalterarm der wie vorstehend spezifizierten allgemeinen Formel (b) an eine Trägermatrix in Position (A) angelagert ist.

**69.** Verwendung nach Anspruch 68, wobei es sich bei dem Liganden um 11 a handelt.

**70.** Verwendung nach Anspruch 68 oder 69, wobei es sich bei der Trägermatrix um gegebenenfalls aktivierte/s Agarose, Cellulose, Siliciumdioxid oder Glas handelt.

**71.** Abtrennung, Isolierung, Reinigung, Charakterisierung, Identifizierung oder Quantifizierung von Immunglobulinen durch ein beliebiges Verfahren, wodurch die Immunglobuline auf wie in einem der vorstehenden Ansprüche für Affinitätsligand-Matrix-Konjugate definierte Affinitätsligand-Matrix-Konjugate bei einem pH-Wert im Bereich von 5,0 bis 12,0 aufgebracht und anschließend durch Senken des pH-Werts auf 4,9 oder niedriger entfernt, eluiert oder desorbiert werden.

**72.** Abtrennung, Isolierung, Reinigung, Charakterisierung, Identifizierung oder Quantifizierung von Insulinen oder Insulinanaloga oder Derivaten davon und Vorstufen durch ein beliebiges Verfahren, wodurch die Insuline auf wie in einem der vorstehenden Ansprüche für Affinitätsligand-Matrix-Konjugate definierte Affinitätsligand-Matrix-Konjugate bei einem pH-Wert im Bereich von 4,0 bis 9,0 aufgebracht und anschließend durch Senken des pH-Werts auf 3,99 oder niedriger oder 9,01 oder höher entfernt, eluiert oder desorbiert werden.

**73.** Abtrennung von rekombinantem FVIIa durch selektives Binden von FVIIa an die Affinitätsmatrix der Formel (viii)

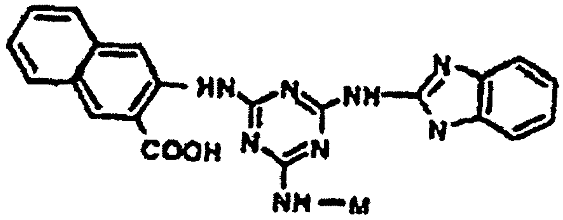

wobei M Aminogruppen tragende Agarose ist, von Zellkulturmedien, wodurch der FVIIa auf das Affinitätsligand-Matrix-Konjugat in Gegenwart von 5 mM $Ca^{2+}$ aufgebracht und anschließend eluiert wird.

## Revendications

**1.** Conjugués ligand d'affinité-matrice, comprenant un ligand de formule générale (a) :

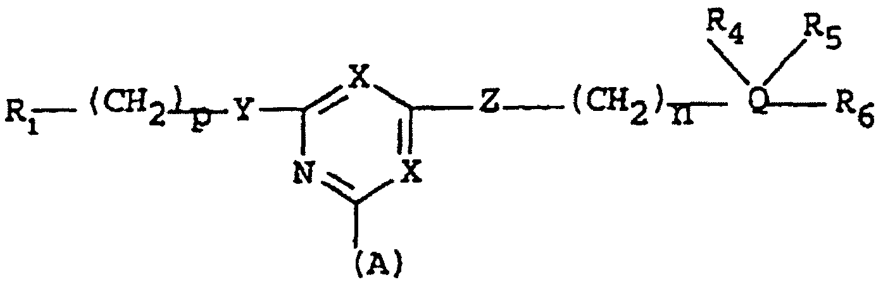

(a)

dans laquelle

$R_1$ représente un atome d'hydrogène, un groupe alkyle ayant de 1 à 6 atomes de carbone, un groupe hydroxyalkyle ayant de 1 à 6 atomes de carbone un groupe cyclohexyle, un groupe amino, un groupe phényle, un groupe naphtyle, un groupe 1-phénylpyrazole, indazole, benzothiazole, un groupe benzoxazole ou un groupe benzimidazole, chaque cycle benzène, naphtalène, 1-phénylpyrazole, indazole, benzothiazole, benzoxazole ou benzimidazole étant éventuellement substitué par un ou plusieurs substituants choisis indépendamment les uns des autres dans l'ensemble consistant en les groupes alkyle ayant de 1 à 6 atomes de carbone, les groupes alcoxy ayant de 1 à 6 atomes de carbone, les groupes acyloxy ou acylamino ayant de 1 à 6 atomes de carbone, les groupes amino, les groupes hydroxyle, les groupes acide carboxylique, les groupes acide sulfonique, les groupes carbamoyle, les groupes sulfamoyle, les groupes alkylsulfonyle ayant de 1 à 6 atomes de carbone et les atomes d'halogène ;

Y représente un atome d'oxygène, un atome de soufre ou un groupe N-$R_2$ ;

Z représente un atome d'oxygène, un atome de soufre ou un groupe N-$R_3$ ;

$R_2$ et $R_3$ représentent chacun indépendamment de l'autre un atome d'hydrogène, un groupe alkyle ayant de 1 à 6 atomes de carbone ; un groupe hydroxyalkyle ayant de 1 à 6 atomes de carbone, un groupe benzyle ou un groupe β-phényléthyle ;

$R_4$, $R_5$ et $R_6$ représentent chacun indépendamment des autres un atome d'hydrogène, un groupe hydroxyle, un groupe alkyle ayant de 1 à 6 atomes de carbone, un groupe alcoxy ayant de 1 à 6 atomes de carbone, un groupe amino, un groupe acyloxy ou acylamino ayant de 1 à 6 atomes de carbone, un groupe acide carboxylique, un groupe acide sulfonique, un groupe carbamoyle ou sulfamoyle, un groupe alkylsulfonyle ayant de 1 à 6 atomes de carbone ou un atome d'halogène ;

l'un des symboles X représente un atome d'azote et l'autre symbole X représente un atome d'azote ou un atome de carbone portant un atome de chlore ou un groupe cyano ;

Q représente un noyau benzène, naphtalène, 1-phénylpyrazole, indazole, benzothiazole, benzoxazole ou benzimidazole ;

n est un entier compris entre 0 et 6 ;

p est un entier compris entre 0 et 20 ;

et

ledit ligand est fixé à une matrice support en position (A), éventuellement par l'intermédiaire d'un bras espaceur intercalé entre la matrice et le ligand,

à la condition que la formule (a) ne comprenne pas les produits de réaction, avec le Dextran T500, des composés acide 4-chloro-2,6-di(phénylamino)-1,3,5-triazine-3'-sulfonique, acide 4-chloro-2,6-di(phénylamino)-1,3,5-triazine-3',2"-disulfonique et acide 4-chloro-2-(4"-amino-phénylamino)-2,3,5-triazine-3',2"-disulfonique.

**2.** Conjugués ligand d'affinité-matrice selon la revendication 1, dans lesquels le bras espaceur en option intercalé entre le ligand et la matrice est représenté par la formule générale (b)

$$-T-[L-V-]_m- \quad (b)$$

dans laquelle T représente un atome d'oxygène, un atome de soufre ou un groupe $N-R_7$ ; où $R_7$ représente un atome d'hydrogène ou un groupe alkyle ayant de 1 à 6 atomes de carbone ;

V représente un atome d'oxygène, un atome de soufre, un groupe -COO-, un groupe CONH ou un groupe NHCO ou un groupe $-PO_3H-$, un groupe NH-arylène-$SO_2$-$CH_2$-$CH_2$ ou un groupe $N-R_8$, où $R_8$ représente un atome d'hydrogène ou un groupe alkyle ayant de 1 à 6 atomes de carbone ;

L représente une liaison hydrocarbonée éventuellement substituée et ayant de 2 à 20 atomes de carbone ; et

m vaut 0 ou 1.

**3.** Conjugués ligand d'affinité-matrice selon la revendication 1, qui sont représentés par la formule générale (I) :*

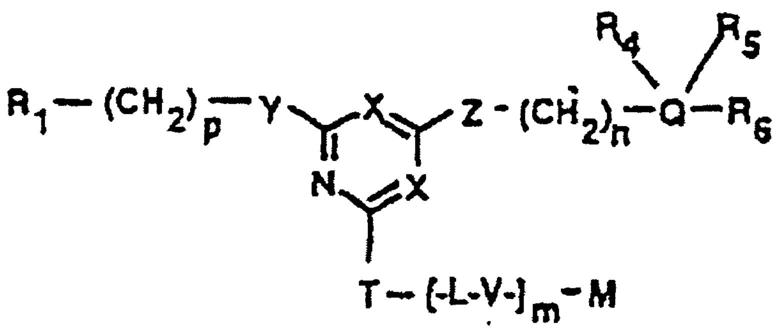

(I)

dans laquelle

$R_1$, Y, Z, $R_2$, $R_3$, $R_4$, $R_5$, $R_6$, X, Q, n et p sont tels que définis ci-dessus,

T représente un atome d'oxygène, un atome de soufre ou un groupe $N-R_7$ ;

V représente un atome d'oxygène, un atome de soufre, un groupe -COO-, un groupe CONH ou un groupe NHCO ou un groupe $-PO_3H-$, un groupe NH-arylène-$SO_2$-$CH_2$-$CH_2$ ou un groupe $N-R_8$ ;

$R_7$ et $R_8$ représentent chacun indépendamment de l'autre un atome d'hydrogène ou un groupe alkyle ayant de 1 à 6 atomes de carbone ;

L représente une liaison hydrocarbonée éventuellement substituée et ayant de 2 à 20 atomes de carbone ;

m vaut 0 ou 1 ; et

M représente le résidu d'une matrice support.

**4.** Conjugués ligand d'affinité-matrice selon l'une quelconque des revendications précédentes, dans lesquels M représente le résidu d'une matrice support qui peut être un composé ou un matériau quelconque, particulaire ou non particulaire, soluble ou insoluble, poreux ou non poreux, qui peut être utilisé en liaison avec des ligands d'affinité pour former un nouveau conjugué ligand d'affinité-matrice selon l'une quelconque des revendications précédentes, et qui donne un moyen commode permettant de séparer les ligands d'affinité d'avec des solutés dans une solution de contact.

**5.** Conjugués ligand d'affinité-matrice selon l'une quelconque des revendications précédentes, dans lesquels $R_1$ représente un groupe phényle ou naphtyle, dont chacun est éventuellement substitué sur le noyau benzène ou naphtalène par un ou plusieurs substituants choisis indépendamment les uns des autres parmi les groupes hydroxyle et les groupes acide carboxylique.

**6.** Conjugués ligand d'affinité-matrice selon l'une quelconque des revendications précédentes, dans lesquels $R_2$ re-

présente un atome d'hydrogène.

**7.** Conjugués ligand d'affinité-matrice selon l'une quelconque des revendications précédentes, dans lesquels $R_3$ représente un atome d'hydrogène.

**8.** Conjugués ligand d'affinité-matrice selon l'une quelconque des revendications précédentes, dans lesquels $R_4$ représente un atome d'hydrogène, un groupe hydroxyle, un groupe acide carboxylique ou un groupe amino.

**9.** Conjugués ligand d'affinité-matrice selon l'une quelconque des revendications précédentes, dans lesquels $R_5$ représente un atome d'hydrogène, un groupe hydroxyle, un groupe acide carboxylique ou un groupe amino.

**10.** Conjugués ligand d'affinité-matrice selon l'une quelconque des revendications précédentes, dans lesquels $R_6$ représente un atome d'hydrogène, un groupe hydroxyle, un groupe acide carboxylique ou un groupe amino.

**11.** Conjugués ligand d'affinité-matrice selon l'une quelconque des revendications précédentes, dans lesquels $R_7$ représente un atome d'hydrogène.

**12.** Conjugués ligand d'affinité-matrice selon l'une quelconque des revendications précédentes, dans lesquels T représente un atome d'oxygène ou un groupe NH.

**13.** Conjugués ligand d'affinité-matrice selon l'une quelconque des revendications précédentes, dans lesquels Y représente N-$R_2$, $R_2$ étant tel que défini ci-dessus.

**14.** Conjugués ligand d'affinité-matrice selon l'une quelconque des revendications précédentes, dans lesquels Z représente N-$R_3$, $R_3$ étant tel que défini ci-dessus.

**15.** Conjugués ligand d'affinité-matrice selon l'une quelconque des revendications précédentes, dans lesquels les deux radicaux X sont des atomes d'azote.

**16.** Conjugués ligand d'affinité-matrice selon l'une quelconque des revendications précédentes, dans lesquels Q représente un noyau benzène ou naphtalène.

**17.** Conjugués ligand d'affinité-matrice selon l'une quelconque des revendications précédentes, dans lesquels n vaut 0 ou 2.

**18.** Conjugués ligand d'affinité-matrice selon l'une quelconque des revendications précédentes, dans lesquels p vaut 0 ou 2.

**19.** Conjugués ligand d'affinité-matrice selon l'une quelconque des revendications précédentes, dans lesquels m vaut 0 ou 1.

**20.** Conjugués ligand d'affinité-matrice selon l'une quelconque des revendications précédentes, dans lesquels L représente un groupe éthyle, propyle, hydroxypropyle, butyle, pentyle, hexyle, octyle ou décyle, et V et m sont tels que définis ci-dessus.

**21.** Conjugué ligand d'affinité-matrice selon l'une quelconque des revendications précédentes, dans lequel V représente un atome d'oxygène ou un groupe NH, et L et m sont tels que définis ci-dessus.

**22.** Conjugués ligand d'affinité-matrice selon l'une quelconque des revendications précédentes, dans lesquels m vaut 1, et L et V sont tels que définis ci-dessus.

**23.** Conjugués ligand d'affinité-matrice selon l'une quelconque des revendications précédentes, dans lesquels le résidu d'une matrice support M représente une agarose éventuellement activée, une silice, une cellulose, du verre, une résine Toyopearl, du méthacrylate d'hydroxyéthyle, du polyacrylamide, du styrène, du divinylbenzène, de l'Hyper D, des composés perfluorocarbonés.

**24.** Conjugués ligand d'affinité-matrice selon la revendication 23, dans lesquels M représente une agarose éventuellement activée par un groupe trésyle, par du chlorure de sulfonyle, par un groupe tosyle, par une vinylsulfone ou

par un groupe époxy.

**25.** Conjugués ligand d'affinité-matrice selon l'une quelconque des revendications précédentes, choisis parmi les composés suivants :

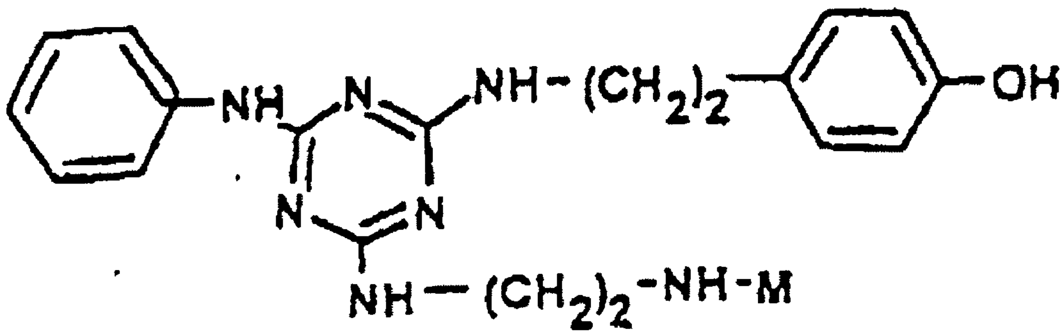

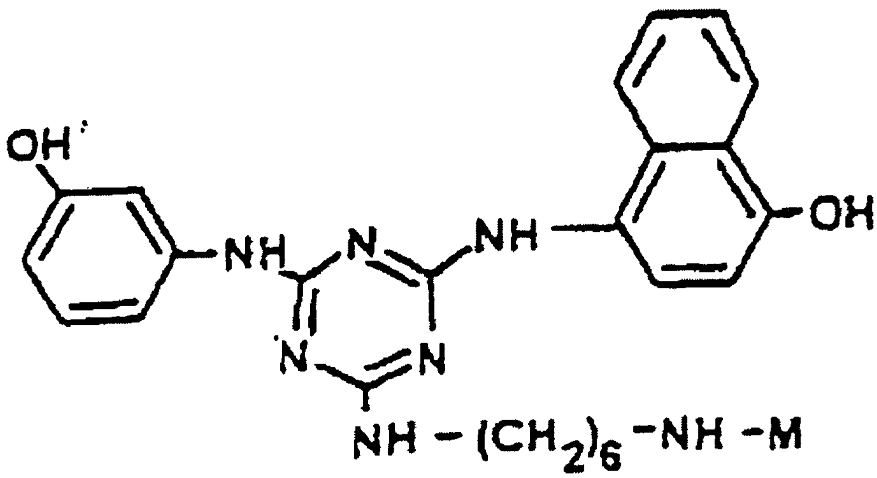

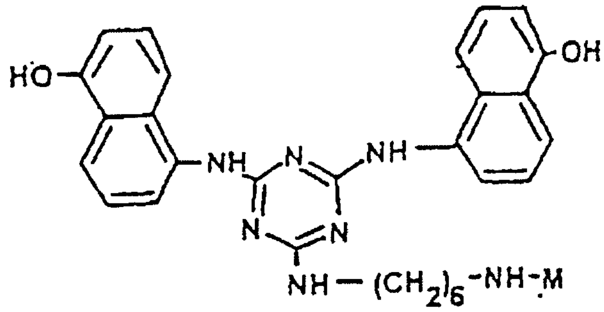

iii)

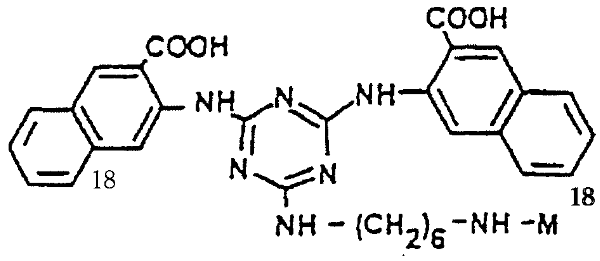

iv)

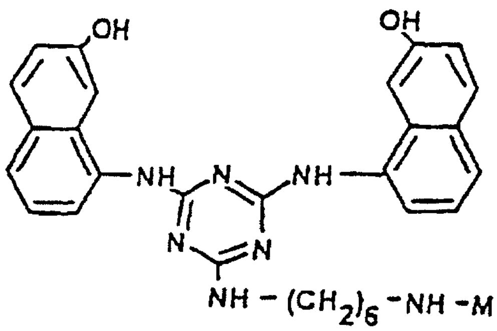

v)

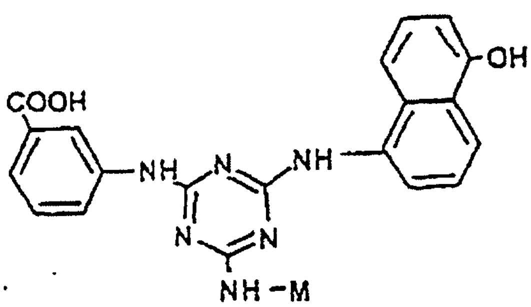

vi)

vii) 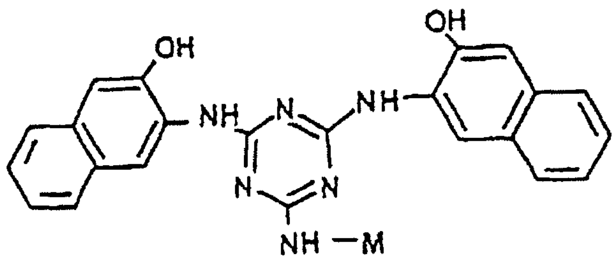

viii) 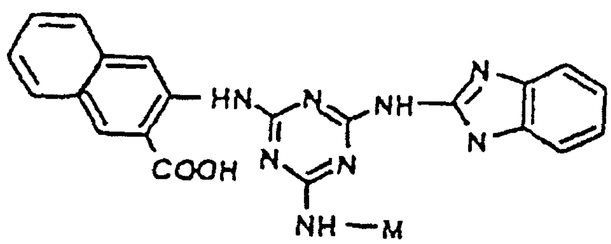

où M est tel que défini ci-dessus.

**26.** Procédé de préparation des nouveaux conjugués ligand d'affinité-matrice selon l'une quelconque des revendications précédentes, qui comprend la réaction, dans un ordre quelconque, d'un composé halogéno-hétérocyclique de formule (II) :

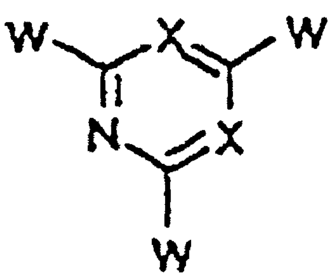

(II)

dans laquelle les symboles X ont les significations données dans l'une quelconque des revendications précédentes, et W représente un atome d'halogène, avec

(i) un composé de formule (III) :

$$R_1\text{-}(CH_2)_p\text{-}Y\text{-}H \qquad \text{(III)}$$

dans laquelle les symboles $R_1$, p et Y ont les significations données dans l'une quelconque des revendications précédentes,

(ii) un composé de formule générale (IV)

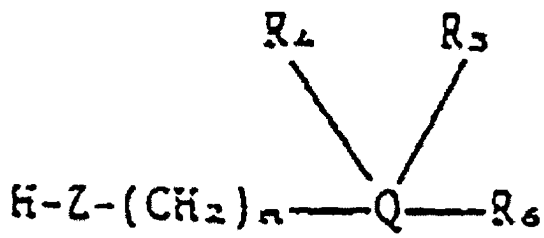

(IV)

dans laquelle les symboles $R_4$, $R_5$, $R_6$, Q, Z et n ont les significations données dans l'une quelconque des revendications précédentes, et

(iii) une matrice support éventuellement dérivatisée de formule générale (V) :

$$H\text{-}T\text{-}\ [L\text{-}V\text{-}]_m\text{-}M \qquad (V)$$

dans laquelle les symboles L, M, V, T et m ont les significations données dans l'une quelconque des revendications précédentes.

**27.** Procédé de préparation des nouveaux conjugués ligand d'affinité-matrice selon l'une quelconque des revendications précédentes, qui comprend la réaction, dans un ordre quelconque, d'un composé halogéno-hétérocyclique de formule générale (II) telle que définie dans la revendication 26, avec

(i) un composé de formule générale (III) telle que définie dans la revendication 26,
(ii) un composé de formule générale (IV) telle que définie dans la revendication 26, et
(iii) avec une unité de liaison de formule générale (VI) :

$$H\text{-}T\text{-}L\text{-}V\text{-}H \qquad (VI)$$

dans laquelle les symboles L, V et T ont les significations données dans l'une quelconque des revendications précédentes, pour donner un composé de formule générale (VII) :

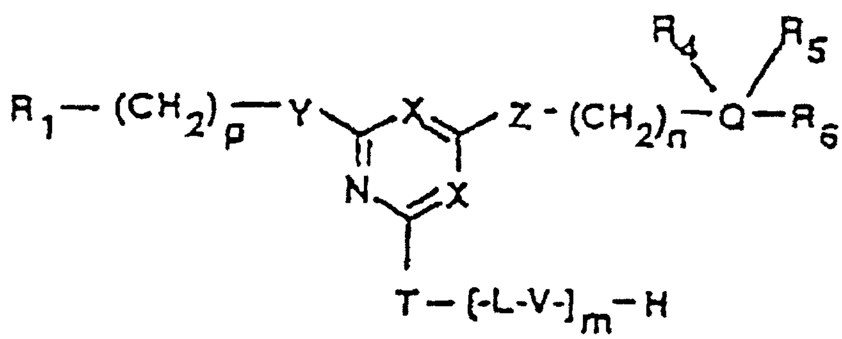

(VII)

dans laquelle $R_1$, $R_4$, $R_5$, $R_6$, T, Q, L, V, X, Y, Z, m, n et p ont les significations données dans l'une quelconque des revendications précédentes, puis la réaction du composé de formule générale (VII) avec une matrice support.

**28.** Nouveaux ligands d'affinité de formule générale (XII) :

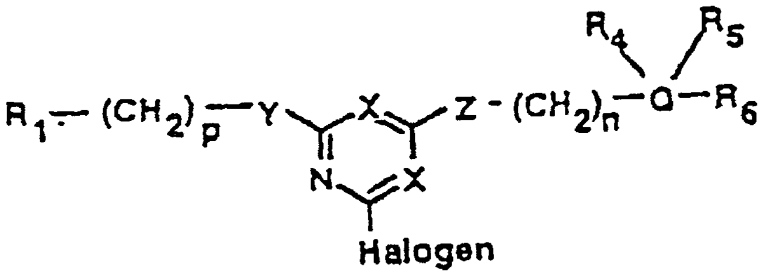

(XII)

dans laquelle $R_1$, $R_4$, $R_5$, $R_6$, Q, X, Y, Z, n et p ont les significations données dans l'une quelconque des revendications précédentes, et Halogen représente un atome de fluor, de chlore, de brome ou d'iode, à la condition que la formule (XII) ne comprenne pas les composés acide 4-chloro-2,6-di(phénylamino)-1,3,5-triazine-3'-sulfonique, acide 4-chloro-2,6-di(phénylamino)-1,3,5-triazine-3',2"-disulfonique et acide 4-chloro-2-(4"-amino-phénylamino)-1,3,5-triazine-3',2"-disulfonique.

**29.** Procédé de fixation des nouveaux ligands d'affinité de formule générale (XII) selon la revendication 28 à une matrice de formule générale (V) selon la revendication 26 par réaction des nouveaux ligands d'affinité avec la matrice à des températures comprises entre -20 et 121°C, éventuellement en présence d'un agent liant les acides.

**30.** Nouveaux ligands d'affinité de formule générale (XIII) :

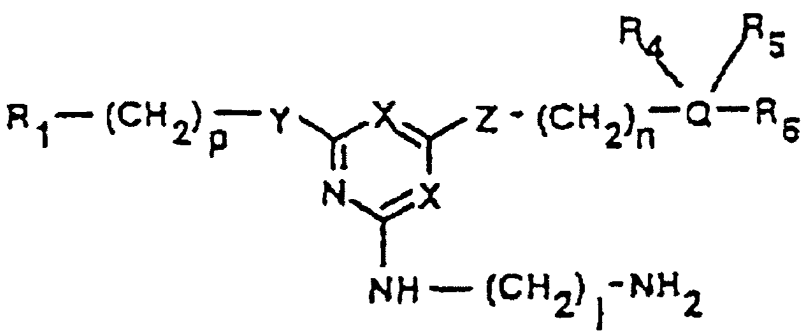

(XIII)

dans laquelle $R_1$, $R_4$, $R_5$, $R_6$, Q, X, Y, Z, m, n et p ont les significations données dans l'une quelconque des revendications précédentes, et j est un entier compris entre 2 et 20.

**31.** Procédé de préparation des nouveaux ligands d'affinité de formule générale (XIII) selon la revendication 30 par réaction d'un composé de formule générale (XII) selon la revendication 28 avec une alkylènediamine de formule générale $H_2N-(CH_2)_j-NH_2$ à des températures comprises entre 0 et 100°C en présence d'un agent liant les acides.

**32.** Nouveaux ligands d'affinité de formule générale (XIV) :

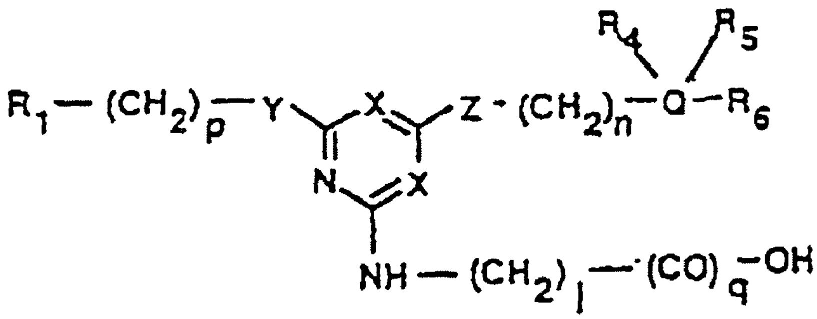

(XIV)

dans laquelle $R_1$, $R_4$, $R_5$, Q, X, Y, Z, m, n et p ont les significations données dans l'une quelconque des revendications précédentes, q vaut 0 ou 1, et j est un entier compris entre 2 et 20.

**33.** Procédé de préparation de nouveaux ligands d'affinité de formule générale (XIV) selon la revendication 32 par réaction d'un composé de formule générale (XII) selon la revendication 28 avec un composé aminohydroxylé de formule générale $H_2N-(CH_2)_j-(CO)_q-OH$ à des températures comprises entre 0 et 100°C, éventuellement en présence d'un agent liant les acides.

**34.** Nouveaux ligands d'affinité de formule générale (VIII) :

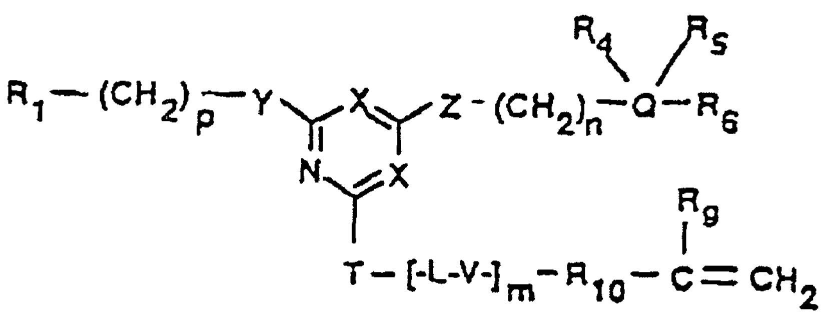

(VIII)

dans laquelle $R_1$, $R_4$, $R_5$, $R_6$, L, Q, T, V, X, Y, Z, m, n et p ont les significations données dans l'une quelconque des revendications précédentes ; $R_9$ est un atome d'hydrogène ou un groupe alkyle ayant de 1 à 6 atomes de carbone ; $R_{10}$ représente un groupe carbonyle, un groupe méthylène, un groupe $-NH-CH_2-$ ou un groupe $-S-CH_2-$,

**35.** Nouveaux ligands d'affinité de formule générale (XV) :

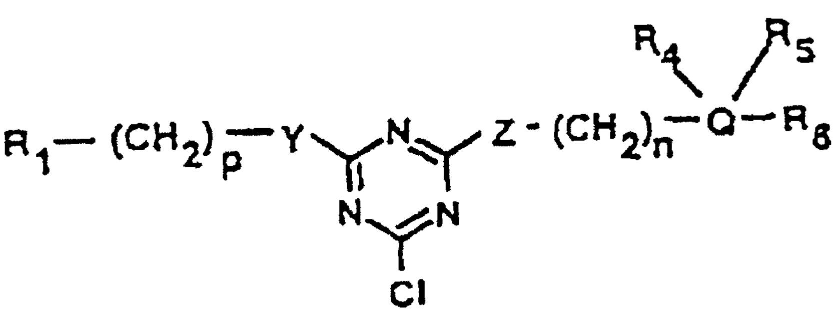

(XV)

dans laquelle $R_1$, $R_4$, $R_5$, $R_6$, Q, n et p ont les significations données dans l'une quelconque des revendications précédentes, à la condition que la formule (XV) ne comprenne pas les composés acide 4-chloro-2,6-di(phénylamino)-1,3,5-triazine-3'-sulfonique, acide 4-chloro-2,6-di(phénylamino)-1,3,5-triazine-3',2''-disulfonique et acide 4-chloro-2-(4''-amino-phénylamino)-1,3,5-triazine-3',2''-disulfonique.

**36.** Nouveaux ligands d'affinité de formule générale (XVI) :

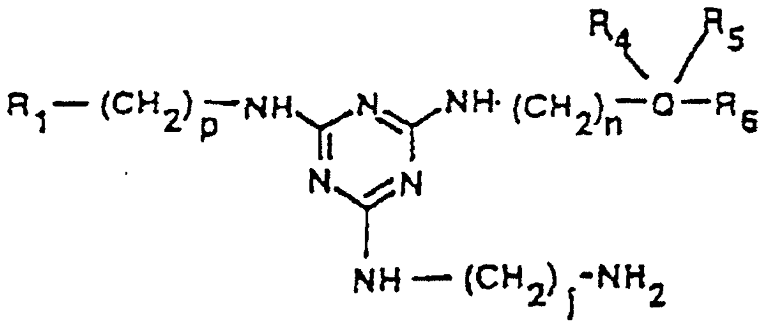

(XVI)

dans laquelle $R_1$, $R_4$, $R_5$, $R_6$, Q, n et p ont les significations données dans l'une quelconque des revendications précédentes, et j est un entier compris entre 2 et 20.

**37.** Ligands d'affinité selon l'une quelconque des revendications 28, 30, 32, 34, 35 ou 36, dans lesquels $R_1$ représente un groupe phényle ou naphtyle, dont chacun est éventuellement substitué sur le noyau benzène ou naphtalène par un ou plusieurs substituants choisis indépendamment les uns des autres parmi les groupes hydroxyle et les groupes acide carboxylique.

**38.** Ligands d'affinité selon l'une quelconque des revendications 28, 30, 32, 34, 35 ou 36, dans lesquels $R_4$ est un atome d'hydrogène, un groupe hydroxyle, un groupe acide carboxylique ou un groupe amino.

**39.** Ligands d'affinité selon l'une quelconque des revendications 28, 30, 32, 34, 35 ou 36, dans lesquels $R_5$ est un atome d'hydrogène, un groupe hydroxyle, un groupe acide carboxylique ou un groupe amino.

**40.** Ligands d'affinité selon l'une quelconque des revendications 28, 30, 32, 34, 35 ou 36, dans lesquels $R_6$ est un atome d'hydrogène, un groupe hydroxyle, un groupe acide carboxylique ou un groupe amino.

**41.** Ligands d'affinité selon l'une quelconque des revendications 28, 30, 32, 34, 35 ou 36, dans lesquels Q est un noyau benzène ou naphtalène.

**42.** Ligands d'affinité selon l'une quelconque des revendications 28, 30, 32 ou 34, dans lesquels X est un atome d'azote.

**43.** Ligands d'affinité selon l'une quelconque des revendications 28, 30, 32, 34 ou 35, dans lesquels Y est un groupe -NH-.

**44.** Ligands d'affinité selon l'une quelconque des revendications 28, 30, 32, 34 ou 35, dans lesquels Z est un groupe -NH-.

**45.** Ligands d'affinité selon l'une quelconque des revendications 28, 30, 32, 34, 35 ou 36, dans lesquels n vaut 0 ou 2.

**46.** Ligands d'affinité selon l'une quelconque des revendications 28, 30, 32, 34, 35 ou 36, dans lesquels p vaut 0 ou 2.

**47.** Ligands d'affinité selon l'une quelconque des revendications 30, 32 ou 36, dans lesquels j vaut 2, 4 ou 6.

**48.** Ligands d'affinité selon la revendication 34, dans lesquels L est un groupe éthyle, butyle ou hexyle.

**49.** Ligands d'affinité selon la revendication 34, dans lesquels T est un groupe -NH-.

**50.** Ligands d'affinité selon la revendication 34, dans lesquels V est un groupe -NH-.

**51.** Ligands d'affinité selon la revendication 34, dans lesquels m vaut 1.

**52.** Nouveaux ligands d'affinité de formule générale (XI) :

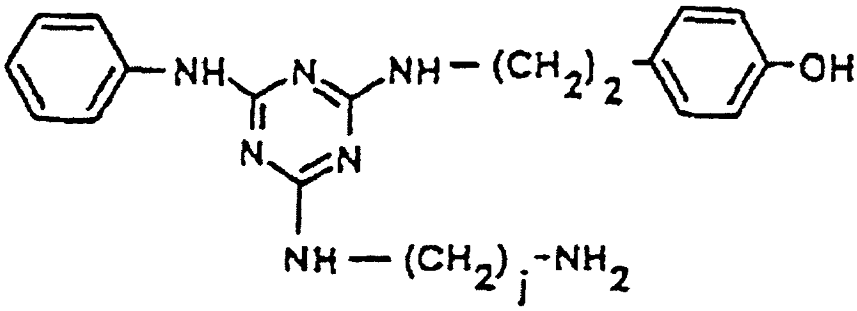

(XI)

dans laquelle j est un entier compris entre 2 et 20.

**53.** Ligands d'affinité selon l'une quelconque des revendications 28 à 51, choisis parmi les composés suivants :

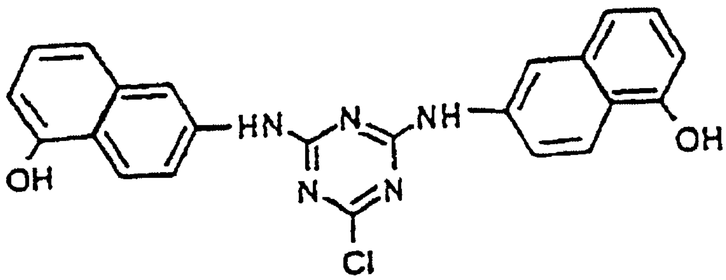

(1)

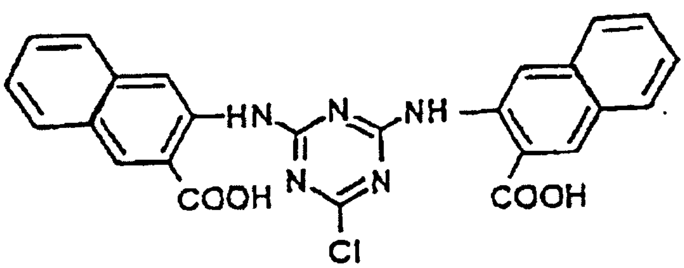

(2)

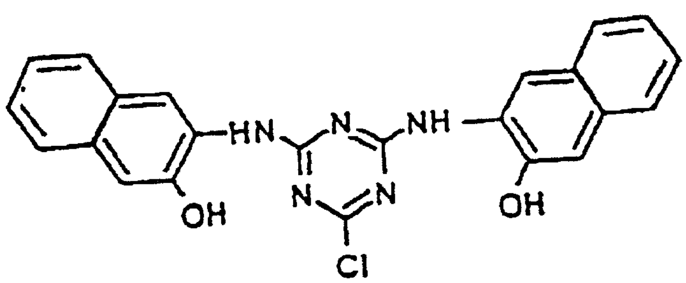

(3)

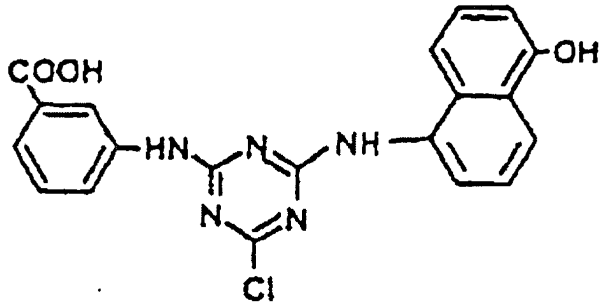

(4)

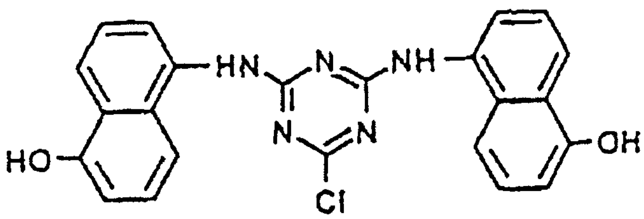

(5)

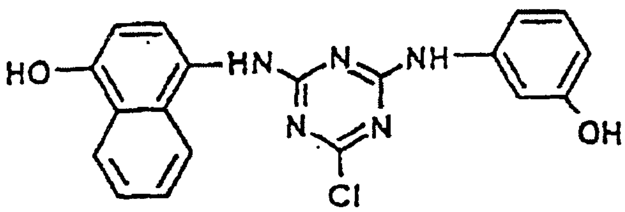

(6)

(7)

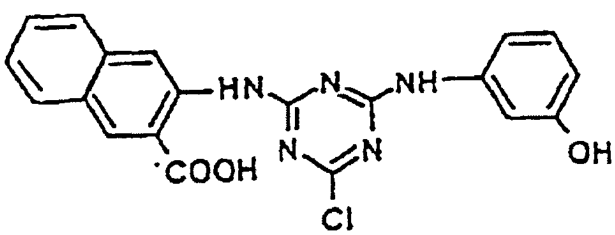

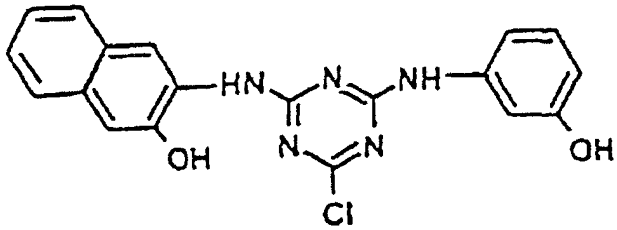

(8)

16

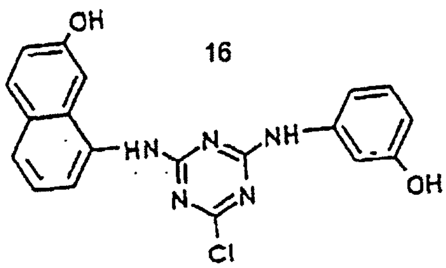

(9)

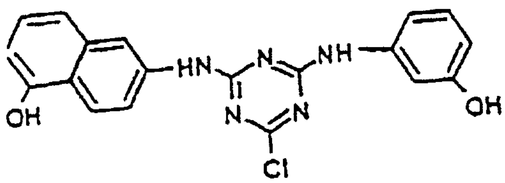

(10)

(11)

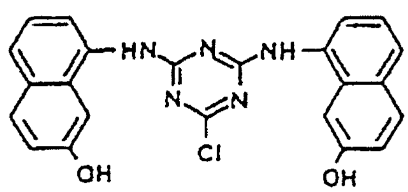

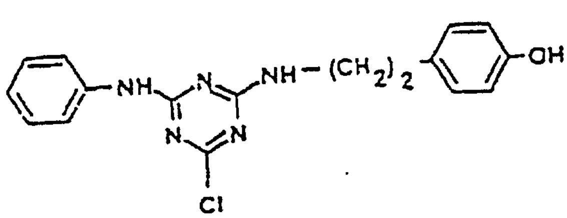

(12)

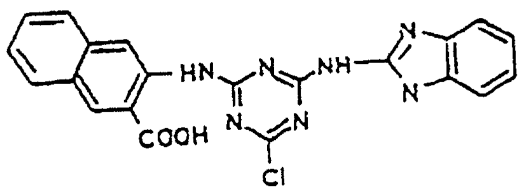

(13)

**54.** Utilisation de ligands d'affinité selon l'une quelconque des revendications précédentes pour la préparation de conjugués ligand d'affinité-matrice.

**55.** Procédé de fixation des nouveaux ligands d'affinité ayant les formules générales (VII) selon la revendication 27 et (XIII) selon la revendication 30, (XVI) selon la revendication 36 et (XI) selon la revendication 52, à des matrices à base d'hydrates de carbone ou de polymères organiques, par réaction de la matrice activée avec le nouveau ligand d'affinité, éventuellement en présence d'un agent liant les acides.

**56.** Procédé de fixation des nouveaux ligands d'affinité ayant les formules générales (XIV) selon la revendication 32 à des matrices constituées d'un hydrate de carbone ou d'un polymère organique, par condensation avec la matrice.

**57.** Procédé de fixation des nouveaux ligands d'affinité ayant les formules générales (VII) selon la revendication 27 et (XIII) selon la revendication 30, (XVI) selon la revendication 36 et (XI) selon la revendication 52 à des matrices d'oxyde métallique, de verre ou de silice, éventuellement revêtues d'un polymère organique, par réaction de la matrice d'oxyde métallique, de verre ou de silice éventuellement revêtue, avec un agent d'activation, puis réaction de la matrice activée avec le nouveau ligand d'affinité, éventuellement en présence d'un agent de liaison des acides.

**58.** Procédé de fixation des nouveaux ligands d'affinité ayant les formules générales (XVI) selon la revendication 32 à des matrices d'oxyde métallique, de verre ou de silice, éventuellement revêtues d'un polymère organique, par condensation avec la matrice.

**59.** Procédé de fixation des nouveaux ligands d'affinité ayant les formules générales (XV) selon la revendication 35 et (XII) selon la revendication 28 à une matrice de formule générale (V) selon la revendication 26 par réaction des nouveaux ligands d'affinité avec la matrice à des températures comprises entre -20 et 121°C, éventuellement en présence d'un agent de liaison des acides.

**60.** Conjugués ligand d'affinité-matrice préparés selon les revendications 26, 27, 29, 54, 55, 56, 57 et 58.

**61.** Utilisation des conjugués ligand d'affinité-matrice selon l'une quelconque des revendications précédentes concernant des conjugués ligand d'affinité-matrice pour séparer, isoler, purifier, caractériser, identifier ou quantifier des protéines.

**62.** Utilisation selon la revendication 61, pour laquelle le matériau protéiné est l'IgG, l'IgM, l'IgA, les insulines, le facteur VII, l'hormone de croissance humaine, ou des analogues, dérivés et fragments de ceux-ci, et les précurseurs de ceux-ci.

**63.** Procédé de séparation ou de purification de matériaux protéinés, comprenant la mise en oeuvre d'une chromatographie d'affinité, qui utilise en tant que ligand biospécifique un ligand de formule générale (a) telle que définie ci-dessus.

**64.** Utilisation selon la revendication 61, pour laquelle le matériau protéiné est constitué d'immunoglobulines ou de sous-classes, fragments, précurseurs ou dérivés de ces dernières, la dérivation provenant de sources naturelles ou recombinantes.

**65.** Utilisation selon la revendication 61, pour laquelle le matériau protéiné est constitué de l'immunoglobuline G (IgG),

de l'immunoglobuline M (IgM), de l'immunoglobuline A (IgA) , ou de sous-classes, fragments, précurseurs ou dérivés de celles-ci, la dérivation provenant de sources naturelles ou recombinantes.

**66.** Utilisation selon la revendication 61, pour laquelle le matériau protéiné est constitué d'insulines ou d'analogues de l'insuline, de dérivés et fragments de ces dernières, et de précurseurs, la dérivation provenant de sources naturelles ou recombinantes.

**67.** Utilisation selon la revendication 61, pour laquelle le matériau protéiné est le FVII ou des analogues, dérivés et fragments de ce dernier et des précurseurs, la dérivation provenant de sources naturelles ou recombinantes.

**68.** Utilisation selon la revendication 61, pour laquelle les Conjugués ligand d'affinité-matrice comprennent un ligand choisi parmi les composés suivants :

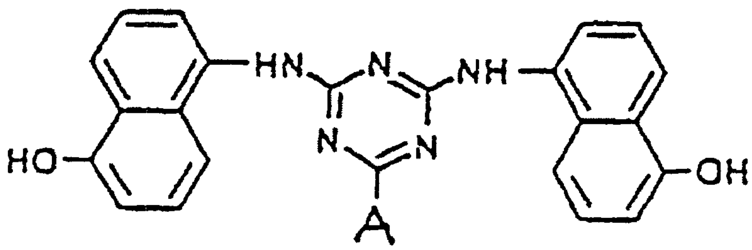

(5a)

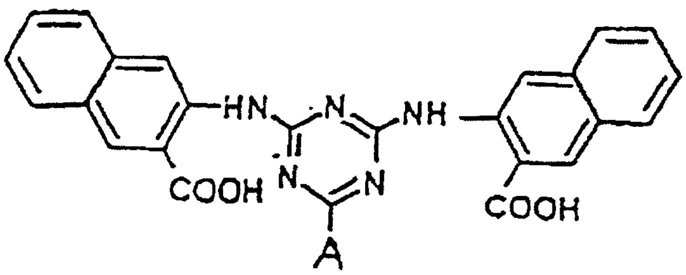

(2a)

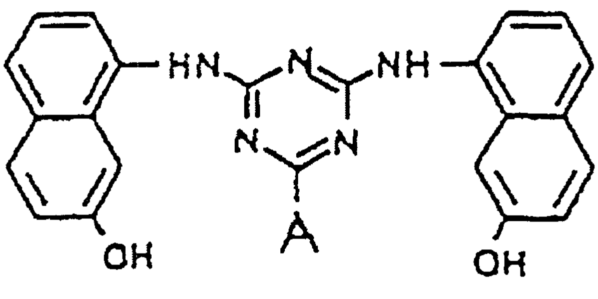

(11a)

ce ligand étant fixé à une matrice support en position (A), éventuellement par l'intermédiaire d'un bras espaceur représenté par la formule générale (b) spécifiée ci-dessus.

**69.** Utilisation selon la revendication 68, pour laquelle le ligand est le composé 11a.

**70.** Utilisation selon la revendication 68 ou 69, pour laquelle la matrice-support est une agarose éventuellement activée, une cellulose, de la silice ou du verre.

**71.** Séparation, isolement, purification, caractérisation, identification ou quantification d'immunoglobulines par un procédé quelconque, dans lequel lesdites immunoglobulines sont appliquées sur des conjugués ligand d'affinité-matrice selon l'une quelconque des revendications ci-dessus portant sur des conjugués ligand d'affinité-matrice,

à un pH compris dans la plage de 5,0 à 12,0, puis sont éliminées, élués ou désorbées par réduction du pH à 4,9 ou moins.

**72.** Séparation, isolement, purification, caractérisation, identification ou quantification d'insulines ou d'analogues de l'insuline ou de dérivés de ces derniers et de précurseurs par un procédé quelconque dans lequel lesdites insulines et lesdits dérivés de l'insuline, analogues et précurseurs, sont appliqués à des conjugués ligand d'affinité-matrice selon l'une quelconque des revendications ci-dessus relatives à des conjugués ligand d'affinité-matrice à un pH compris dans la plage de 4,0 à 9,0, puis sont éliminés, élués ou désorbés par réduction du pH à 3,99 ou moins ou 9,01 ou plus.

**73.** Séparation du FVIIa recombinant par liaison sélective du FVIIa à la matrice d'affinité ayant la formule viii)

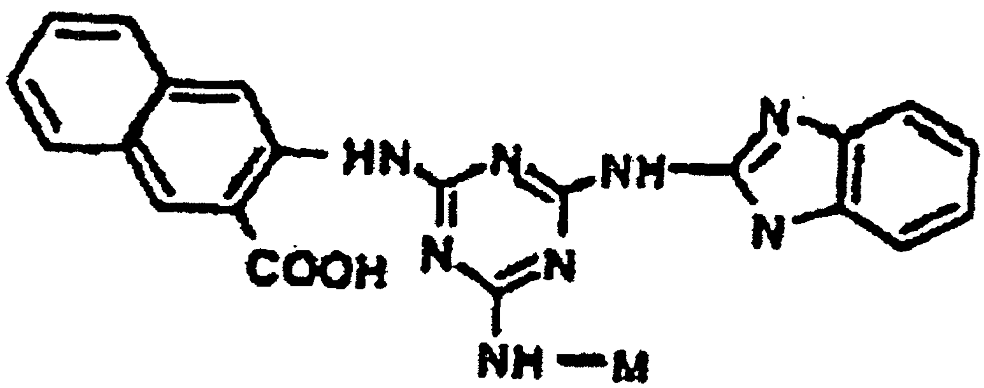

dans laquelle M est une agarose portant des groupes amino, à partir des milieux de culture cellulaire, ledit FVIIa étant appliqué audit conjugué ligand d'affinité-matrice en présence de $Ca^{2+}$ 5 mM, puis est élué.